# EUROPÄISCHE PATENTSCHRIFT

(11) **EP 1 888 541 B1**
(45) Veröffentlichungstag und Bekanntmachung des Hinweises auf die Patenterteilung: **05.08.2009**
(21) Anmeldenummer: 06742972.0
(22) Anmeldetag: 18.05.2006
(51) Int. Cl.: C07D 261/20, C07D 413/04, C07D 413/12, A61K 31/423, A61K 31/4439, A61P 29/00

(54) **BENZO (D) ISOXAZOL-3-YL-AMIN- VERBINDUNGEN UND DEREN VERWENDUNG ALS VANILLOID-REZEPTOR LIGANDEN**
BENZO(D)ISOXAZOL-3-YL-AMINE COMPOUNDS AND THEIR USE AS VANILLOID RECEPTOR LIGANDS
COMPOSES BENZO(D)ISOXAZOL-3-YL-AMINE ET LEURS UTILISATIONS COMME LIGANDS RECEPTEURS VANILLOIDES

(30) Priorität: 18.05.2005 DE 102005023589; 16.08.2005 DE 102005038947
(43) Veröffentlichungstag der Anmeldung: 20.02.2008
(73) Patentinhaber: Grünenthal GmbH, 52078 Aachen (DE)
(72) Erfinder: FRANK, Robert, 52070 Aachen (DE); MERLA, Beatrix, 52078 Aachen (DE); REICH, Melanie, 52072 Aachen (DE); JOSTOCK, Ruth, 52223 Stolberg (DE)
(74) Vertreter: Bülle, Jan
(86) Internationale Anmeldenummer: PCT/EP2006/004698
(87) Internationale Veröffentlichungsnummer: WO 2006/122799

(56) Entgegenhaltungen:
- WO-A-01/87845
- WO-A-20/05016915

## Beschreibung

Die vorliegende Erfindung betrifft substituierte Benzo[d]isoxazol-3-yl-amin-Verbindungen, Verfahren zu ihrer Herstellung, Arzneimittel enthaltend diese Verbindungen sowie die Verwendung dieser Verbindungen zur Herstellung von Arzneimitteln.

Die Behandlung von Schmerz, insbesondere von neurflpathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Einen geeigneten Ansatzpunkt zur Behandlung von Schmerz; insbesondere von Schmerz ausgewählt aus der Gruppe bestehend aus akutem Schmerz, chronischem Schmerz, neuropathischem Schmerz und visceralem Schmerz, besonders bevorzugt von neurophatischem Schmerz; stellt der Vanilloid-Rezeptor vom Subtyp 1 (VR1/TRPV1) dar, der häufig auch als Capsaicin-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung. Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Störungen des kardiovaskulären Systems; Störungen der Nahrungsaufnahme; Medikamentenabhängigkeit; Medikamentenmißbrauch und insbesondere Harninkontinenz.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass substituierte Benzo[d]isoxazol-3-yl-amin-Verbindungen der nachstehend angegebenen allgemeinen Formel I sich zur Behandlung von Schmerzen eignen und auch eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1/TRPV1) vermittelt werden.

Ein Gegenstand der vorliegenden Erfindung sind daher substituierte Benzo[d]isoxazol-3-yl-amin-Verbindungen der allgemeinen Formel I, 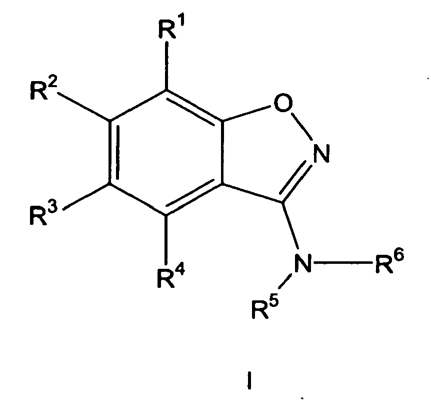 worin
- R¹, R², R³ und R⁴,: unabhängig voneinander, jeweils für
H; F, Cl, Br, I; -CN; -NC; -NO₂; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷;
für einen linearen oder verzweigten, gesättigten oder ungesättigten, unsubstituierten oder wenigstens einfach substituierten aliphatischen Rest;
für einen gesättigten oder ungesättigten, unsubstituierten oder wenigstens einfach substituierten, ggf. wenigstens ein Heteroatom als Ringglied aufweisenden cycloaliphatischen Rest, der mit einem mono- oder polyzyklischen Ringsystem kondensiert und/oder über eine lineare oder verzweigte Alkylen-, Alkenylen- oder Alkinylen-Gruppe gebunden sein kann;
oder für einen unsubstituierten oder wenigstens einfach substituierten Aryl-oder Heteroaryl-Rest steht, der mit einem mono- oder polyzyklischen Ringsystem kondensiert und/oder über eine lineare oder verzweigte Alkylen, Alkenylen- oder Alkinylen-Gruppe gebunden sein kann,
wobei wenigstens einer der Reste R¹, R², R³ und R⁴ für einen unsubstituierten oder wenigstens einfach substituierten Aryl- oder Heteroaryl-Rest steht;
- R⁵ und R⁶,: unabhängig voneinander, jeweils für
H; -C(=O)R¹⁸; -C(=O)NR¹⁹R²⁰; -C(=S)NR²¹R²²; -S(=O)₂R²³;
für einen linearen oder verzweigten, gesättigten oder ungesättigten, unsubstituierten oder wenigstens einfach substituierten aliphatischen Rest;
für einen gesättigten oder ungesättigten, unsubstituierten oder wenigstens einfach substituierten, ggf. wenigstens ein Heteroatom als Ringglied aufweisenden cycloaliphatischen Rest, der mit einem mono- oder polyzyklischen Ringsystem kondensiert und/oder über eine lineare oder verzweigte Alkylen-Gruppe gebunden sein kann;
oder für einen unsubstituierten oder wenigstens einfach substituierten Aryl-oder Heteroaryl-Rest steht, der mit einem mono- oder polyzyklischen Ringsystem kondensiert und/oder über eine lineare oder verzweigte Alkylen-Gruppe gebunden sein kann,
oder
- R⁵ und R⁶: zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
- R⁷ und R⁸,: unabhängig voneinander, jeweils für
H, -C(=O)R¹⁵ oder für einen linearen oder verzweigten, gesättigten oder ungesättigten, unsubstituierten oder wenigstens einfach substituierten aliphatischen Rest stehen, oder
- R⁷ und R⁸: zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
- R⁹, R¹⁰, R¹¹ und R¹⁶: unabhängig voneinander, jeweils für
H;
für einen linearen oder verzweigten, gesättigten oder ungesättigten, unsubstituierten oder wenigstens einfach substituierten aliphatischen Rest;
für einen gesättigten oder ungesättigten, unsubstituierten oder wenigstens einfach substituierten, ggf. wenigstens ein Heteroatom als Ringglied aufweisenden cycloaliphatischen Rest, der mit einem mono- oder polyzyklischen Ringsystem kondensiert und/oder über eine lineare oder verzweigte Alkylen-Gruppe gebunden sein kann;
oder für einen unsubstituierten oder wenigstens einfach substituierten Aryl-oder Heteroaryl-Rest stehen, der mit einem mono- oder polyzyklischen Ringsystem kondensiert und/oder über eine lineare oder verzweigte Alkylen-Gruppe gebunden sein kann;
- R¹² und R¹³,: unabhängig voneinander, jeweils für
H oder für einen linearen oder verzweigten, gesättigten oder ungesättigten, unsubstituierten oder wenigstens einfach substituierten aliphatischen Rest stehen, oder
- R¹² und R¹³: zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
- R¹⁴ und R²³,: unabhängig voneinander, jeweils für
-NR⁷R⁸;
für einen linearen oder verzweigten, gesättigten oder ungesättigten, unsubstituierten oder wenigstens einfach substituierten aliphatischen Rest,
für einen gesättigten oder ungesättigten, unsubstituierten oder wenigstens einfach substituierten, ggf. wenigstens ein Heteroatom als Ringglied aufweisenden cycloaliphatischen Rest, der mit einem mono- oder polyzyklischen Ringsystem kondensiert und/oder über eine lineare oder verzweigte Alkylen-Gruppe gebunden sein kann,
oder für einen unsubstituierten oder wenigstens einfach substituierten Aryl-oder Heteroaryl-Rest steht, der mit einem mono- oder polyzyklischen Ringsystem kondensiert und/oder über eine lineare oder verzweigte Alkylen-Gruppe gebunden sein kann,
- R¹⁵, R¹⁷ und R¹⁸,: unabhängig voneinander, jeweils
für einen linearen oder verzweigten, gesättigten oder ungesättigten, unsubstituierten oder wenigstens einfach substituierten aliphatischen Rest,
für einen gesättigten oder ungesättigten, unsubstituierten oder wenigstens einfach substituierten, ggf. wenigstens ein Heteroatom als Ringglied aufweisenden cycloaliphatischen Rest, der mit einem mono- oder polyzyklischen Ringsystem kondensiert und/oder über eine lineare oder verzweigte Alkylen-Gruppe gebunden sein kann,
oder für einen unsubstituierten oder wenigstens einfach substituierten Aryl-oder Heteroaryl-Rest steht, der mit einem mono- oder polyzyklischen Ringsystem kondensiert und/oder über eine lineare oder verzweigte Alkylen-Gruppe gebunden sein kann,
- R¹⁹ und R²⁰,: unabhängig voneinander, jeweils für
H oder für einen linearen oder verzweigten, gesättigten oder ungesättigten, unsubstituierten oder wenigstens einfach substituierten aliphatischen Rest stehen, oder
- R¹⁹ und R²⁰: zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
- R²¹ und R²²,: unabhängig voneinander, jeweils für
H oder für einen linearen oder verzweigten, gesättigten oder ungesättigten, unsubstituierten oder wenigstens einfach substituierten aliphatischen Rest stehen, oder
- R²¹ und R²²: zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Vorzugsweise stehen R⁵ und R⁶ nicht gleichzeitig für einen Rest ausgewählt aus der Gruppe bestehend aus linearen oder verzweigten, gesättigten oder ungesättigten, unsubstituierten oder wenigstens einfach substituierten aliphatischen Rest und Wasserstoff stehen.

Vorzugsweise sind cycloaliphatische Reste in Position der Substituenten R⁵ und R⁶ mit Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NO₂, -O-CF₃, -S-CF₃, -SH, -S-C₁₋₅-Alkyl, -C₁₋₅-Alkyl, -C(=O)-OH, -C=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert, wobei jeweils der zyklische Teil der Reste -O-Phenyl, -O-Benzyl, Phenyl und Benzyl mit Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Vorzugsweise sind Aryl- und Heteroaryl-Reste in Position der Substituenten R⁵ und R⁶ jeweils mit Substituenten unabhängig voneinander ausgewählt aus der Gruppe bestehend aus F, Cl, Br, I, -CN, -CF₃, -SF₅, -OH, -O-C₁₋₅-Alkyl, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, - C(=O)NH-C₁₋₅-Alkyl, -C(=O)-N(C₁₋₅-Alkyl)₂, -S(=O)₂NH₂; -S(=O)₂N(H)(C₁₋₅-Alkyl); - S(=O)₂N(C₁₋₅-Alkyl)(C₁₋₅-Alkyl); -S(=O)₂Phenyl; -S(=O)₂-C₁₋₅-Alkyl; -CH₂-NH-Cyclopropyl; -CH₂-N(C₁₋₅-Alkyl)-Cyclopropyl; -C(=O)-N(Cyclopropyl)-C₁₋₅-Alkyl, - N(Cyclopropyl)-S(=O)₂-C₁₋₅-Alkyl, -CH₂-NH-S(=O)₂-C₁₋₅-Alkyl, -CH₂-N(Cyclopropyl)-S(=O)₂-C₁₋₅-Alkyl, -CH₂-N(Cyclopropyl)-C(=O)-C₁₋₅-Alkyl, -CH₂-N(Cyclopropyl)-C(=O)-Phenyl, -O-Phenyl, -O-Benzyl, Phenyl, Benzyl, Thiophenyl (Thienyl) und Furanyl substituiert, wobei jeweils der zyklische Teil der Reste -CH₂-N(Cyclopropyl)-C(=O)-Phenyl, -O-Phenyl, -O-Benzyl, Phenyl, -S(=O)₂Phenyl und Benzyl mit Substituenten unabhängig voneinander ausgewählt aus der Gruppe bestehend aus F, Cl, Br, -OH, - CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Vorzugsweise sind Aryl- und Heteroaryl-Reste in Position der Subsituenten R¹, R², R³ und R⁴ mit Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, - S-CF₃, -SH, -S-C₁₋₅-Alkyl, -C₁₋₅-Alkyl, -C(=O)-OH, -C(=O)-O-C₁₋₅-Alkyl, -NH-(C₁₋₅-Alkyl), -N(C₁₋₅-Alkyl)(C₁₋₅-Alkyl), -NH-C(=O)-O-(C₁₋₅-Alkyl), -C(=O)-NH-CH₃, -C(=O)-NH-C₂H₅, -C(=O)-H, -C(=O)-(C₁₋₅-Alkyl), -C(=O)-N(C₁₋₅-Alkyl)(C₁₋₅-Alkyl), -S(=O)₂NH₂; -S(=O)₂N(H)(C₁₋₅-Alkyl); -S(=O)₂N(C₁₋₅-Alkyl)(C₁₋₅-Alkyl); -S(=O)₂Phenyl; -S(=O)₂-C₁₋₅-Alkyl; -C(=O)-N(Cyclopropyl)-C₁₋₅-Alkyl, -N(Cyclopropyl)-S(=O)₂-C₁₋₅-Alkyl, -CH₂-NH-S(=O)₂-C₁₋₅-Alkyl, -CH₂-N(Cyclopropyl)-S(=O)₂-C₁₋₅-Alkyl, -CH₂-N(Cyclopropyl)-C(=O)-C₁₋₅-Alkyl, -CH₂-N(Cyclopropyl)-C(=O)-Phenyl, -O-Phenyl, -O-Benzyl, Phenyl, Benzyl, Thiophenyl (Thienyl), Furanyl und Pyridinyl substituiert, wobei jeweils der zyklische Teil der Reste -CH₂-N(Cyclopropyl)-C(=O)-Phenyl, -O-Phenyl, -O-Benzyl, Phenyl, -S(=O)₂Phenyl, Benzyl, Thiophenyl (Thienyl), Furanyl und Pyridinyl mit Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Vorzugsweise können die vorstehend genannten (hetero)cycloaliphatischen Reste ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, - OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann. Sofern ein cycloaliphatischer Rest ein oder mehrere, beispielsweise 1, 2, 3, 4 oder 5, Heteroatome als Ringglieder aufweist, so können diese unabhängig voneinander bevorzugt ausgewählt werden aus der Gruppe bestehend aus Sauerstoff, Stickstoff und Schwefel.

Als (hetero)cycloaliphatische Reste seien beispielsweise Cyclopropyl, Cyclobutyl, Cyclopentyl, Cyclohexyl, Cycloheptyl, Cyclooctyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl und Dithiolanyl genannt.

Unter einem mono- oder polyzyklischen Ringsystem werden im Sinne der vorliegenden Erfindung mono- oder polyzyklische Kohlenwasserstoffreste verstanden, die gesättigt oder ungesättigt sein und ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind. Ein solches mono- bzw. polyzyklisches Ringsystem kann beispielsweise mit einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien (1,3)-Benzodioxolyl und (1,4)-Benzodioxanyl genannt.

Vorzugsweise sind die Ringe der vorstehend genannten mono- oder polyzyklischen Ringsysteme jeweils 5-, 6- oder 7-gliedrig und können jeweils ggf. 1, 2, 3, 4 oder 5 Heteroatom(e) als Ringglied(er) aufweisen, die unabhängig voneinander aus der Gruppe bestehend aus Sauerstoff, Stickstoff und Schwefel ausgewählt sind.

Ebenfalls bevorzugt können die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, - N(C₁₋₅-Alkyl)₂, -O-Phenyl, -O-Benzyl, Phenyl und Benzyl substituiert sein, wobei jeweils der zyklische Teil der Reste -O-Phenyl, -O-Benzyl, Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Sofern nicht anders angegeben, können die vorstehend genannten Aryl- oder Heteroaryl-Reste ebenfalls bevorzugt ggf. jeweils mit 1, 2, 3, 4 oder 5 Substituenten unabhängig voneinander ausgewählt aus der Gruppe bestehend aus F, Cl, Br, I, - CN, -CF₃, -SF₅, -OH, -O-C₁₋₅-Alkyl, -O-CF₃, -S-CF₃, -SH, -S-C₁₋₅-Alkyl, - C₁₋₅-Alkyl, -C(=O)-OH, -C(=O)-O-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)NH-C₁₋₅-Alkyl, - C(=O)-N(C₁₋₅-Alkyl)₂, -S(=O)₂NH₂; -S(=O)₂N(H)(C₁₋₅-Alkyl); -S(=O)₂N(C₁₋₅-Alkyl)(C₁₋₅-Alkyl); -S(=O)₂Phenyl; -S(=O)₂-C₁₋₅-Alkyl; -CH₂-NH-Cyclopropyl; -CH₂-N(C₁₋₅-Alkyl)-Cyclopropyl; -C(=O)-N(Cyclopropyl)-C₁₋₅-Alkyl, -N(Cyclopropyl)-S(=O)₂-C₁₋₅-Alkyl, - CH₂-NH-S(=O)₂-C₁₋₅-Alkyl, -CH₂-N(Cyclopropyl)-S(=O)₂-C₁₋₅-Alkyl, -CH₂-N(Cyclopropyl)-C(=O)-C₁₋₅-Alkyl, -CH₂-N(Cyclopropyl)-C(=O)-Phenyl, -O-Phenyl, -O-Benzyl, Phenyl, Benzyl, Thiophenyl (Thienyl), Furanyl und Pyridinyl substituiert sind, wobei jeweils der zyklische Teil der Reste -CH₂-N(Cyclopropyl)-C(=O)-Phenyl, -O-Phenyl, -O-Benzyl, Phenyl, -S(=O)₂Phenyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Ebenfalls bevorzugt weisen die vorstehend genannten Heteroaryl-Reste jeweils 1, 2, 3, 4 oder 5 Heteroatom(e) unabhängig voneinander ausgewählt aus der Gruppe bestehend aus Sauerstoff, Stickstoff und Schwefel als Ringglied(er) auf.

Als Aryl-Reste seien beispielsweise Phenyl und Naphthyl (umfassend 1-Naphthyl und 2-Naphthyl) genannt.

Als Heteroaryl-Reste seien beispielsweise Thiophenyl, Furanyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinoxalinyl, Chinolinyl und Isochinolinyl genannt.

Die vorstehend genannten aliphatischen Reste, d.h. die Alkyl-, Alkenyl- und Alkinyl-Reste, können bevorzugt 1-10 bzw. 2-10 Kohlenstoffatome im Alkylteil aufweisen und bevorzugt mit ggf. 1, 2, 3, 4, 5, 6, 7, 8 oder 9 Substituenten unabhängig voneinander ausgewählt aus der Gruppe bestehend aus F, Cl, Br, I, -CN, -NO₂, -OH, -NH₂, -SH, -O(C₁₋₅-Alkyl), -S(C₁₋₅-Alkyl), -NH(C₁₋₅-Alkyl), -N(C₁₋₅-Alkyl)(C₁₋₅-Alkyl), - OCF₃ und -SCF₃ substituiert sein. Alkenyl-Reste weisen wenigstens eine, vorzugsweise 1, 2, 3 oder 4 C-C-Doppelbindungen und Alkinyl-Reste wenigstens eine, vorzugsweise 1, 2, 3 oder 4 C-C-Dreifachbindungen auf.

Bevorzugt sind Alkyl-Reste ausgewählt aus der Gruppe bestehend aus Methyl, Ethyl, n-Propyl, Isopropyl, n-Butyl, sec-Butyl, Isobutyl, tert-Butyl, n-Pentyl, sec-Pentyl, Neopentyl und n-Hexyl, die ggf. mit 1, 2, 3, 4, 5, 6, 7, 8 oder 9 Substituenten unabhängig voneinander ausgewählt aus der Gruppe bestehend aus F, Cl, Br, I, - CN, -NO₂, -OH, -NH₂, -SH, -OCH₃, -O-C₂H₅, -SCH₃, -S-C₂H₅, -OCF₃, -SCF₃, -NH-CH₃, -N(CH₃)₂, -N(C₂H₅)₂ und -N(CH₃)(C₂H₅) substituiert sein können.

Bevorzugt sind ferner Alkenyl-Reste ausgewählt aus der Gruppe bestehend aus Vinyl, 1-Propenyl, 2-Propenyl, 1-Butenyl, 2-Butenyl, 3-Butenyl, 2-Methyl-buten-2-yl, 1-Pentenyl, 2-Pentenyl, 3-Pentenyl und 4-Pentenyl, die ggf. mit 1, 2 oder 3 Substituenten unabhängig voneinander ausgewählt aus der Gruppe bestehend aus F, Cl, Br, I, -CN, -NO₂, -OH, -NH₂, -SH, -OCH₃, -O-C₂H₅, -SCH₃, -S-C₂H₅, -OCF₃, - SCF₃, -NH-CH₃, -N(CH₃)₂, -N(C₂H₅)₂ und -N(CH₃)(C₂H₅) substituiert sein können.

Weiterhin bevorzugt sind Alkinyl-Reste ausgewählt aus der Gruppe bestehend aus Ethinyl, 1-Propinyl, 2-Propinyl, 1-Butinyl, 2-Butinyl und 3-Butinyl, die ggf. mit 1, 2 oder 3 Substituenten unabhängig voneinander ausgewählt aus der Gruppe bestehend aus F, Cl, Br, I, -CN, -NO₂, -OH, -NH₂, -SH, -OCH₃, -O-C₂H₅, -SCH₃, -S-C₂H₅, -OCF₃, -SCF₃, -NH-CH₃, -N(CH₃)₂, -N(C₂H₅)₂ und -N(CH₃)(C₂H₅) substituiert sein können.

Bevorzugt sind substituierte Benzo[d]isoxazol-3-yl-amin-Verbindungen der allgemeinen Formel I, worin
- R¹, R², R³ und R⁴,: unabhängig voneinander, jeweils für
H; F; CI; Br; I; -CN; -NC; -NO₂; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl oder -(C₁₋₅-Alkylen)-Heteroaryl stehen;
wobei wenigstens einer der Reste R¹, R², R³ und R⁴ für einen Aryl- oder Heteroaryl-Rest steht;
- R⁵ und R⁶,: unabhängig voneinander, jeweils für
H; -C(=O)R¹⁸; -C(=O)NR¹⁹R²⁰; -C(=S)NR²¹R²²; -S(=O)₂R²³; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl, Aryl; Heteroaryl; -(C₁₋₅-Alkylen)-Aryl oder -(C₁₋₅-Alkylen)-Heteroaryl stehen;
mit der Maßgabe, dass R⁵ und R⁶ nicht gleichzeitig für einen Rest ausgewählt aus der Gruppe bestehend aus C₁₋₁₀-Alkyl und Wasserstoff stehen;
oder
- R⁵ und R⁶: zusammen mit dem sie verbindenden Stickstoffatom als Ringglied einen Rest bilden, der ausgewählt ist aus der Gruppe H bestehend aus 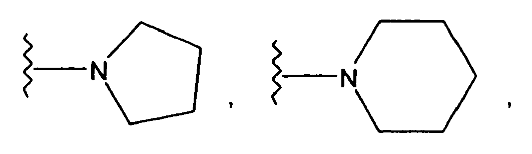 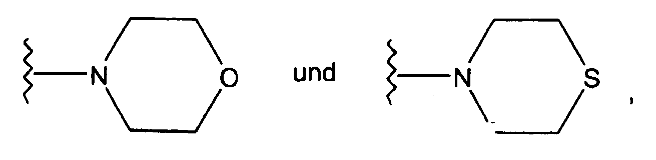
- R⁷ und R⁸,: unabhängig voneinander, jeweils für
H, -C(=O)R¹⁵ oder C₁₋₁₀-Alkyl stehen, oder
- R⁷ und R⁸: zusammen mit dem sie verbindenden Stickstoffatom als Ringglied einen Rest bilden, der ausgewählt ist aus der vorstehend angegebenen Gruppe H;
- R⁹, R¹⁰, R¹¹ und R¹⁶: unabhängig voneinander, jeweils für
H; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl oder -(C₁₋₅-Alkylen)-Heteroaryl stehen;
- R¹² und R¹³,: unabhängig voneinander, jeweils für
H oder für einen C₁₋₁₀-Alkyl-Rest; oder
- R¹² und R¹³: zusammen mit dem sie verbindenden Stickstoffatom als Ringglied einen Rest bilden, der ausgewählt ist aus der vorstehend angegebenen Gruppe H;
- R¹⁴ und R²³,: unabhängig voneinander, jeweils für
-NR⁷R⁸; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl oder -(C₁₋₅-Alkylen)-Heteroaryl stehen;
- R¹⁵, R¹⁷ und R¹⁸,: unabhängig voneinander, jeweils für
C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl oder -(C₁₋₅-Alkylen)-Heteroaryl stehen;
- R¹⁹ und R²⁰,: unabhängig voneinander, jeweils für
H oder für C₁₋₁₀-Alkyl stehen, oder
- R¹⁹ und R²⁰: zusammen mit dem sie verbindenden Stickstoffatom als Ringglied einen Rest bilden, der ausgewählt ist aus der vorstehend angegebenen Gruppe H;
- R²¹ und R²²,: unabhängig voneinander, jeweils für
H oder für C₁₋₁₀-Alkyl stehen, oder
- R²¹ und R²²: zusammen mit dem sie verbindenden Stickstoffatom als Ringglied einen Rest bilden, der ausgewählt ist aus der vorstehend angegebenen Gruppe H;
wobei
die vorstehend genannten C₁₋₁₀-Alkyl-, C₂₋₁₀-Alkenyl- und C₂₋₁₀-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; -CN; -OH; -SH; - O-C₁₋₂-Alkyl; -S-C₁₋₂-Alkyl und -NH₂ substituiert sein können,
die vorstehend genannten C₃₋₈-Cycloalkyl-Reste jeweils ggf. mit 1, 2, 3, 4 oder 5 Substituenten unabhängig voneinander ausgewählt aus der Gruppe bestehend aus F; Cl; Br; -CN; -OH; -SH; -C₁₋₅-Alkyl; -O-C₁₋₂-Alkyl; -S-C₁₋₂-Alkyl und -NH₂ substituiert sein können,
die vorstehend genannten Heterocycloalkyl-Reste jeweils 4-, 5-, 6- oder 7-gliedrig sind, 1 oder 2 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; -CN; -OH; -SH; -C₁₋₅-Alkyl; -O-C₁₋₂-Alkyl; -S-C₁₋₂-Alkyl und -NH₂ substituiert sein können,
die vorstehend genannten Aryl-Reste jeweils für einen Phenyl- oder Naphthyl-Rest stehen, der mit 1, 2, 3, 4 oder 5 Substituenten unabhängig voneinander ausgewählt aus der Gruppe bestehend aus F; Cl; Br; I; -CF₃; -OCF₃; -SCF₃; -SF₅; -CN; -OH; -SH; -C₁₋₅-Alkyl; -O-C₁₋₅-Alkyl; -S-C₁₋₅-Alkyl; -C(=O)-OH; -C(=O)-O-C₁₋₅-Alkyl; -C(=O)-NH-CH₃, -C(=O)-NH-C₂H₅, -N(H)(C₁₋₅-Alkyl); -N(C₁₋₅-Alkyl)(C₁₋₅-Alkyl); -C(=O)-H; - C(=O)-C₁₋₅-Alkyl; -C(=O)N(C₁₋₅-Alkyl)(C₁₋₅-Alkyl); -S(=O)₂NH₂; -S(=O)₂N(H)(C₁₋₅-Alkyl); -S(=O)₂N(C₁₋₅-Alkyl)(C₁₋₅-Alkyl); -S(=O)₂-Phenyl; -S(=O)₂-C₁₋₅-Alkyl; -CH₂-NH-Cyclopropyl; -CH₂-N(C₁-₅-Alkyl)-Cyclopropyl; -C(=O)-N(Cyclopropyl)-C₁₋₅-Alkyl, - N(Cyclopropyl)-S(=O)₂-C₁₋₅-Alkyl, -CH₂-NH-S(=O)₂-C₁₋₅-Alkyl, -CH₂-N(Cyclopropyl)-S(=O)₂-C₁₋₅-Alkyl, -CH₂-N(Cyclopropyl)-C(=O)-C₁₋₅-Alkyl, -CH₂-N(Cyclopropyl)-C(=O)-Phenyl, Phenyl; Phenoxy, Benzyl; Thiophenyl (Thienyl), Furanyl und Pyridinyl substituiert sein kann,
die vorstehend genannten Heteroaryl-Reste jeweils 5- oder 6-gliedrig sind, 1, 2 oder 3 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; I; -CF₃; -OCF₃; -SCF₃; -SF₅; -CN; -OH; -SH; -C₁₋₅-Alkyl; -O-C₁₋₅-Alkyl; -S-C₁₋₅-Alkyl; -C(=O)-OH; -C(=O)-OC₁₋₅-Alkyl; -NH₂; -N(H)(C₁₋₅-Alkyl); - N(C₁₋₅-Alkyl)(C₁₋₅-Alkyl); -C(=O)NH₂; -C(=O)-NH-CH₃, -C(=O)-NH-C₂H₅, -C(=O)N(C₁₋₅-Alkyl)(C₁₋₅-Alkyl); -S(=O)₂NH₂; -S(=O)₂N(H)(C₁₋₅-Alkyl); -S(=O)₂N(C₁₋₅-Alkyl)(C₁₋₅-Alkyl); -S(=O)₂-Phenyl; -S(=O)₂-C₁₋₅-Alkyl; -C(=O)-N(Cyclopropyl)-C₁₋₅-Alkyl, -N(Cyclopropyl)-S(=O)₂-C₁₋₅-Alkyl, -CH₂-NH-S(=O)₂-C₁₋₅-Alkyl, -CH₂-N(Cyclopropyl)-S(=O)₂-C₁₋₅-Alkyl, -CH₂-N(Cyclopropyl)-C(=O)-C₁₋₅-Alkyl, - CH₂-N(Cyclopropyl)-C(=O)-Phenyl, Phenyl; Phenoxy, Benzyl; Thiophenyl (Thienyl), Furanyl und Pyridinyl substituiert sein können,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind Verbindungen der allgemeinen Formel I, worin
- R¹, R², R³ und R⁴,: unabhängig voneinander, jeweils für
H; F; CI; Br; I, -CN; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; C₁₋₄-Alkyl, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
wobei wenigstens einer der Reste R¹, R², R³ und R⁴ für einen Aryl- oder Heteroaryl-Rest steht;
- R⁵ und R⁶,: unabhängig voneinander, jeweils für
H; -C(=O)R¹⁸; -C(=O)NR¹⁹R²⁰; -C(=S)NR²¹R²²; -S(=O)₂R²³; C₁₋₄-Alkyl, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
mit der Maßgabe, dass R⁵ und R⁶ nicht gleichzeitig für einen Rest ausgewählt aus der Gruppe bestehend aus C₁₋₄-Alkyl und Wasserstoff stehen;
oder
- R⁵ und R⁶: zusammen mit dem sie verbindenden Stickstoffatom als Ringglied einen Rest bilden, der ausgewählt ist aus der Gruppe H bestehend aus 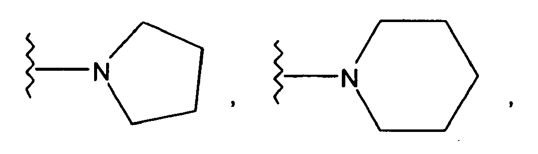 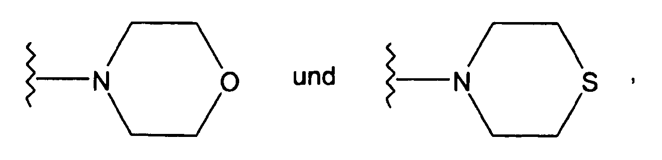
- R⁷ und R⁸,: unabhängig voneinander, jeweils für
H, -C(=O)R¹⁵ oder C₁₋₄-Alkyl stehen, oder
- R⁷ und R⁸: zusammen mit dem sie verbindenden Stickstoffatom als Ringglied einen Rest bilden, der ausgewählt ist aus der vorstehend angegebenen Gruppe H;
- R⁹, R¹⁰, R¹¹ und R¹⁶: unabhängig voneinander, jeweils für
H; C₁₋₄-Alkyl, C₂₋₄-Alkenyl, C₂₋₄-Alkinyl; C₃₋₈-Cycloalkyl; -(C_{1, 2 oder 3}-Alkylen)-C₃-₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
- R¹² und R¹³,: unabhängig voneinander, jeweils für
H oder für C₁₋₄-Alkyl; oder
- R¹² und R¹³: zusammen mit dem sie verbindenden Stickstoffatom als Ringglied einen Rest bilden, der ausgewählt ist aus der vorstehend angegebenen Gruppe H;
- R¹⁴ und R²³,: unabhängig voneinander, jeweils für
-NR⁷R⁸; C₁₋₄-Alkyl, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
- R¹⁵, R¹⁷ und R¹⁸,: unabhängig voneinander, jeweils für
C₁₋₄-Alkyl, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
- R¹⁹ und R²⁰,: unabhängig voneinander, jeweils für
H oder für C₁₋₄-Alkyl stehen, oder
- R¹⁹ und R²⁰: zusammen mit dem sie verbindenden Stickstoffatom als Ringglied einen Rest bilden, der ausgewählt ist aus der vorstehend angegebenen Gruppe H;
- R²¹ und R²²,: unabhängig voneinander, jeweils für
H oder für C₁₋₄-Alkyl stehen, oder
- R²¹ und R²²: zusammen mit dem sie verbindenden Stickstoffatom als Ringglied einen Rest bilden, der ausgewählt ist aus der vorstehend angegebenen Gruppe H;
wobei
die vorstehend genannten C₁₋₄-Alkyl-, C₂₋₄-Alkenyl- und C₂₋₄-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; -CN; -OH; -OCH₃ und -NH₂ substituiert sein können,
die vorstehend genannten C₃₋₈-Cycloalkyl-Reste jeweils ggf. mit 1, 2, 3, 4 oder 5 Substituenten unabhängig voneinander ausgewählt aus der Gruppe bestehend aus F; Cl; Br; -CN; -OH; -CH₃; -C₂H₅; -OCH₃; und -NH₂ substituiert sein können,
die vorstehend genannten Heterocycloalkyl-Reste jeweils 4-, 5-, 6- oder 7-gliedrig sind, 1 oder 2 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; -CN; -OH; -CH₃; -C₂H₅; -OCH₃; und -NH₂ substituiert sein können,
die vorstehend genannten Aryl-Reste jeweils für einen Phenyl- oder Naphthyl-Rest stehen, der mit 1, 2, 3, 4 oder 5 Substituenten unabhängig voneinander ausgewählt aus der Gruppe bestehend aus F; Cl; Br; -CF₃; -OCF₃; -SCF₃; -SF₅; -CN; -OH; Methyl; Ethyl; n-Propyl; Isopropyl; n-Butyl; Isobutyl; sec-Butyl; tert-Butyl; Methoxy; Ethoxy; -N(CH₃)₂; -N(C₂H₅)₂; Phenyl; Phenoxy, Benzyl; Thiophenyl (Thienyl), Furanyl, Pyridinyl, -C(=O)-NH-CH₃, -C(=O)-NH-C₂H₅, -C(=O)-H, -C(=O)-CH₃, - C(=O)-C₂H₅, -C(=O)-CH(CH₃)₂, -C(=O)-C(CH₃)₃, -C(=O)-O-CH₃, -C(=O)-O-C₂H₅, - C(=O)-O-C(CH₃)₃, -C(=O)-O-CH(CH₃)₂, -C(=O)-OH, -C(=O)-N(Cyclopropyl)-CH₃, - C(=O)-N(Cyclopropyl)-C₂H₅, -C(=O)-N(Cyclopropyl)-CH(CH₃)₂, -C(=O)-N(Cyclopropyl)-C(CH₃)₃, -CH₂-NH-Cyclopropyl, -CH₂-N(Cyclopropyl)-S(=O)₂-CH₃, - CH₂-N(Cyclopropyl)-S(=O)₂-C₂H₅, -CH₂-N(Cyclopropyl)-C(=O)-CH₃, -CH₂-N(Cyclopropyl)-C(=O)-C₂H₅, -CH₂-N(Cyclopropyl)-C(=O)-CH(CH₃)₂, -CH₂-N(Cyclopropyl)-C(=O)-C(CH₃)₃ und -CH₂-N(Cyclopropyl)-C(=O)-Phenyl substituiert sein kann;
die vorstehend genannten Heteroaryl-Reste jeweils für einen Furanyl-, Thienyl-(Thiophenyl-) oder Pyridinyl-Rest stehen und ggf. mit 1, 2, 3, 4 oder 5 Substituenten unabhängig voneinander ausgewählt aus der Gruppe bestehend F; Cl; Br; -CF₃; -OCF₃; -SCF₃, -SF₅; -CN; -OH; Methyl; Ethyl; n-Propyl; Isopropyl; n-Butyl; Isobutyl; sec-Butyl; tert-Butyl; Methoxy; Ethoxy; -NH₂; -N(CH₃)₂; -N(C₂H₅)₂; Phenyl; Phenoxy, Benzyl; Thiophenyl (Thienyl), Furanyl, Pyridinyl, -C(=O)-H, -C(=O)-CH₃, -C(=O)-C₂H₅, -C(=O)-CH(CH₃)₂, -C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-NH-CH₃, -C(=O)-NH-C₂H₅, - C(=O)-N(Cyclopropyl)-CH₃, -C(=O)-N(Cyclopropyl)-C₂H₅, -C(=O)-N(Cyclopropyl)-CH(CH₃)₂, -C(=O)-N(Cyclopropyl)-C(CH₃)₃, -CH₂-NH-Cyclopropyl, -CH₂-N(Cyclopropyl)-S(=O)₂-CH₃, -CH₂-N(Cyclopropyl)-S(=O)₂-C₂H₅, -CH₂-N(Cyclopropyl)-C(=O)-CH₃, -CH₂-N(Cyclopropyl)-C(=O)-C₂H₅, -CH₂-N(Cyclopropyl)-C(=O)-CH(CH₃)₂, -CH₂-N(Cyclopropyl)-C(=O)-C(CH₃)₃ und -CH₂-N(Cyclopropyl)-C(=O)-Phenyl substituiert sein können,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind Verbindungen der allgemeinen Formel I', 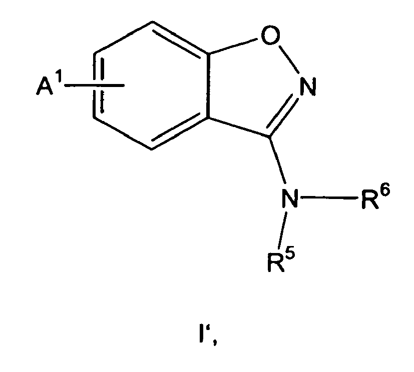 worin
- A¹: für einen (Hetero)aryl-Rest steht, der ausgewählt ist aus der Gruppe bestehend aus Phenyl, 1-Naphthyl, 2-Naphthyl, Thiophenyl (Thienyl), Furanyl und Pyridinyl und der ggf. mit 1, 2, 3, 4 oder 5 Substituenten unabhängig voneinander ausgewählt aus der Gruppe bestehend F; Cl; Br; -CF₃; -OCF₃;-SCF₃; -SF₅; -CN; -OH; Methyl; Ethyl; n-Propyl; Isopropyl; n-Butyl; Isobutyl; sec-Butyl; tert-Butyl; Methoxy; Ethoxy; -NH₂; -N(CH₃)₂; -N(C₂H₅)₂; Phenyl; Phenoxy, Benzyl; Thiophenyl (Thienyl), Furanyl, Pyridinyl, -C(=O)-NH-CH₃,-C(=O)-NH-C₂H₅, -C(=O)-H, -C(=O)-CH₃, -C(=O)-C₂H₅, -C(=O)-CH(CH₃)₂,-C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(Cyclopropyl)-CH₃, -C(=O)-N(Cyclopropyl)-C₂H₅, -C(=O)-N(Cyclopropyl)-CH(CH₃)₂, -C(=O)-N(Cyclopropyl)-C(CH₃)₃,-CH₂-NH-Cyclopropyl, -CH₂-N(Cyclopropyl)-S(=O)₂-CH₃, -CH₂-N(Cyclopropyl)-S(=O)₂-C₂H₅, -CH₂-N(Cyclopropyl)-C(=O)-CH₃, -CH₂-N(Cyclopropyl)-C(=O)-C₂H₅, -CH₂-N(Cyclopropyl)-C(=O)-CH(CH₃)₂, -CH₂-N(Cyclopropyl)-C(=O)-C(CH₃)₃ und -CH₂-N(Cyclopropyl)-C(=O)-Phenyl substituiert sein kann;
- R⁵ und R⁶,: unabhängig voneinander, jeweils für
H; einen Alkyl-Rest ausgewählt aus der Gruppe bestehend aus Methyl; Ethyl; n-Propyl; Isopropyl; n-Butyl; Isobutyl; sec-Butyl, tert-Butyl, n-Pentyl und Neopentyl; für -C(=O)-Phenyl; -(CH₂)-Phenyl; -(CH₂)₂-Phenyl; -(CH₂)₃-Phenyl; -(CH₂)-Pyridinyl; -(CH₂)₂-Pyridinyl; oder -(CH₂)₃-Pyridinyl stehen; wobei die vorstehend genannten Phenyl und Pyridinyl-Reste ggf. mit 1, 2, 3, 4 oder 5 Substituenten unabhängig voneinander ausgewählt aus der Gruppe bestehend F; Cl; Br; -CF₃; -OCF₃; -SCF₃; -SF₅; -CN; -OH; Methyl; Ethyl; n-Propyl; Isopropyl; n-Butyl; Isobutyl; sec-Butyl; tert-Butyl; Methoxy; Ethoxy; -N(CH₃)₂; -N(C₂H₅)₂; Phenyl; Phenoxy, Benzyl; Thiophenyl (Thienyl), Furanyl und Pyridinyl substituiert sein können;
mit der Maßgabe, dass R⁵ und R⁶ nicht gleichzeitig für einen Rest ausgewählt aus der Gruppe bestehend aus Alkyl-Resten und Wasserstoff stehen;
oder
- R⁵ und R⁶: zusammen mit dem sie verbindenden Stickstoffatom als Ringglied einen Rest bilden, der ausgewählt ist aus der Gruppe H bestehend aus 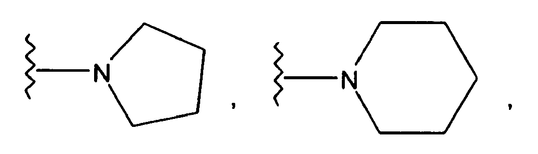 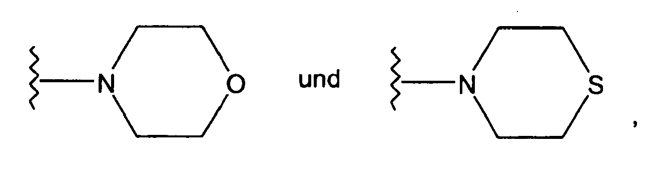
jeweils ggf. in Form entsprechender Salze, insbesondere der Hydrochlorid-Additionssalze, oder jeweils in Form entsprechender Solvate.

Weiterhin bevorzugt sind erfindungsgemäße Verbindungen der allgemeinen Formel I-A, I-B, I-C und I-D, 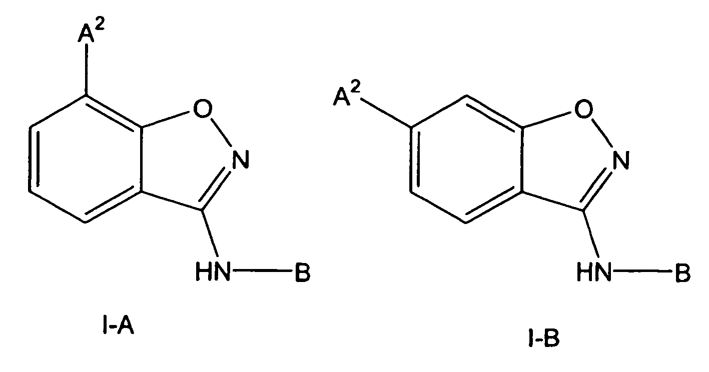 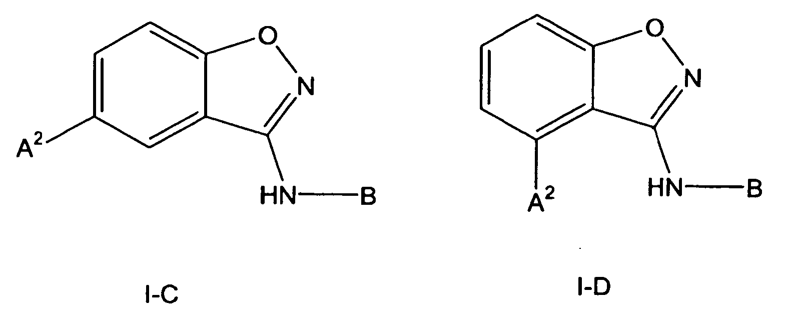 worin jeweils
- A²: für einen (Hetero)aryl-Rest steht, der ausgewählt ist aus der Gruppe bestehend aus Phenyl, 1-Naphthyl, 2-Naphthyl, Thiophenyl (Thienyl), Furanyl und Pyridinyl und der ggf. mit 1, 2, 3, 4 oder 5 Substituenten unabhängig voneinander ausgewählt aus der Gruppe bestehend F; Cl; Br; -CF₃; -OCF₃;-SCF₃; -SF₅; -CN; -OH; Methyl; Ethyl; n-Propyl; Isopropyl; n-Butyl; Isobutyl; sec-Butyl; tert-Butyl; Methoxy; Ethoxy; -NH₂; -N(CH₃)₂; -N(C₂H₅)₂; Phenyl; Phenoxy, Benzyl; Thiophenyl (Thienyl), Furanyl, Pyridinyl, -C(=O)-NH-CH₃,-C(=O)-NH-C₂H₅, -C(=O)-H, -C(=O)-CH₃, -C(=O)-C₂H₅, -C(=O)-CH(CH₃)₂,-C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(Cyclopropyl)-CH₃, -C(=O)-N(Cyclopropyl)-C₂H₅, -C(=O)-N(Cyclopropyl)-CH(CH₃)₂, -C(=O)-N(Cyclopropyl)-C(CH₃)₃,-CH₂-NH-Cyclopropyl, -CH₂-N(Cyclopropyl)-S(=O)₂-CH₃, -CH₂-N(Cyclopropyl)-S(=O)₂-C₂H₅, -CH₂-N(Cyclopropyl)-C(=O)-CH₃, -CH₂-N(Cyclopropyl)-C(=O)-C₂H₅, -CH₂-N(Cyclopropyl)-C(=O)-CH(CH₃)₂, -CH₂-N(Cyclopropyl)-C(=O)-C(CH₃)₃ und -CH₂-N(Cyclopropyl)-C(=O)-Phenyl substituiert sein kann;
- B: für für -C(=O)-Phenyl; -(CH₂)-Phenyl; -(CH₂)₂-Phenyl; -(CH₂)₃-Phenyl; -(CH₂)-Pyridinyl; -(CH₂)₂-Pyridinyl oder -(CH₂)₃-Pyridinyl steht; wobei die vorstehend genannten Phenyl und Pyridinyl-Reste ggf. mit 1, 2, 3, 4 oder 5 Substituenten unabhängig voneinander ausgewählt aus der Gruppe bestehend F; Cl; Br;-CF₃; -OCF₃; -SCF₃; -SF₅; -CN; -OH; Methyl; Ethyl; n-Propyl; Isopropyl; n-Butyl; Isobutyl; sec-Butyl; tert-Butyl; Methoxy; Ethoxy; -NH₂; -N(CH₃)₂; -N(C₂H₅)₂; Phenyl; Phenoxy, Benzyl; Thiophenyl (Thienyl), Furanyl und Pyridinyl substituiert sein können,
jeweils ggf. in Form entsprechender Salze, insbesondere der Hydrochlorid-Additionssalze, oder jeweils in Form entsprechender Solvate.

Ganz besonders bevorzugt sind erfindungsgemäße Verbindungen der allgemeinen Formel I-A, I-B, I-C und I-D, worin jeweils A² für einen der folgenden Reste A²¹ oder A²² steht: 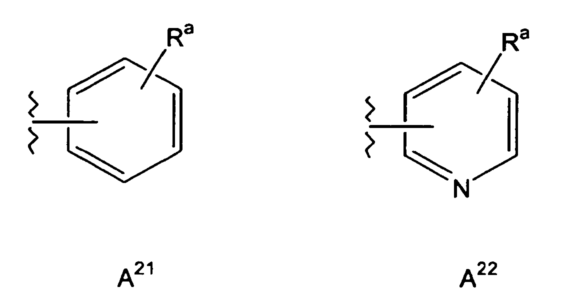 worin
- R^{a}: jeweils für einen Rest steht, der ausgewählt ist aus der Gruppe bestehend aus H, F, Cl, Br, I, -CN; -CF₃, -OCF₃, -SCF₃, -SF₅, -OH, -SH, Methyl; Ethyl; n-Propyl; Isopropyl; n-Butyl; Isobutyl; sec-Butyl; tert-Butyl; -C(=O)-NH-CH₃,-C(=O)-NH-C₂H₅, -OCH₃; -OC₂H₅; -S-CH₃, -S-C₂H₅, -C(=O)-H, -C(=O)-O-C₂H₅, -C(=O)-OH, -CH₂-NH-Cyclopropyl, -CH₂-N(Cyclopropyl)-S(=O)₂-CH₃, -CH₂-N(Cyclopropyl)-C(=O)-CH₃ und -CH₂-N(Cyclopropyl)-C(=O)-Phenyl steht und
- B: für einen der folgenden Reste B¹, B² oder B³ steht, 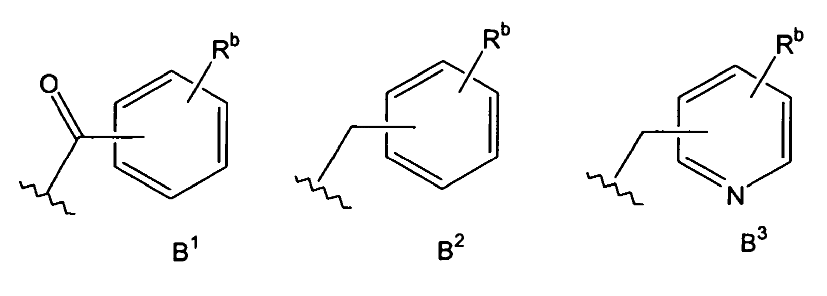
worin
- R^{b}: jeweils für einen Rest steht, der ausgewählt ist aus der Gruppe bestehend aus H, F, Cl, Br, I, -CN; -CF₃, -OCF₃, -SCF₃, -SF₅, -OH, -SH, Methyl; Ethyl; n-Propyl; Isopropyl; n-Butyl; Isobutyl, sec-Butyl; tert-Butyl; -OCH₃; -OC₂H₅; -S-CH₃ und -S-C₂H₅.
jeweils ggf. in Form entsprechender Salze, insbesondere der Hydrochlorid-Additionssalze, oder jeweils in Form entsprechender Solvate.

Vorzugsweise sind die erfindungsgemäßen substituierten Benzo[d]isoxazol-3-yl-amin-Verbindungen in ihrer 3-Position nicht mit einer freien Aminogruppe substituiert, d.h. die erfindungsgemäßen Reste R⁵ und R⁶ stehen nicht gleichzeitig für einen Wasserstoff-Rest.

Am meisten bevorzugt sind erfindungsgemäße Verbindungen ausgewählt aus der Gruppe bestehend aus
[1] Benzyl-[6-(3-chlor-phenyl)-benzo[d]isoxazol-3-yl]-amin,
[2] Benzyl-[6-(4-chlor-phenyl)-benzo[d]isoxazol-3-yl]-amin,
[3] Benzyl-(6-p-tolyl-benzo[d]isoxazol-3-yl)-amin,
[4] Benzyl-[6-(2-methoxy-phenyl)-benzo[d]isoxazol-3-yl]-amin,
[5] N-[6-(3-Chlor-phenyl)-benzo[d]isoxazol-3-yl]-benzamid,
[6] N-[6-(4-Chlor-phenyl)-benzo[d]isoxazol-3-yl]-benzamid,
[7] N-(6-p-Tolyl-benzo[d]isoxazol-3-yl)-benzamid,
[8] N-[6-(2-Methoxy-phenyl)-benzo[d]isoxazol-3-yl]-benzamid,
[9] (4-tert-Butyl-benzyl)-(6-pyridin-2-yl-benzo[d]isoxazol-3-yl)-amin,
[10] (4-tert-Butyl-benzyl)-[6-(3-methyl-pyridin-2-yl)-benzo[d]isoxazol-3-yl]-amin,
[11] (4-tert-Butyl-benzyl)-[6-(6-methyl-pyridin-2-yl)-benzo[d]isoxazol-3-yl]-amin,
[12] (4-tert-Butyl-benzyl)-(6-phenyl-benzo[d]isoxazol-3-yl)-amin,
[13] [6-(4-Chlor-phenyl)-benzo[d]isoxazol-3-yl]-pyridin-2-ylmethyl-amin,
[14] (6-Phenyl-benzo[d]isoxazol-3-yl)-pyridin-2-ylmethyl-amin,
[15] Pyridin-2-ylmethyl-(6-o-tolyl-benzo[d]isoxazol-3-yl)-amin,
[16] [6-(4-Chlor-phenyl)-benzo[d]isoxazol-3-yl]-pyridin-3-ylmethyl-amin,
[17] (6-Phenyl-benzo[d]isoxazol-3-yl)-pyridin-3-ylmethyl-amin,
[18] Pyridin-3-ylmethyl-(6-o-tolyl-benzo[d]isoxazol-3-yl)-amin,
[19] 2-{3-[(Pyridin-3-ylmethyl)-amino]-benzo[d]isoxazol-6-yl}-phenol,
[20] [6-(4-Chlor-phenyl)-benzo[d]isoxazol-3-yl]-pyridin-4-ylmethyl-amin,
[21] (6-Phenyl-benzo[d]isoxazol-3-yl)-pyridin-4-ylmethyl-amin,
[22] Pyridin-4-ylmethyl-(6-o-tolyl-benzo[d]isoxazol-3-yl)-amin,
[23] 4-{[6-(4-Chlor-phenyl)-benzo[d]isoxazol-3-ylamino]-methyl}-benzonitril,
[24] 4-[(6-Phenyl-benzo[d]isoxazol-3-ylamino)-methyl]-benzonitril,
[25] 4-[(6-o-Tolyl-benzo[d]isoxazol-3-ylamino)-methyl]-benzonitril,
[26] 4-{[6-(2-Hydroxy-phenyl)-benzo[d]isoxazol-3-ylamino]-methyl}- benzonitril,
[27] [6-(4-Chlor-phenyl)-benzo[d]isoxazol-3-yl]-(3-methyl-benzyl)-amin,
[28] (3-Methyl-benzyl)-(6-phenyl-benzo[d]isoxazol-3-yl)-amin,
[29] (3-Methyl-benzyl)-(6-o-tolyl-benzo[d]isoxazol-3-yl)-amin,
[30] 2-[3-(3-Methyl-benzylamino)-benzo[d]isoxazol-6-yl]-phenol,
[31] (3-Chlor-benzyl)-[6-(4-chlor-phenyl)-benzo[d]isoxazol-3-yl]-amin,
[32] (3-Chlor-benzyl)-(6-phenyl-benzo[d]isoxazol-3-yl)-amin,
[33] (3-Chlor-benzyl)-(6-o-tolyl-benzo[d]isoxazol-3-yl)-amin und
[34] 2-[3-(3-Chlor-benzylamino)-benzo[d]isoxazol-6-yl]-phenol,
sowie jeweils deren entsprechende Salze, insbesondere deren Hydrochlorid-Additionssalze, und ggf. jeweils deren entsprechende Solvate.

Weiterhin können erfindungsgemäße substituierte Benzo[d]isoxazol-3-yl-amin-Verbindungen bevorzugt sein, die im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM aufweisen.

Dabei wird Im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist ein Verfahren zur Herstellung der erfindungsgemäßen substituierten Benzo[d]isoxazol-3-yl-amin-Verbindungen, gemäß dem eine ggf. substituierte 2-Fluor-benzonitril-verbindung der allgemeinen Formel II, 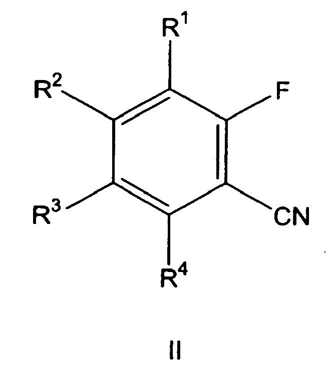 worin die Reste R¹, R², R³ und R⁴ die vorstehend angegebene Bedeutung haben, in einem Reaktionsmedium, vorzugsweise ausgewählt aus der Gruppe bestehend aus Diethylether, Tetrahydrofuran, Acetonitril, Dimethylsulfoxid, Dimethylformamid und Dichlormethan, in Gegenwart einer Base, vorzugsweise in Gegenwart wenigstens eines Alkalimetallalkoholatsalzes, besonders bevorzugt in Gegenwart eines Alkalimetallalkoholatsalzes ausgewählt aus der Gruppe bestehend aus Kaliummethanolat, Natriummethanolat, Kalium-tert-butylat und Natrium-tert-butylat mit Acethydroxamsäure der Formel III 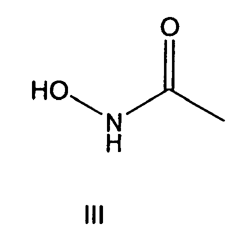 vorzugsweise bei Temperaturen von 20 °C bis 100 °C zu einer Verbindung der allgemeinen Formel I, 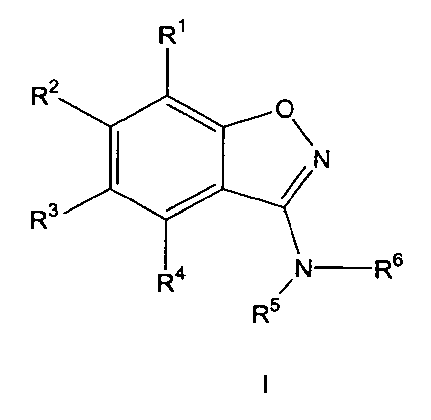 worin die Reste R¹-R⁴ die vorstehend genannte Bedeutung haben sowie die Reste R⁵ und R⁶ jeweils für einen Wasserstoff-Rest stehen, umgesetzt wird, diese ggf. gereinigt und/oder ggf. isoliert wird,
und diese ggf. anschließend in einem Reaktionsmedium, vorzugsweise ausgewählt aus der Gruppe bestehend aus Acetonitril, Toluol, Dimethylformamid, Benzol, Ethanol, Methanol und entsprechenden Mischungen, mit wenigstens einem Isocyanat der allgemeinen Formel R¹⁹-N=C=O, worin R¹⁹ die vorstehend angegebene Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I ungesetzt wird, worin R¹, R², R³, R⁴ die vorstehend genannte Bedeutung haben, R⁵ für einen Wasserstoff-Rest steht und R⁶ für -C(=O)-NR¹⁹R²⁰ steht, wobei R¹⁹ die vorstehend genannte Bedeutung hat und R²⁰ für einen Wasserstoff-Rest steht, und diese ggf. gereinigt und/oder ggf. isoliert wird
oder
diese ggf. anschließend in einem Reaktionsmedium, vorzugsweise ausgewählt aus der Gruppe bestehend aus Acetonitril, Toluol, Dimethylformamid, Benzol, Ethanol, Methanol und entsprechenden Mischungen, mit wenigstens einem Isothiocyanat der allgemeinen Formel S=C=N-R²¹, worin R²¹ die vorstehend angegebene Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I ungesetzt wird, worin R¹, R², R³, R⁴ die vorstehend genannte Bedeutung haben, R⁵ für einen Wasserstoff-Rest steht und R⁶ für -C(=S)-NR²¹R²² steht, wobei R²¹ die vorstehend genannte Bedeutung hat und R²² für einen Wasserstoff-Rest steht, und diese ggf. gereinigt und/oder ggf. isoliert wird,
und ggf. wenigstens eine Verbindung der allgemeinen Formel I, worin R¹, R², R³, R⁴ die vorstehend genannte Bedeutung haben, R⁵ für einen Wasserstoff-Rest steht und R⁶ für -C(=O)-NR¹⁹R²⁰ oder für -C(=S)-NR²¹R²² steht, wobei R¹⁹ und R²¹ die vorstehend genannte Bedeutung haben und R²⁰ und R²² jeweils für einen Wasserstoff-Rest stehen, in einem Reaktionsmedium, vorzugsweise ausgewählt aus der Gruppe bestehend aus Acetonitril, Toluol, Dimethylformamid, Benzol, Ethanol, Methanol und entsprechenden Mischungen, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R²⁰ oder der allgemeinen Formel LG-R²², worin LG für eine Abgangsgruppe, bevorzugt für ein Halogen-Atom, besonders bevorzugt für ein Chloratom steht, und R²² und R²⁴ jeweils die vorstehend genannte Bedeutung mit Ausnahme von Wasserstoff haben, zu wenigstens einer Verbindung der allgemeinen Formel I ungesetzt wird, und diese ggf. gereinigt und/oder ggf. isoliert wird,
oder
ggf. wenigstens eine Verbindung der allgemeinen Formel I, worin R¹, R², R³, R⁴ die vorstehend genannte Bedeutung haben und R⁵ und R⁶ jeweils für H stehen, in einem Reaktionsmedium, vorzugsweise ausgewählt aus der Gruppe bestehend aus Acetonitril, Toluol, Dimethylformamid, Benzol, Ethanol, Methanol und entsprechenden Mischungen, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R⁵ oder der allgemeinen Formel LG-R⁶, worin LG für eine Abgangsgruppe, bevorzugt für ein Halogen-Atom, besonders bevorzugt für ein Chloratom steht, und R⁵ und R⁶ jeweils die vorstehend genannte Bedeutung haben, zu wenigstens einer Verbindung der allgemeinen Formel I umgesetzt und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen in einem Reaktionsmedium, vorzugsweise Dichlormethan, in Gegenwart wenigstens eines Reduktionsmittels, bevorzugt in Gegenwart von Trifluoressigsäure und Triethylsilan, mit wenigstens einer Verbindung der allgemeinen Formel R⁵-(C=O)-H, worin R⁵ die vorstehend genannte Bedeutung mit Ausnahme von H, - C(=O)R¹⁸, -C(=O)-NR¹⁹R²⁰, -C(=S)-NR²¹R²² und -S(=O)₂R²³ hat, zu wenigstens einer Verbindung der allgemeinen Formel I ungesetzt und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium, vorzugsweise Pyridin, mit wenigstens einer Verbindung der allgemeinen Formel R¹⁸-C(=O)-LG, worin R¹⁸ die vorstehend genannte Bedeutung hat und LG für eine Abgangsgruppe, vorzugsweise für ein Halogenatom, besonders bevorzugt für ein Chloratom steht zu wenigstens einer Verbindung der allgemeinen Formel I ungesetzt wird, worin R¹ bis R⁴ die vorstehend genannte Bedeutung haben, R⁵ für einen -C(=O)R¹⁸-Rest und R⁶ für einen Wasserstoff-Rest steht; und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1H-1, 2, 3-triazolo[4,5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU), O-(Benzotriazol-1-yl)-N,N,N',N'-tetramethyluroniom tetrafluoroborat (TBTU), 1-Hydroxybenzotriazol (HOBt) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, N-Methylmorpholin, Pyridin, N,N-Dimethylaminopyridin und Diisopropylethylamin vorzugsweise bei Temperaturen von -70°C bis 100°C mit wenigstens einer Verbindung der allgemeinen Formel R¹⁸-C(=O)-OH, worin R¹⁸ die vorstehend angegebene Bedeutung hat zu wenigstens einer Verbindung der allgemeinen Formel I umgesetzt wird, worin R¹ bis R⁴ die vorstehend genannte Bedeutung haben, R⁵ für einen -C(=O)R¹⁸-Rest und R⁶ für einen Wasserstoff-Rest steht; und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium, vorzugsweise ausgewählt aus der Gruppe bestehend aus Acetonitril, Toluol, Dimethylformamid, Benzol, Ethanol, Methanol und entsprechenden Mischungen, ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, N-Methylmorpholin, Pyridin, N,N-Dimethylaminopyridin und Diisopropylethylamin, mit wenigstens einer Verbindung der allgemeinen Formel R²³-S(=O)₂-LG, worin R²³ die vorstehend angegebene Bedeutung hat und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ bis R⁴ die vorstehend genannte Bedeutung haben, R⁵ für eine -S(=O)₂-R²³-Gruppe und R⁶ für einen Wasserstoff-Rest steht, und diese ggf. gereinigt und/oder ggf. isoliert wird.

Ebenfalls Gegenstand der vorliegenden Erfindung ist ein Verfahren zur Herstellung erfindungsgemäßer Benzo[d]isoxazol-3-yl-amin-Verbindungen, gemäß dem eine ggf. substituierte 2-Fluor-benzonitril-verbindung der allgemeinen Formel II, 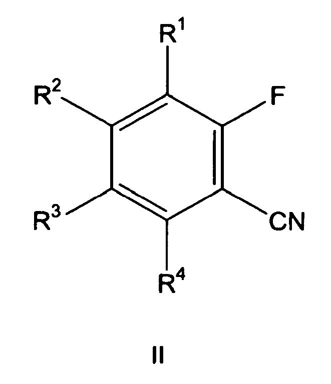 worin einer Reste R¹, R², R³ und R⁴ für eine Abgangsgruppe, vorzugsweise für ein Bromatom, steht, in einem Reaktionsmedium, vorzugsweise ausgewählt aus der Gruppe bestehend aus Diethylether, Tetrahydrofuran, Acetonitril, Dimethylsulfoxid, Dimethylformamid und Dichlormethan, in Gegenwart einer Base, vorzugsweise in Gegenwart wenigstens eines Alkalimetallalkoholatsalzes, besonders bevorzugt in Gegenwart eines Alkalimetallalkoholatsalzes ausgewählt aus der Gruppe bestehend aus Kaliummethanolat, Natriummethanolat, Kalium-tert-butylat und Natrium-tert-butylat mit Acethydroxamsäure der Formel III 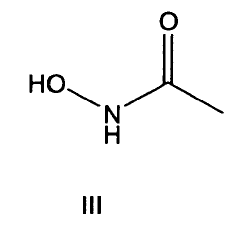 vorzugsweise bei Temperaturen von 20 °C bis 100 °C zu einer Verbindung der allgemeinen Formel I, 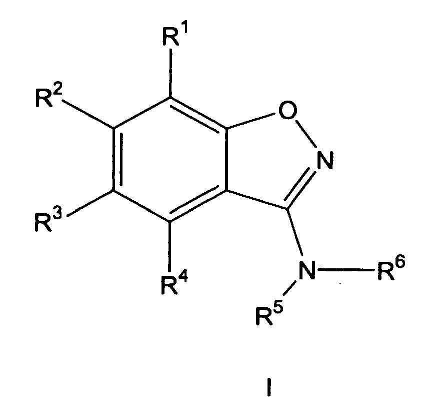 worin die Reste R¹-R⁴ die vorstehend genannte Bedeutung haben sowie die Reste R⁵ und R⁶ jeweils für einen Wasserstoff-Rest stehen, umgesetzt wird, diese ggf. gereinigt und/oder ggf. isoliert wird,
und diese ggf. anschließend in einem Reaktionsmedium, vorzugsweise ausgewählt aus der Gruppe bestehend aus Acetonitril, Toluol, Dimethylformamid, Benzol, Ethanol, Methanol und entsprechenden Mischungen, mit wenigstens einem Isocyanat der allgemeinen Formel R¹⁹-N=C=O, worin R¹⁹ die vorstehend angegebene Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹, R², R³, R⁴ die vorstehend genannte Bedeutung haben, R⁵ für einen Wasserstoff-Rest steht und R⁶ für -C(=O)-NR¹⁹R²⁰ steht, wobei R¹⁹ die vorstehend genannte Bedeutung hat und R²⁰ für einen Wasserstoff-Rest steht, und diese ggf. gereinigt und/oder ggf. isoliert wird
oder
diese ggf. anschließend in einem Reaktionsmedium, vorzugsweise ausgewählt aus der Gruppe bestehend aus Acetonitril, Toluol, Dimethylformamid, Benzol, Ethanol, Methanol und entsprechenden Mischungen, mit wenigstens einem Isothiocyanat der allgemeinen Formel S=C=N-R²¹, worin R²¹ die vorstehend angegebene Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I ungesetzt wird, worin R¹, R², R³, R⁴ die vorstehend genannte Bedeutung haben, R⁵ für einen Wasserstoff-Rest steht und R⁶ für -C(=S)-NR²¹R²² steht, wobei R²¹ die vorstehend genannte Bedeutung hat und R²² für einen Wasserstoff-Rest steht, und diese ggf. gereinigt und/oder ggf. isoliert wird,
und ggf. wenigstens eine Verbindung der allgemeinen Formel I, worin R¹, R², R³, R⁴ die vorstehend genannte Bedeutung haben, R⁵ für einen Wasserstoff-Rest steht und R⁶ für -C(=O)-NR¹⁹R²⁰ oder für -C(=S)-NR²¹R²² steht, wobei R¹⁹ und R²¹ die vorstehend genannte Bedeutung haben und R²⁰ und R²² jeweils für einen Wasserstoff-Rest stehen, in einem Reaktionsmedium, vorzugsweise ausgewählt aus der Gruppe bestehend aus Acetonitril, Toluol, Dimethylformamid, Benzol, Ethanol, Methanol und entsprechenden Mischungen, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R²⁰ oder der allgemeinen Formel LG-R²², worin LG für eine Abgangsgruppe, bevorzugt für ein Halogen-Atom, besonders bevorzugt für ein Chloratom steht, und R²² und R²⁴ jeweils die vorstehend genannte Bedeutung mit Ausnahme von Wasserstoff haben, zu wenigstens einer Verbindung der allgemeinen Formel I umgesetzt wird, und diese ggf. gereinigt und/oder ggf. isoliert wird,
oder
ggf. wenigstens eine Verbindung der allgemeinen Formel I, worin R¹, R², R³, R⁴ die vorstehend genannte Bedeutung haben und R⁵ und R⁶ jeweils für H stehen, in einem Reaktionsmedium, vorzugsweise ausgewählt aus der Gruppe bestehend aus Acetonitril, Toluol, Dimethylformamid, Benzol, Ethanol, Methanol und entsprechenden Mischungen, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R⁵ oder der allgemeinen Formel LG-R⁶, worin LG für eine Abgangsgruppe, bevorzugt für ein Halogen-Atom, besonders bevorzugt für ein Chloratom steht, und R⁵ und R⁶ jeweils die vorstehend genannte Bedeutung haben, zu wenigstens einer Verbindung der allgemeinen Formel I umgesetzt und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen in einem Reaktionsmedium, vorzugsweise Dichlormethan, in Gegenwart wenigstens eines Reduktionsmittels, bevorzugt in Gegenwart von Trifluoressigsäure und Triethylsilan, mit wenigstens einer Verbindung der allgemeinen Formel R⁵-(C=O)-H, worin R⁵ die vorstehend genannte Bedeutung mit Ausnahme von H, - C(=O)R¹⁸, -C(=O)-NR¹⁹R²⁰, -C(=S)-NR²¹R²² und -S(=O)₂R²³ hat, zu wenigstens einer Verbindung der allgemeinen Formel I umgesetzt und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium, vorzugsweise Pyridin, mit wenigstens einer Verbindung der allgemeinen Formel R¹⁸-C(=O)-LG, worin R¹⁸ die vorstehend genannte Bedeutung hat und LG für eine Abgangsgruppe, vorzugsweise für ein Halogenatom, besonders bevorzugt für ein Chloratom steht zu wenigstens einer Verbindung der allgemeinen Formel I umgesetzt wird, worin R¹ bis R⁴ die vorstehend genannte Bedeutung haben, R⁵ für einen -C(=O)R¹⁸-Rest und R⁶ für einen Wasserstoff-Rest steht; und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium, vorzugsweise ausgewählt aus der Gruppe bestehend aus Diethylether, Tetrahydrofuran, Acetonitril, Methanol, Ethanol, Dimethylformamid, Dichlormethan und entsprechenden Mischungen, in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU), O-(Benzotriazol-1-yl)-N,N,N',N'-tetramethyluroniom tetrafluoroborat (TBTU), 1-Hydroxybenzotriazol (HOBt) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, N-Methylmorpholin, Pyridin, N,N-Dimethylaminopyridin und Diisopropylethylamin vorzugsweise bei Temperaturen von -70°C bis 100°C mit wenigstens einer Verbindung der allgemeinen Formel R¹⁸-C(=O)-OH, worin R¹⁸ die vorstehend angegebene Bedeutung hat zu wenigstens einer Verbindung der allgemeinen Formel I ungesetzt wird, worin R¹ bis R⁴ die vorstehend genannte Bedeutung haben, R⁵ für einen -C(=O)R¹⁸-Rest und R⁶ für einen Wasserstoff-Rest steht; und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium, vorzugsweise ausgewählt aus der Gruppe bestehend aus Acetonitril, Toluol, Dimethylformamid, Benzol, Ethanol, Methanol und entsprechenden Mischungen, ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, N-Methylmorpholin, Pyridin, N,N-Dimethylaminopyridin und Diisopropylethylamin, mit wenigstens einer Verbindung der allgemeinen Formel R²³-S(=O)₂-LG, worin R²³ die vorstehend angegebene Bedeutung hat und LG für eine Abgangsgruppe, bevorzugt für ein Halogen-Atom, besonders bevorzugt für ein Chloratom steht, zu wenigstens einer Verbindung der allgemeinen Formel I ungesetzt wird, worin R¹ bis R⁴ die vorstehend genannte Bedeutung haben, R⁵ für eine -S(=O)₂-R²³-Gruppe und R⁶ für einen Wasserstoff-Rest steht, und diese ggf. gereinigt und/oder ggf. isoliert wird,
und jeweils die so erhaltene Verbindung der allgemeinen Formel I, in der wenigstens einer der Reste R¹ bis R⁴ für eine Abgangsgruppe, vorzugsweise für einen Halogen-Rest oder einen Sulfonsäureester, besonders bevorzugt für eine Abgangsgruppe ausgewählt aus der Gruppe bestehend aus Chlor, Brom, Iod, Triflat, Mesylat und Tosylat, ganz besonders bevorzugt für ein Bromatom, steht, und die übrigen Reste die vorstehend genannte Bedeutung haben, ggf. in wenigstens einem Reaktionsmedium, bevorzugt in wenigstens einem Reaktionsmedium ausgewählt aus der Gruppe bestehend aus Methanol, Ethylacetat, Ethanol, Isopropanol, Diethylether, Dimethoxyethan, Dioxan, Tetrahydrofuran, Dimethylformamid, Acetonitril, Dimethylsulfoxid, Toluol, N-Methyl-pyrrolidin und Wasser oder entsprechenden Mischungen, ggf. in Gegenwart wenigstens einer Base und eines Katalysators, der polymergebunden sein kann, bevorzugt in Gegenwart wenigstens einer Base ausgewählt aus der Gruppe bestehend aus Kaliumcarbonat, Natriumcarbonat, Kaliumphosphat, Natriumhydrogenphosphat, Cäsiumcarbonat, Triethylamin, [1,4]-Diazabicyclo-[2.2.2]-octan, Diisopropylamin, Diisopropylethylamin und N-Methylmorpholin und eines Katalysators, der polymergebunden sein kann, ausgewählt aus der Gruppe bestehend aus Palladium(II)acetat, Tris(dibenzylidenaceton)dipalladium, Palladium(O)bis(dibenzylidenaceton), Tetrakis(triphenylphosphin)palladium(O), (1,1'-Bis(diphenylphosphino)-ferrocen)dichloropalladium(II), Bis(acetonitril)dichloropalladium(II), Palladium(II)chlorid, Dichlorobis(triphenyl-phosphin)palladium(II), Dichloro(tricyclohexylphosphin)palladium(II), Bis(acetato)bis(triphenylphosphin)-palladium(II), Bistriphenylphosphinpalladium(II)dichlorid, Bistriphenyl-phosphinpalladium(II)acetat und Eisen(III)chlorid, besonders bevorzugt in Gegenwart von einem Alkalicarbonat und einem Palladiumkatalysator, ganz besonders bevorzugt in Gegenwart von Natriumcarbonat und Tetrakis(triphenylphosphin)-palladium (0), ggf. in Gegenwart wenigstens eines Liganden, der polymergebunden sein kann, bevorzugt in Gegenwart wenigstens eines Liganden, der polymergebunden sein kann, ausgewählt aus der Gruppe bestehend aus 2-Dicyclohexylphosphino-2',6'-dimethoxybiphenyl (S-Phos), 2-Dicyclohexylphosphino-2',4',6'-triisopropylbiphenyl (X-Phos), Tricyclohexylphosphin, Tricyclohexylphosphin Tetrafluoroborat, Tri-tert-butylphosphin Tetrafluoroborat, Triphenylphosphin und Imidazolium-Salzen, ggf. unter Verwendung von Mikrowellenstrahlung, mit wenigstens einer Boronsäure-Verbindung der folgenden Formel umsetzt, 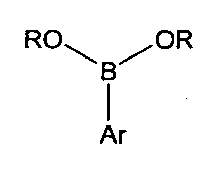 worin Ar für einen unsubstituierten oder wenigstens einfach substituierten Aryl- oder Heteroaryl-Rest mit der vorstehend genannten Bedeutung steht und R für Wasserstoff oder einen organischen Rest, vorzugsweise für Wasserstoff oder einen Alkyl-Rest steht oder zwei Reste R zusammen mit der sie verbindenden -O-B-O-Gruppe einen Heterocycloalkyl-Rest bilden, und die so erhaltene Verbindung der allgemeinen Formel I, in der wenigstens einer der Reste R¹ bis R⁴ für einen unsubstituierten oder wenigstens einfach substituierten Aryl- oder Heteroaryl-Rest steht, ggf. reinigt und/oder ggf. isoliert,
oder jeweils die so erhaltene Verbindung der allgemeinen Formel I, in der wenigstens einer der Reste R¹ bis R⁴ für eine Abgangsgruppe, vorzugsweise für einen Halogen-Rest oder einen Sulfonsäureester, besonders bevorzugt für eine Abgangsgruppe ausgewählt aus der Gruppe bestehend aus Chlor, Brom, Iod, Triflat, Mesylat und Tosylat, ganz besonders bevorzugt für ein Bromatom, steht, und die übrigen Reste die vorstehend genannte Bedeutung haben, mit einer Organometall-Verbindung der allgemeinen Formel R'-M-X, worin R' für einen unsubstituierten oder wenigstens einfach substituierten Aryl- oder Heteroaryl-Rest mit der vorstehend genannten Bedeutung steht, M für ein Übergangsmetall-Ion, vorzugsweise für ein Magnesium-oder Zink-Ion, und X für eine Abgangsgruppe, vorzugsweise für einen Halogen-Rest oder einen Sulfonsäureester, besonders bevorzugt für eine Abgangsgruppe ausgewählt aus der Gruppe bestehend aus Chlor, Brom, Iod, Triflat, Mesylat und Tosylat, ganz besonders bevorzugt für ein Bromatom, steht, ggf. in wenigstens einem Reaktionsmedium, bevorzugt in wenigstens einem Reaktionsmedium ausgewählt aus der Gruppe bestehend aus Methanol, Ethylacetat, Ethanol, Isopropanol, Diethylether, Dimethoxyethan, Dioxan, Tetrahydrofuran, Dimethylformamid, Acetonitril, Dimethylsulfoxid, Toluol, N-Methyl-pyrrolidin und Wasser oder entsprechenden Mischungen, ggf. in Gegenwart wenigstens eines Katalysators, der polymergebunden sein kann, bevorzugt in Gegenwart eines Katalysators, der polymergebunden sein kann, ausgewählt aus der Gruppe bestehend aus Palladium(II)acetat, Tris(dibenzylidenaceton)dipalladium, Palladium(O)bis(dibenzylidenaceton), Tetrakis(triphenylphosphin)palladium(O), (1,1'-Bis(diphenylphosphino)-ferrocen)dichloropalladium(II), Bis(acetonitril)dichloropalladium(II), Palladium(II)chlorid, Dichlorobis(triphenylphosphin)palladium(II), Dichloro(tricyclohexylphosphin)palladium(II), Bis(acetato)bis(triphenylphosphin)-palladium(II), Bistriphenylphosphinpalladium(II)dichlorid, Bistriphenyl-phosphinpalladium(II)acetat und Eisen(III)chlorid, besonders bevorzugt in Gegenwart von Tetrakis(triphenylphosphin)-palladium(O), umsetzt und die so erhaltene Verbindung der allgemeinen Formel I, in der wenigstens einer der Reste R¹ bis R⁴ für einen unsubstituierten oder wenigstens einfach substituierten Aryl- oder Heteroaryl-Rest steht, ggf. reinigt und/oder isoliert.

Verbindungen der allgemeinen Formel I, wobei wenigstens einer der Reste R¹, R², R³ und R⁴ für einen Aryl- oder Heteroaryl-Rest steht, der mit wenigstens einem Rest ausgewählt aus der Gruppe bestehend aus -C(=O)-H und -C(=O)-(C₁₋₅-Alkyl) substituiert ist, können mit wenigstens einer Verbindung der allgemeinen Formel H₂N(Cyclopropyl) vorzugsweise in wenigstens einem Reaktionsmedium ausgewählt aus der Gruppe bestehend aus Tetrahydrofuran, Dioxan, Dichlormethan, Methanol und Ethanol oder entsprechenden Mischungen, in Gegenwart wenigstens eines Reduktionsmittels, das polymergebunden sein kann, vorzugsweise in Gegenwart wenigstens eines Reduktionsmittels, das polymergebunden sein kann, ausgewählt aus der Gruppe bestehend aus Natriumtriacetoxyborhydrid, Natriumcyanoborhydrid und Natriumdiacetoxyborhydrid, oder in Gegenwart wenigstens eines Katalysators, bevorzugt in Gegenwart von Palladium auf Kohle oder in Gegenwart eines Rhodium-Katalysators, unter einer Wasserstoffatmosphäre, vorzugsweise bei einer Temperatur von -100 °C bis 200 °C, in wenigstens eine entsprechende Verbindung der allgemeinen Formel I, wobei wenigstens einer der Reste R¹, R², R³ und R⁴ für einen Aryl- oder Heteroaryl-Rest steht, der mit wenigstens einem Rest ausgewählt aus der Gruppe bestehend aus -CH(C₁₋₅-Alkyl)-NH-(Cyclopropyl) und -CH₂-NH-Cyclopropyl substituiert ist, überführt werden und diese ggf. gereinigt und/oder isoliert werden.

Verbindungen der allgemeinen Formel I, wobei wenigstens einer der Reste R¹, R², R³ und R⁴ für einen Aryl- oder Heteroaryl-Rest steht, der mit wenigstens einem Rest ausgewählt aus der Gruppe bestehend aus -CH(C₁₋₅-Alkyl)-NH-(Cyclopropyl) und - CH₂-NH-Cyclopropyl substituiert ist, können mit wenigstens einer Verbindung der allgemeinen Formel Z-S(=O)₂-C₁₋₅-Alkyl, worin Z für eine Abgangsgruppe, vorzugsweise für einen Halogen-Rest, besonders bevorzugt für ein Chloratom steht, ggf. in wenigstens einem Reaktionsmedium, vorzugsweise in wenigstens einem Reaktionsmedium ausgewählt aus der Gruppe bestehend aus Tetrahydrofuran, Dioxan, Dichlormethan, Diethylether, Toluol, Acetonitril und Dimethylformamid, oder entsprechenden Mischungen, ggf. in Gegenwart wenigstens einer Base, bevorzugt in Gegenwart wenigstens einer Base ausgewählt aus der Gruppe bestehend aus Triethylamin, Pyridin, Diisopropylethylamin, Dimethylaminopyridin, N-Methyl-morpholin und Diisopropylamin, vorzugsweise bei einer Temperatur von - 70 °C bis 200 °C, in wenigstens eine Verbindung der allgemeinen Formel I, wobei wenigstens einer der Reste R¹, R², R³ und R⁴ für einen Aryl- oder Heteroaryl-Rest steht, der mit wenigstens einem Rest ausgewählt aus der Gruppe bestehend aus -CH(C₁₋₅-Alkyl)-N(Cyclopropyl)-S(=O)₂-C₁₋₅-Alkyl und -CH₂-N(Cyclopropyl)-S(=O)₂-C₁₋₅-Alkyl substituiert ist, überführt werden und diese ggf. gereinigt und/oder isoliert werden.

Verbindungen der allgemeinen Formel I, wobei wenigstens einer der Reste R¹, R², R³ und R⁴ für einen Aryl- oder Heteroaryl-Rest steht, der mit wenigstens einem Rest ausgewählt aus der Gruppe bestehend aus -CH(C₁₋₅-Alkyl)-NH-(Cyclopropyl) und - CH₂-NH-Cyclopropyl substituiert ist, können mit wenigstens einer Verbindung der allgemeinen Formel Z-C(=O)-C₁₋₅-Alkyl oder Z-C(=O)-Phenyl, worin Z für eine Abgangsgruppe, vorzugsweise für einen Halogen-Rest, besonders bevorzugt für ein Chloratom steht, ggf. in wenigstens einem Reaktionsmedium, vorzugsweise in wenigstens einem Reaktionsmedium ausgewählt aus der Gruppe bestehend aus Tetrahydrofuran, Dioxan, Dichlormethan, Diethylether, Toluol, Acetonitril und Dimethylformamid, oder entsprechenden Mischungen, ggf. in Gegenwart wenigstens einer Base, bevorzugt in Gegenwart wenigstens einer Base ausgewählt aus der Gruppe bestehend aus Triethylamin, Pyridin, Diisopropylethylamin, Dimethylaminopyridin, N-Methyl-morpholin und Diisopropylamin, vorzugsweise bei einer Temperatur von - 70 °C bis 200 °C, in wenigstens eine Verbindung der allgemeinen Formel I, wobei wenigstens einer der Reste R¹, R², R³ und R⁴ für einen Aryl- oder Heteroaryl-Rest steht, der mit wenigstens einem Rest ausgewählt aus der Gruppe bestehend aus -CH(C₁₋₅-Alkyl)-N(Cyclopropyl)-C(=O)-C₁₋₅-Alkyl, -CH(C₁₋₅-Alkyl)-N(Cyclopropyl)-C(=O)-Phenyl, -CH₂-N(Cyclopropyl)-C(=O)-C₁₋₅-Alkyl und - CH₂-N(Cyclopropyl)-C(=O)-Phenyl substituiert ist, überführt werden und diese ggf. gereinigt und/oder isoliert werden.

Verbindungen der allgemeinen Formel I, wobei wenigstens einer der Reste R¹, R², R³ und R⁴ für einen Aryl- oder Heteroaryl-Rest steht, der mit wenigstens einem - C(=O)-O-C₁₋₅-Alkyl-Rest substituiert ist, können in einem Reaktionsmedium, bevorzugt in einem Reaktionsmedium ausgewählt aus der Gruppe bestehend aus Methanol, Ethanol, Wasser, Isopropanol und entsprechenden Mischungen, in Gegenwart wenigstens einer Base, bevorzugt in Gegenwart von Lithiumhydroxidmonohydrat, bei Temperaturen zwischen 0 °C und 50 °C zu Verbindungen der allgemeinen Formel I umgesetzt werden, wobei wenigstens einer der Reste R¹, R², R³ und R⁴ für einen Aryl- oder Heteroaryl-Rest steht, der mit wenigstens einem -C(=O)-OH-Rest substituiert ist.

Die in den vorstehend beschriebenen Umsetzungen zum Einsatz kommenden Chemikalien und Reaktionskomponenten sind käuflich erhältlich oder können jeweils nach üblichen, dem Fachmann bekannten Methoden hergestellt werden.

Die vorstehend beschriebenen Umsetzungen können ferner jeweils unter üblichen, dem Fachmann geläufigen Bedingungen, beispielsweise in Hinblick auf Druck, Temperatur, Schutzgasatmosphäre oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre oder Argonatmosphäre, durchgeführt werden.

Die erfindungsgemäßen substituierten Benzo[d]isoxazol-3-yl-amin-Verbindungen können sowohl in Form ihrer freien Basen, ihrer freien Säuren wie auch jeweils in Form entsprechender Salze, insbesondere physiologisch verträglicher Salze, isoliert werden.

Die freien Basen der jeweiligen erfindungsgemäßen substituierten Benzo[d]isoxazol-3-yl-amin-Verbindungen können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden.

Die freien Basen der jeweiligen erfindungsgemäßen substituierten Benzo[d]isoxazol-3-yl-amin-Verbindungen können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der erfindungsgemäßen substituierten Benzo[d]isoxazol-3-yl-amin-Verbindungen durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen substituierten Benzo[d]isoxazol-3-yl-amin-Verbindungen können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Benzo[d]isoxazol-3-yl-amin-Verbindungen nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Benzo[d]isoxazol-3-yl-amin-Verbindungen sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße substituierte Benzo[d]isoxazol-3-yl-amin-Verbindung sowie vorstehend definiert einschließlich Verbindungen; worin einer der Reste R⁵ und R⁶ für einen Rest ausgewählt aus der Gruppe bestehend aus einen linearen oder verzweigten, gesättigten oder ungesättigten, unsubstituierten oder wenigstens einfach substituierten aliphatischen Resten und Wasserstoff steht und der andere der Reste R⁵ und R⁶ für einen linearen oder verzweigten, gesättigten oder ungesättigten, unsubstituierten oder wenigstens einfach substituierten aliphatischen Rest steht und die anderen Reste die vorstehend angegebene Bedeutung haben; sowie ggf. einen oder mehrere pharmazeutisch verträgliche Hilfsstoffe.

Besonders bevorzugt ist ein Medikament enthaltend wenigstens eine erfindungsgemäße substituierte Benzo[d]isoxazol-3-yl-amin-Verbindung der allgemeinen Formel I-A, I-B, I-C und I-D, 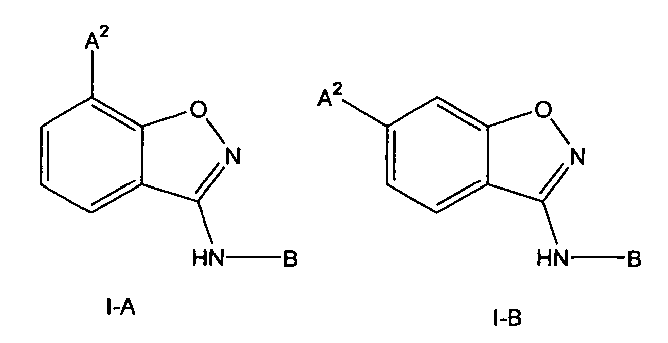 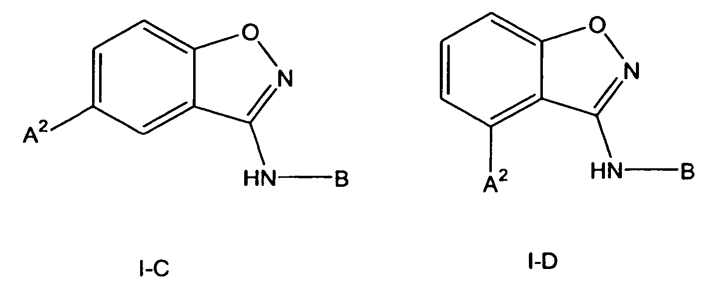 worin jeweils
- A²: für einen (Hetero)aryl-Rest steht, der ausgewählt ist aus der Gruppe bestehend aus Phenyl, 1-Naphthyl, 2-Naphthyl, Thiophenyl (Thienyl), Furanyl und Pyridinyl und der ggf. mit 1, 2, 3, 4 oder 5 Substituenten unabhängig voneinander ausgewählt aus der Gruppe bestehend F; Cl; Br; -CF₃; -OCF₃;-SCF₃; -SF₅; -CN; -OH; Methyl; Ethyl; n-Propyl; Isopropyl; n-Butyl; Isobutyl; sec-Butyl; tert-Butyl; Methoxy; Ethoxy; -NH₂; -N(CH₃)₂; -N(C₂H₅)₂; Phenyl; Phenoxy, Benzyl; Thiophenyl (Thienyl), Furanyl, Pyridinyl, C(=O)-H, -C(=O)-CH₃, -C(=O)-C₂H₅, -C(=O)-CH(CH₃)₂, -C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(Cyclopropyl)-CH₃, -C(=O)-N(Cyclopropyl)-C₂H₅, -C(=O)-N(Cyclopropyl)-CH(CH₃)₂, -C(=O)-N(Cyclopropyl)-C(CH₃)₃, -CH₂-NH-Cyclopropyl, -CH₂-N(Cyclopropyl)-S(=O)₂-CH₃, -CH₂-N(Cyclopropyl)-S(=O)₂-C₂H₅, -CH₂-N(Cyclopropyl)-C(=O)-CH₃, -CH₂-N(Cyclopropyl)-C(=O)-C₂H₅, -CH₂-N(Cyclopropyl)-C(=O)-CH(CH₃)₂, -CH₂-N(Cyclopropyl)-C(=O)-C(CH₃)₃ und-CH₂-N(Cyclopropyl)-C(=O)-Phenyl substituiert sein kann;
- B: für einen Alkyl-Rest ausgewählt aus der Gruppe bestehend aus Methyl; Ethyl; n-Propyl; Isopropyl; n-Butyl; Isobutyl; sec-Butyl, tert-Butyl, n-Pentyl und Neopentyl; für -C(=O)-Phenyl; -(CH₂)-Phenyl; -(CH₂)₂-Phenyl; -(CH₂)₃-Phenyl; -(CH₂)-Pyridinyl; -(CH₂)₂-Pyridinyl oder -(CH₂)₃-Pyridinyl steht; wobei die vorstehend genannten Phenyl und Pyridinyl-Reste ggf. mit 1, 2, 3, 4 oder 5 Substituenten unabhängig voneinander ausgewählt aus der Gruppe bestehend F; Cl; Br; -CF₃; -OCF₃; -SCF₃; -SF₅; -CN; -OH; Methyl; Ethyl; n-Propyl; Isopropyl; n-Butyl; Isobutyl; sec-Butyl; tert-Butyl; Methoxy; Ethoxy;-NH₂; -N(CH₃)₂; -N(C₂H₅)₂; Phenyl; Phenoxy, Benzyl; Thiophenyl (Thienyl), Furanyl und Pyridinyl substituiert sein können,
jeweils ggf. in Form entsprechender Salze, insbesondere der Hydrochlorid-Additionssalze, oder jeweils in Form entsprechender Solvate.

Ganz besonders bevorzugt ist ein Medikament enthaltend wenigstens eine erfindungsgemäße substituierte Benzo[d]isoxazol-3-yl-amin-Verbindung der allgemeinen Formel I-A, I-B, I-C und I-D, worin jeweils A² für einen der folgenden Reste A²¹ oder A²² steht: 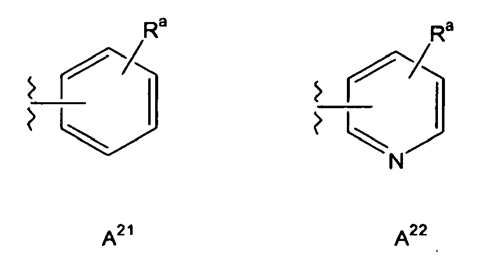 worin
- R^{a}: jeweils für einen Rest steht, der ausgewählt ist aus der Gruppe bestehend aus H, F, Cl, Br, I, -CN; -CF₃, -OCF₃, -SCF₃, -SF₅, -OH, -SH, Methyl; Ethyl; n-Propyl; Isopropyl; n-Butyl; Isobutyl; sec-Butyl; tert-Butyl; -OCH₃; -OC₂H₅; -S-CH₃, -S-C₂H₅, -C(=O)-H, -C(=O)-O-C₂H₅, -C(=O)-OH, -CH₂-NH-Cyclopropyl,-CH₂-N(Cyclopropyl)-S(=O)₂-CH₃, -CH₂-N(Cyclopropyl)-C(=O)-CH₃ und -CH₂-N(Cyclopropyl)-C(=O)-Phenyl steht und
- B: für einen Alkyl-Rest ausgewählt aus der Gruppe bestehend aus Methyl, Ethyl, n-Propyl, Isopropyl, n-Butyl, Isobutyl, sec-Butyl, tert-Butyl, n-Pentyl und Neopentyl steht;
oder für einen der folgenden Reste B¹, B² oder B³ steht, 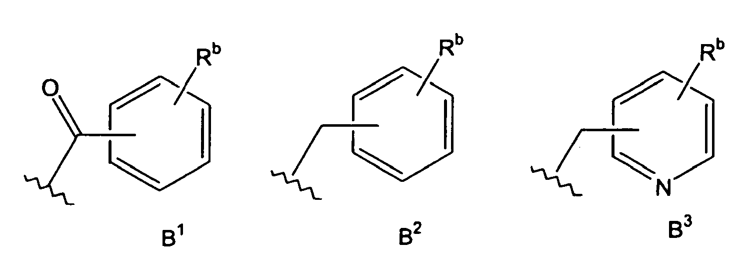 worin
- R^{b}: jeweils für einen Rest steht, der ausgewählt ist aus der Gruppe bestehend aus H, F, Cl, Br, I, -CN; -CF₃, -OCF₃, -SCF₃, -SF₅, -OH, -SH, Methyl; Ethyl; n-Propyl; Isopropyl; n-Butyl; Isobutyl; sec-Butyl; tert-Butyl; -OCH₃; -OC₂H₅; -S-CH₃ und -S-C₂H₅.

Am meisten bevorzugt ist ein Arzneimittel enthaltend wenigstens eine erfindungsgemäße Verbindung wie vorstehend definiert und/oder wenigstens eine Verbindung ausgewählt aus der Gruppe bestehend aus
[35] 2-(3-(Neopentylamino)benzo[d]isoxazol-5-yl)benzoesäure,
[36] Ethyl 4-(3-(neopentylamino)benzo[d]isoxazol-5-yl)benzoat,
[37] N-Neopentyl-5-(3-(trifluormethyl)phenyl)benzo[d]isoxazol-3-amin,
[38] 3-(3-(Neopentylamino)benzo[d]isoxazol-5-yl)benzonitril,
[39] N-Methyl-3-(3-(neopentylamino)benzo[d]isoxazol-5-yl)benzamid,
[40] 3-(3-(Neopentylamino)benzo[d]isoxazol-5-yl)benzoesäure,
[41] Ethyl 3-(3-(neopentylamino)benzo[d]isoxazol-5-yl)benzoat,
[42] N-Cyclopropyl-N-(2-methoxy-5-(3-(neopentylamino)benzo[d]isoxazol-5-yl)benzyl)methansulfonamid,
[43] N-Cyclopropyl-N-(2-methoxy-5-(3-(neopentylamino)benzo[d]isoxazol-5-yl)benzyl)acetamid,
[44] N-Cyclopropyl-N-(2-fluor-5-(3-(neopentylamino)benzo[d]isoxazol-5-yl)benzyl)acetamid,
[45] 5-(3-((Cyclopropylamino)methyl)-4-methoxyphenyl)-N-neopentylbenzo[d]isoxazol-3-amin,
[46] 5-(3-((Cyclopropylamino)methyl)-4-fluorphenyl)-N-neopentylbenzo[d]isoxazol-3-amin,
[47] 2-Methoxy-5-(3-(neopentylamino)benzo[d]isoxazol-5-yl)benzaldehyd,
[48] 2-Fluor-5-(3-(neopentylamino)benzo[d]isoxazol-5-yl)benzaldehyd,
[49] N-Cyclopropyl-N-(3-(3-(neopentylamino)benzo[d]isoxazol-5-yl)benzyl)benzamid,
[50] N-Cyclopropyl-N-(3-(3-(neopentylamino)benzo[d]isoxazol-5-yl)benzyl)acetamid,
[51] 5-(3-((Cyclopropylamino)methyl)phenyl)-N-neopentylbenzo[d]isoxazol-3-amin und
[52] 3-(3-(Neopentylamino)benzo[d]isoxazol-5-yl)benzaldehyd;
sowie jeweils deren entsprechende Salze, insbesondere deren Hydrochlorid-Additionssalze, und ggf. jeweils deren entsprechende Solvate.

Diese erfindungsgemäßen Arzneimittel eignen sich insbesondere zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1(VR1/TRPV1)-Stimulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Besonders bevorzugt eignet sich das erfindungsgemäße Arzneimittel zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz, und/oder Harninkontinenz.

Ein weiterer Gegenstand der vorliegenden Erfindung ist die Verwendung wenigstens einer erfindungsgemäßen substituierten Benzo[d]isoxazol-3-yl-amin-Verbindung sowie ggf. eines oder mehrerer pharmazeutisch verträglicher Hilfsstoffe zur Herstellung eines Arzneimittels zur Vanilloid-Rezeptor 1-(VR1/TRPV1)-Regulation, vorzugsweise zur Vanilloid-Rezeptor 1-(VR1/TRPV1)-Hemmung und/oder zur Vanilloid-Rezeptor 1 (VR1/TRPV1)-Stimulation.

Bevorzugt ist die Verwendung wenigstens einer erfindungsgemäßen substituierten Benzo[d]isoxazol-3-yl-amin-Verbindung sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens einer erfindungsgemäßen substituierten Benzo[d]isoxazol-3-yl-amin-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

Ganz besonders bevorzugt ist die Verwendung wenigstens einer erfindungsgemäßen substituierten Benzo[d]isoxazol-3-yl-amin-Verbindung sowie ggf. eines oder mehrerer pharmazeutisch verträglicher Hilfsstoffe zur Herstellung eines Arzneimittels zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Harninkontinenz; einer überaktiven Blase (overactive bladder, OAB); Medikamentenabhängigkeit; Medikamentenmißbrauch; Entzugserscheinungen bei Medikamentenabhängigkeit; Toleranzentwicklung gegenüber Medikamenten, vorzugsweise Toleranzentwicklung gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit.

Noch weiter bevorzugt ist die Verwendung wenigstens einer erfindungsgemäßen substituierten Benzo[d]isoxazol-3-yl-amin-Verbindung sowie ggf. eines oder mehrerer pharmazeutisch verträglicher Hilfsstoffe zur Herstellung eines Arzneimittels zur Behandlung und/oder Prophylaxe von Schmerz, vorzugsweise ausgewählt aus der Gruppe bestehend aus akutem Schmerz, chronischem Schmerz, neuropathischem Schmerz und visceralem Schmerz, und/oder Harninkontinenz.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden. Neben wenigstens einer erfindungsgemäßen substituierten Benzo[d]isoxazol-3-yl-amin-Verbindung enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten Benzo[d]isoxazol-3-yl-amin-Verbindungen können in einem Depot, in gelöster Form oder in einem Pflaster, gegebenenfalls unter Zusatz von die Hautpenetration fördernden Mitteln, als geeignete perkutane Applikationszubereitungen vorliegen. Oral oder perkutan anwendbare Zubereitungsformen können die jeweilige erfindungsgemäße substituierte Benzo[d]isoxazol-3-yl-amin-Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remingtons Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen substituierten Benzo[d]isoxazol-3-yl-amin-Verbindung kann variieren und ist beispielsweise abhängig vom Gewicht oder Alter des Patienten sowie von der Applikationsart, der Indikation und dem Schweregrad der Erkrankung. Üblicherweise werden 0,005 bis 100 mg/kg, vorzugsweise 0,05 bis 75 mg/kg Körpergewicht des Patienten wenigstens einer solchen erfindungsgemäßen Verbindung appliziert.

### Pharmakologische Methoden:

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit
10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in einen kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die DRGs werden jeweils mit 500 µL Komplett-Medium versetzt.

Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt.

Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

Ausgehend von der prozentualen Verdrängung durch unterschiedliche Konzentrationen der zu prüfenden Verbindungen der allgemeinen Formel I werden IC₅₀ Hemmkonzentrationen berechnet, die eine 50-prozentige Verdrängung des Capsaicin bewirken.

### II. Funktionelle Untersuchungen am Vanilloid Rezeptor (VR1)

Die agonistische bzw. antagonistische Wirkung der zu untersuchenden Substanzen auf den Vanilloid Rezeptor (VR1) kann auch mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden, Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37 °C und 5 % CO₂ in einem Kulturmedium (Nutrient Mixture Ham's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0,01 Vol-%, Molecular Probes in HBSS (Hank's buffered saline solution), Gibco Invitrogen GmbH, Karlsruhe, Deutschland) für 30 Minuten bei 37 °C inkubiert. Anschließend werden die Platten 3 mal mit HBSS-Puffer gewaschen und nach einer weiteren Inkubation von 15 Minuten bei Raumtemperatur zur Ca²⁺-Messung im FLIPR eingesetzt. Die Ca²⁺-abhängige Fluoreszenz wird dabei vor und nach Zugabe der zu untersuchenden Substanzen gemessen (Wellenlänge λₑₓ=488 nm, λₑₘ= 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺⁻-Signal nach Zugabe von 10 µM Capsaicin). Nach 5 Minuten Inkubation werden 100 nM Capsaicin appliziert und ebenfalls der Einstrom von Ca²⁺ ermittelt.

Desensitisierende Agonisten und Antagonisten führten zu einer Unterdrückung des Ca²⁺-Einstroms. Es werden % Inhibierung im Vergleich zu der maximal erreichbaren Inhibierung mit 10 µM Capsazepin berechnet.

### III. Formalin-Test an der Maus

Die Untersuchung zur Bestimmung der antinociceptiven Wirkung der erfindungsgemäßen substituierten Benzo[d]isoxazol-3-yl-amin-Verbindungen wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen substituierten Benzo[d]isoxazol-3-yl-amin-Verbindungen werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen substituierten Benzo[d]isoxazol-3-yl-amin-Verbindungen vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wäßrige Lösung) in die dorsale Seite der rechten Hinterpfote, so dass bei freibeweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfaßt. Die Quantifizierung des Schmer-zverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wäßrige Natriumchlorid-Lösung) vor Formalinapplikation erhalten.

Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

Im Folgenden wird die Erfindung mit Hilfe einiger Beispiele erläutert. Diese Erläuterungen sind lediglich beispielhaft und schränken den allgemeinen Erfindungsgedanken nicht ein.

### Beispiele:

Die Ausbeuten der hergestellten Verbindungen sind nicht optimiert.

Alle Temperaturen sind unkorrigiert.

### Abkürzungen

- aq.: wäßrig
- APCI: atmospheric pressure chemical ionisation
- Äquiv.: Stoffmengenäquivalente
- DCM: Dichlormethan
- DMF: Dimethylformamid
- DMSO: Dimethylsulfoxid
- EtOAc: Ethylacetat
- EtOH: Ethanol
- ges.: gesättigt
- h: Stunden
- min: Minuten
- NMR: Kernresonanzspektroskopie
- MeOH: Methanol
- RT: Raumtemperatur
- THF: Tetrahydrofuran

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik erfolgte durch Massenspektroskopie und NMR.

### Allgemeine Vorschrift zur Herstellung des Benzisoxazol-Grundgerüstes:

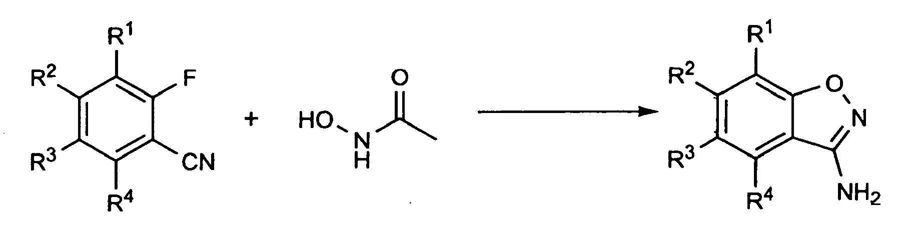

Das Grundgerüst der erfindungsgemäßen Benzisoxazole wurde analog der Vorschrift von Palermo (M. G. Palermo, Tetrahedron Lett. 1996; 37; 17; 2885-2886) hergestellt. Die entsprechende Beschreibung wird hiermit als Referenz eingeführt und gilt somit als Teil der vorliegenden Offenbarung. Abweichend von der genannten Vorschrift erfolgt die Aufreinigung der Benzisoxazol-Verbindungen zum Teil durch Fällen des entsprechenden HCl-Salzes.

### Durchführung:

In einem Dreihalskolben wurde Acethydroxamsäure (1,1 Äquiv.) in DMF (1,45 mL / mmol Acethydroxamsäure) suspendiert. Unter Inertgas wurde Kalium-*t*-Butylat (1,1 Äquiv.) zugegeben. Die Mischung wurde 30 min. bei Raumtemperatur gerührt und anschließend mit dem ggf. substituierten 2-Fluor-benzonitril (1 Äquiv.) versetzt. Der Reaktionsansatz wurde auf 50°C erwärmt und 1 h bei dieser Temperatur gerührt. Nach dem Abkühlen wurde die Reaktionsmischung auf eine Mischung (1,8 mL / mmol Acethydroxamsäure) aus gleichen Volumenanteilen gesättigter NaCl-Lösung und Essigsäureethylester gegeben und 30 min gut gerührt. Die Phasen wurden getrennt und die wäßrige Phase 3mal mit Essigester (je 0,8 mL / mmol Acethydroxamsäure) extrahiert.
Die organischen Phasen wurden vereinigt und 3mal mit gesättigter NaCl- Lösung (je 0,8 mL / mmol Acethydroxamsäure) gewaschen und anschließend über Magnesiumsulfat getrocknet.
Das Magnesiumsulfat wurde abfiltriert und das Filtrat zunächst am Rotationsverdampfer und anschließend an der Ölpumpe aufkonzentriert.

Zur weiteren Aufreinigung war es in einigen Fällen vorteilhaft das erhaltene Hydrochlorid zu fällen.

Hierzu wurde der Rückstand in Methylethylketon (8,7 mL / g Rückstand) gelöst. Nach Zugabe von Wasser (0,1 mL / g Rückstand) wurde unter langsamen Rühren und EisWasser- Kühlung Trimethylchlorsilan (0,7 mL / g Rückstand) zu getropft. Der Kolben wurde über Nacht in den Kühlschrank gestellt, der entstandene Niederschlag abfiltriert und im Exikkator mit Hilfe von Phosphorpentoxid als Trockenmittel getrocknet.

Die Verbindungen A und B wurden gemäß der vorstehenden Vorschrift hergestellt:

| | Benzisoxazol | Ausbeute |
|---|---|---|
| A | 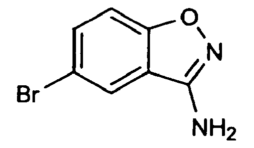 | 42 % |
| B | 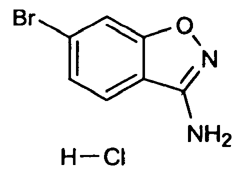 | 42 % |

### Allgemeine Vorschrift zur reduktiven Aminierung des Amino-substituierten Benzisoxazol-Grundgerüstes:

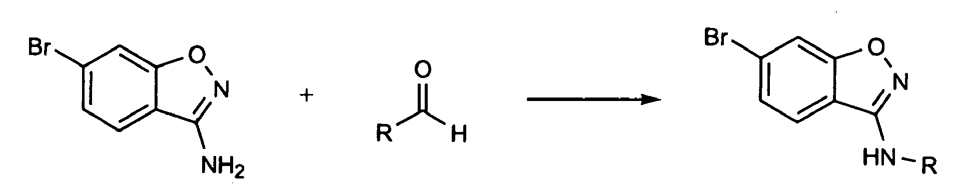

### Durchführung

Das jeweilige Benzisoxazol (3,71 mmol, 1 Äquiv.) wurde in DCM (48 ml) gelöst, mit dem entsprechenden Aldehyd (3,71 mmol, 1 Äquiv.) versetzt und 1 h bei Raumtemperatur gerührt. Anschließend wurden Triethylsilan (3,71 mmol, 1 Äquiv.) und Trifluoressigsäure (11,13 mmol, 3 Äquiv.) zu getropft und die Mischung unter Inertgas 15 - 20 h unter Rückfluß erhitzt. Nach dem Abkühlen wurde der Ansatz mit ges. NaHCO₃-Lsg. auf pH 8 - 9 gestellt und die wäßrige Phase 4mal mit DCM extrahiert. Die vereinigten organischen Phasen wurde über MgSO₄ getrocknet und eingeengt. Das erhaltene Rohprodukt wurde durch Flash-Chromatographie (Diethylether/Hexan) auf gereinigt.

Man erhält so beispielsweise die folgenden Vorstufen (Ausbeute 86 % bzw. 82 %):

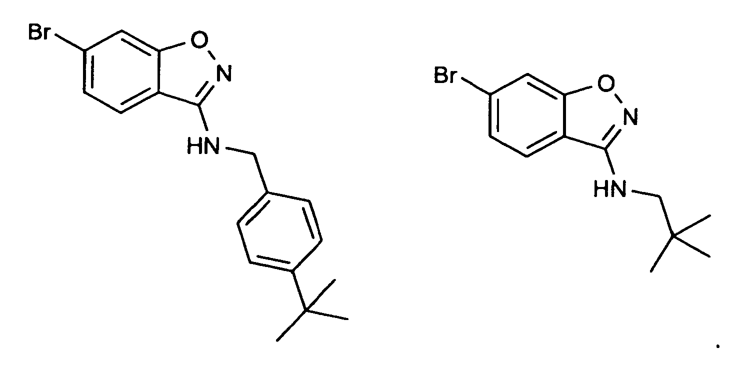

### Allgemeine Vorschrift zur Acylierung des Amino-substituierten Benzisoxazol-Grundgerüstes:

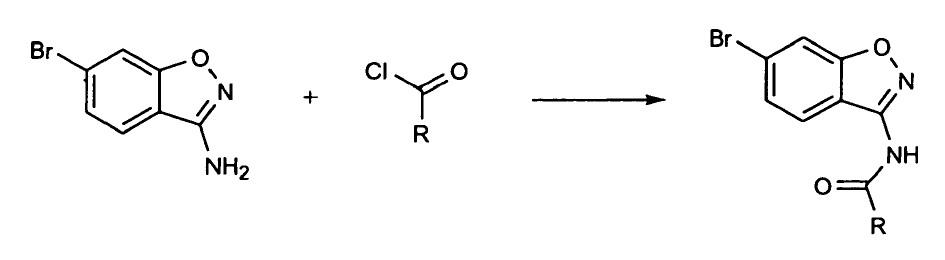

### Durchführung

Das jeweilige Benzisoxazol (23,5 mmol) wurde in Pyridin (28 ml) gelöst und im Eisbad auf 0 °C abgekühlt. Es entstand eine weiße Suspension, zu der das jeweilige Säurechlorid (47 mmol) zu getropft wurde. Anschließend wurde 4h bei RT gerührt. Nach Ende der Reaktion wurde der Ansatz mit DCM (200 ml) verdünnt und dreimal mit (100 ml) 5 % HCl-Lsg. (100 ml) extrahiert. Die organische Phase wurde mit etwas ges. NaHCO₃-Lsg. und ges. NaCl gewaschen, über Na₂SO₄ getrocknet und eingeengt.

Zur Aufreinigung wurde das Rohprodukt mit Ether ausgekocht, der Rückstand nach dem Abkühlung filtriert. Anschließend wurde die Mutterlauge auf ca. 20 ml eingeengt und der Rückstand ebenfalls filtriert.

### Allgemeine Vorschrift zur Herstellung arylsubstituierter Benzisoxazol-Verbindungen:

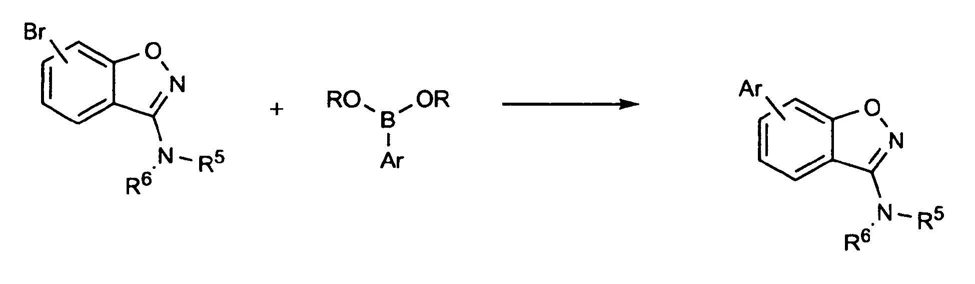

### Durchführung:

Das jeweilige Benzisoxazol (0,96 mmol, 1 Äquiv.) wurde in einem Gemisch aus Dioxan (6,5 ml), Wasser (2,6 ml) und Ethanol (1,9 ml) gelöst und mit dem entsprechenden Boronsäurederivat (1,34 mmol, 1,4 Äquiv.) versetzt. Anschließend wurde Natriumcarbonat (3,16 mmol, 3,3 Äquiv) zugegeben. Die Mischung wurde unter Inertgas gesetzt und mit Tetrakis(triphenylphosphin)-palladium(0) (0,02 mmol, 0,02 Äquiv.) versetzt. Anschließend wurde unter Inertgas 16 h unter Rückfluß erhitzt. Nach dem Abkühlen wurde mit Wasser verdünnt und dreimal mit je 20 ml Toluol extrahiert. Die vereinigten organischen Phasen wurden dreimal mit je 15 ml KOH-Lösung (0,5 N) gewaschen, über Natriumsulfat getrocknet und eingeengt. Das erhaltene Rohprodukt wurde durch Flash-Chromatographie (Ether/Hexan) auf gereinigt.

Gemäß dieser Vorschrift wurden die folgenden Verbindungen erhalten:

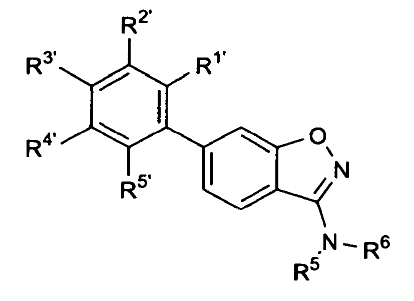

| | R^{1'} | R^{2'} | R^{3'} | R^{4'} | R^{5'} | R⁵ | R⁶ |
|---|---|---|---|---|---|---|---|
| **1** | H | H | H | Cl | H | H | Bn |
| **2** | H | H | Cl | H | H | H | Bn |
| **3** | H | H | CH₃ | H | H | H | Bn |
| **4** | H | H | H | H | OCH₃ | H | Bn |
| **5** | H | H | H | Cl | H | H | C(=O)Ph |
| **6** | H | H | Cl | H | H | H | C(=O)Ph |
| **7** | H | H | CH₃ | H | H | H | C(=O)Ph |
| **8** | H | H | H | H | OCH₃ | H | C(=O)Ph |

wobei Bn = Benzyl und Ph = Phenyl

### Synthese von Beispielverbindung 52:

### 3-(3-(Neopentylamino)benzo[d]isoxazol-5-yl)benzaldehyd

Zu einer Suspension von 5-Brom-N-neopentylbenzo[d]isoxazol-3-amin (1.7 g, 6.0 mmol, 1 Äquiv.) und 3-Formylphenylboronsäure (1.1 g, 7.2 mmol, 1.2 Äquiv.) in Toluol (50 mL) wurde EtOH (15 mL) gegeben, gefolgt von aq. Natriumcarbonat-Lsg. (2.5 M, 15 mL) und Tetrakis(triphenylphosphin)-palladium(0) (0.066 g, 0.057 mmol, 0.01 Äquiv.). Das Reaktionsgemisch wurde anschließend 5 Stunden zum Rückfluß erhitzt und nach Abkühlen auf RT eingeengt. Der Rückstand wurde in EE (150 mL) aufgenommen und mit Wasser (2 x 20 mL) und ges. aq. Natriumchlorid-Lösung (1 x 20 mL) gewaschen, getrocknet (MgSO₄) und das Lösungsmittel im Vakuum entfernt. Nach säulenchromatographischer Reinigung (Hexan/ EE; 10:1; 5:1; 3:1) erhielt man das gewünschte Produkt (1.18 g, 64 %).

### Synthese von Beispielverbindung 35:

### 2-(3-(Neopentylamino)benzo[d]isoxazol-5-yl)benzoesäure

5-Brom-N-neopentylbenzo[d]isoxazol-3-amin (0.30 g, 1.1 mmol, 1 Äquiv.) und 2-Methoxycarbonylphenylboronsäure (0.23 g, 1.27 mmol, 1.2 Äquiv.) wurden in DMF (9 mL) gelöst. Aq. Na₂CO₃-Lsg. (0.54 g / 3.6 mL H₂O) wurde zugegeben und das Gemisch 2 min bei RT gerührt. Anschließend wurde Bis(triphenylphosphin)-palladium(II)-chlorid (0.041 g, 0.053 mmol, 0.05 Äquiv.) zugegeben und das Reaktionsgemisch in der Mikrowelle (CEM Explorer) in einem verschlossenem 10 mL Röhrchen bei 200 Watt 5 min auf 200 °C erhitzt. Das auf RT abgekühlte Reaktionsgemisch wurde in EtOAc (100 mL) aufgenommen und mit Wasser (2 x 20 mL) und ges. aq. NaCl-Lsg. (1 x 20 mL) gewaschen, getrocknet (MgSO₄) und anschließend das Lösungsmittel im Vakuum entfernt. Nach säulenchromatographischer Reinigung (Hexan/ EtOAc; 5:1) erhielt man das gewünschte Produkt (0.1 g, 29 %).

Die Beispielverbindung 36: Ethyl 4-(3-(neopentylamino)benzo[d]isoxazol-5-yl)benzoat; 56 % Ausbeute
Beispielverbindung 37: (N-Neopentyl-5-(3-(trifluoromethyl)phenyl)benzo[d]isoxazol-3-amin; 54 % Ausbeute
Beispielverbindung 38: (3-(3-(Neopentylamino)benzo[d]isoxazol-5-yl)benzonitrile; 62 % Ausbeute;
Beispielverbindung 41: Ethyl 3-(3-(neopentylamino)benzo[d]isoxazol-5-yl)benzoat; 65 % Ausbeute;
Beispielverbindung 48: (2-Fluoro-5-(3-(neopentylamino)benzo[d]isoxazol-5-yl)benzaldehyde; 54 % Ausbeute
und Beispielverbindung 47: 2-Methoxy-5-(3-(neopentylamino)benzo[d]isoxazol-5-yl)benzaldehyde; 50 % Ausbeute wurden analog hergestellt.

### Automatisierte Synthese:

### Die Herstellung arylsubstituierter Benzisoxazol-Verbindungen erfolgte auch durch automatisierte Synthese:

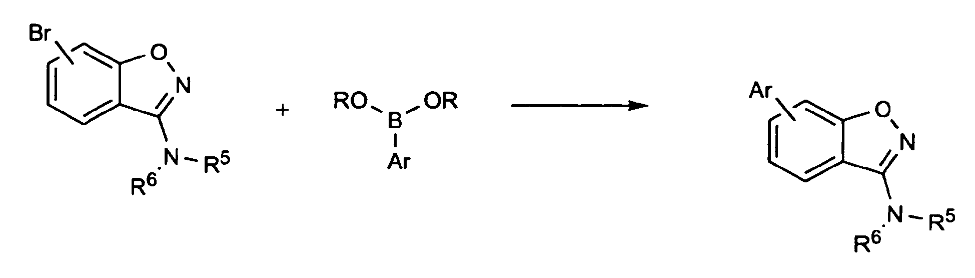

In ein trockenes Gewindeglas mit Septumkappe wurden bei RT nacheinander Benzisoxazol-Lösung (2 ml, 0,05 M in Dioxan), Boronsäure-Lösung (1,25 ml, 0,2 M in Ethanol), Natriumcarbonatlösung (0,3 ml, 1,2 M in Wasser) und frisch angesetzte Tetrakis(triphenylphosphin)-palladium(0)-Lösung (0,3 ml, 0,04 M in Dioxan) pipettiert. Die Reaktionslösung wurde unter Stickstoff und Rückfluß im Heidolph Synthesis 1 für 16 h auf 100°C erhitzt und geschüttelt.
Nach Reaktionsende wurden 2 ml Wasser und 3 ml Toluol zugegeben, gemischt und die Phasen getrennt. Die wäßrige Phase wurde noch einmal mit 3 ml Toluol extrahiert und die vereinigten organischen Phasen mit 3 ml KOH (0,5 N) gewaschen. Die vereinigten organischen Phasen wurden über Magnesiumsulfat getrocknet und in der GeneVac eingeengt.

Gemäß dieser Vorschrift wurden die folgenden Verbindungen erhalten:

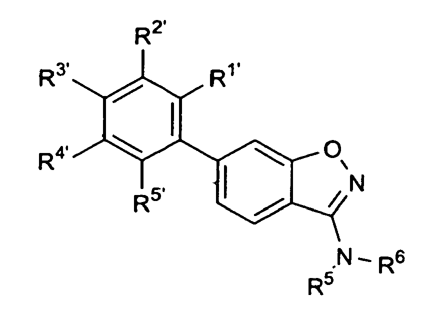

| | R^{1'} | R^{2'} | R^{3'} | R^{4'} | R^{5'} | R⁵ | R⁶ |
|---|---|---|---|---|---|---|---|
| **13** | H | H | Cl | H | H | H | CH₂-2-Pyridyl |
| **14** | H | H | H | H | H | H | CH₂-2-Pyridyl |
| **15** | H | H | H | H | CH₃ | H | CH₂-2-Pyridyl |
| **16** | H | H | Cl | H | H | H | CH₂-3-Pyridyl |
| **17** | H | H | H | H | H | H | CH₂-3-Pyridyl |
| **18** | H | H | H | H | CH₃ | H | CH₂-3-Pyridyl |
| **19** | H | H | H | H | OH | H | CH₂-3-Pyridyl |
| **20** | H | H | Cl | H | H | H | CH₂-4-Pyridyl |
| **21** | H | H | H | H | H | H | CH₂-4-Pyridyl |
| **22** | H | H | H | H | CH₃ | H | CH₂-4-Pyridyl |
| **23** | H | H | Cl | H | H | H | CH₂-(4-CN-Phenyl) |
| **24** | H | H | H | H | H | H | CH₂-(4-CN-Phenyl) |
| **25** | H | H | H | H | CH₃ | H | CH₂-(4-CN-Phenyl) |
| **26** | H | H | H | H | OH | H | CH₂-(4-CN-Phenyl) |
| **27** | H | H | Cl | H | H | H | CH₂-(3-CH₃-Phenyl) |
| **28** | H | H | H | H | H | H | CH₂-(3-CH₃-Phenyl) |
| **29** | H | H | H | H | CH₃ | H | CH₂-(3-CH₃-Phenyl) |
| **30** | H | H | H | H | OH | H | CH₂-(3-CH₃-Phenyl) |
| **31** | H | H | Cl | H | H | H | CH₂-(3-Cl-Phenyl) |
| **32** | H | H | H | H | H | H | CH₂-(3-Cl-Phenyl) |
| **33** | H | H | H | H | CH₃ | H | CH₂-(3-Cl-Phenyl) |
| **34** | H | H | H | H | OH | H | CH₂-(3-Cl-Phenyl) |

### Allgemeine Vorschrift zur Herstellung heteroarylsubstituierter Benzisoxazol-Verbindungen:

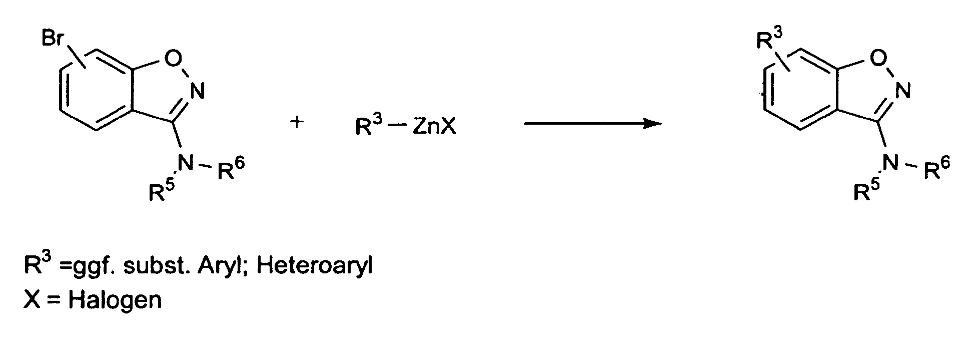

### Durchführung

Das jeweilige Benzisoxazol (0,695 mmol, 1 Äquiv.) wurde in THF (20 ml) gelöst und unter Inertgas mit Tetrakis(triphenylphosphin)-palladium(0) (0,007 mmol, 0,01 Äquiv.) versetzt. Anschließend wurde das Organozink-Reagenz (1,04 mmol, 1,5 Äquiv.; Lsg. In THF) langsam zugegeben. Dann wurde unter Inertgas 2 - 3 h unter Rückfluß erhitzt. Nach dem Abkühlen wurde mit Wasser verdünnt und mit Essigester extrahiert. Die vereinigten organischen Phasen wurden mit ges. NaCl-Lösung gewaschen, über Magnesiumsulfat getrocknet und eingeengt. Das erhaltene Rohprodukt wurde durch Flash-Chromatographie (Diethylether/Hexan) auf gereinigt.

Gemäß dieser Vorschrift wurden die folgenden Verbindungen erhalten:

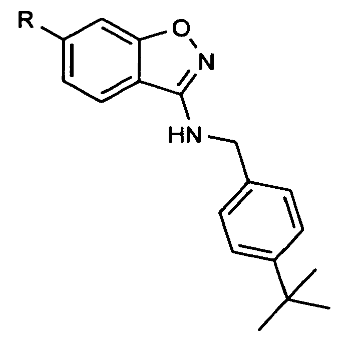

| | R | Ausbeute |
|---|---|---|
| 9 | 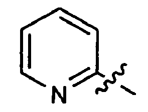 | 55 % |
| 10 | 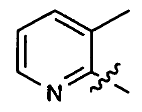 | 77 % |
| 11 | 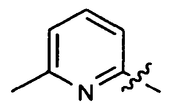 | 44 % |
| 12 | 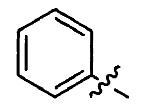 | 21 % |

### Synthese von Beispielverbindung 51:

### 5-(3-((Cyclopropylamino)methyl)phenyl)-N-neopentylbenzo[d]isoxazol-3-amin

Zu einer Lösung von 3-(3-(Neopentylamino)benzo[d]isoxazol-5-yl)benzaldehyd (Beispielverbindung 52) (0.92 g, 2.98 mmol, 1 Äquiv.) in THF (50 mL) wurde Cyclopropylamin (1.04 mL, 14.92 mmol, 5 Äquiv.) gegeben und das Gemisch anschließend mit Natriumcyanoborhydrid (3.16 g, 14.92 mmol, 5 Äquiv.) versetzt. Die Suspension wurde 3 Tage bei RT gerührt, anschießend durch Zusatz von ges. aq.

Natriumhydrogencarbonat-Lsg. (ca. 5 mL) hydrolysiert und das Lösungsmittel im Vakuum entfernt. Der Rückstand wurde in EtOAc (10 mL) aufgenommen und mit ges. aq. Natriumhydrogencarbonat-Lsg. (2 x 15 mL) und ges. aq. NaCl-Lösung (1 x 15 mL) extrahiert, getrocknet (MgSO₄) und das Lösungsmittel im Vakuum entfernt. Nach säulenchromatographischer Reinigung (EtOAc / MeOH; 20 : 1) erhielt man das gewünschte Produkt (0.85 g, 82 %).

Die Beispielverbindung 46: (5-(3-((Cyclopropylamino)methyl)-4-fluorophenyl)-N-neopentylbenzo[d]isoxazol-3-amin; 36 % Ausbeute und Beispielverbindung 45: (5-(3-((Cyclopropylamino)methyl)-4-methoxyphenyl)-N-neopentylbenzo[d]isoxazol-3-amin; 85 % wurden analog hergestellt.

### Synthese von Beispielverbindung 42:

### N-Cyclopropyl-N-(2-methoxy-5-(3-(neopentylamino)benzo[d]isoxazol-5-yl)benzyl)methansulfonamid

Zu einer Lösung von 5-(3-((Cyclopropylamino)methyl)-4-methoxyphenyl)-N-neopentylbenzo[d]isoxazol-3-amin (Beispielverbindung 45) (0.25 g, 0.66 mmol, 1 Äquiv.) in DCM (10 mL) wurde Triethylamin (0.14 mL, 0.99 mmol, 1.5 Äquiv.) gegeben und das Gemisch anschließend auf -70 °C gekühlt. Methansulfonsäurechlorid (0.06 mL, 0.79 mmol, 1.2 Äquiv.) wurde hinzu getropft und das Gemisch langsam auf RT erwärmt und 15 Stunden gerührt. Das Reaktionsgemisch wurde mit DCM (50 mL) verdünnt und mit ges. aq. Natriumhydrogencarbonat-Lsg. (1 x 10 mL) und ges. aq. NaCl-Lösung (1 x 10 mL) extrahiert, getrocknet (MgSO₄) und das Lösungsmittel im Vakuum entfernt. Nach säulenchromatographischer Reinigung (EtOAc / DCM; 1 : 1) erhielt man das gewünschte Produkt (0.21 g, >99 %).

### Synthese von Beispielverbindung 49:

### N-Cyclopropyl-N-(3-(3-(neopentylamino)benzo[d]isoxazol-5-yl)benzyl)benzamid

Zu einer Lösung von 5-(3-((Cyclopropylamino)methyl)phenyl)-N-neopentylbenzo[d]isoxazol-3-amin (Beispielverbindung 51) (0.30 g, 0.86 mmol, 1 Äquiv.) in DCM (10 mL) wurde Triethylamin (0.18 mL, 1.29 mmol, 1,5 Äquiv.) gegeben und das Gemisch anschliessend auf -70 °C gekühlt. Benzoylchlorid (0.12 mL, 1.03 mmol, 1.2 Äquiv.) wurde hinzu getropft und das Gemisch langsam auf RT erwärmt und 15 Stunden gerührt. Das Reaktionsgemisch wurde mit DCM (80 mL) verdünnt und mit ges. aq. Natriumhydrogencarbonat-Lsg. (1 x 15 mL) und ges. aq. NaCl-Lösung (1 x 15 mL) extrahiert, getrocknet (MgSO₄) und das Lösungsmittel im Vakuum entfernt. Nach säulenchromatographischer Reinigung (EtOAc / Hexan; 10 : 1) erhielt man das gewünschte Produkt (0,30 g, 77 %).

Die Beispielverbindung 43: (N-Cyclopropyl-N-(2-methoxy-5-(3-(neopentylamino)benzo[d]isoxazol-5-yl)benzyl)acetamid; 70 % Ausbeute; Beispielverbindung 44 (N-Cyclopropyl-N-(2-fluoro-5-(3-(neopentylamino)benzo[d]isoxazol-5-yl)benzyl)acetamid; 50 % Ausbeute und Beispielverbindung 50 (N-Cyclopropyl-N-(3-(3-(neopentylamino)benzo[d]isoxazol-5-yl)benzyl)acetamid; 64 % Ausbeute wurden analog hergestellt.

### Synthese von Beispielverbindung 40:

### 3-(3-(Neopentylamino)benzo[d]isoxazol-5-yl)benzoesäure

Eine Lösung von Ethyl-3-(3-(neopentylamino)benzo[d]isoxazol-5-yl)benzoat (0.38 g, 1.08 mmol, 1 Äquiv.) in Methanol (4 mL) wurde auf 0 °C gekühlt und eine wässrige Lösung LiOH*H₂O (0.16 g, 2.16 mmol, 2 Äquiv. / in H₂O (1.5 mL)) langsam zugegeben. Das Reaktionsgemisch wurde 15 h bei RT gerührt, und anschließend im Vakuum eingeengt. Der Rückstand wurde in EtOAc (100 mL) aufgenommen, mit 10%-iger aq. Zitronensäure-Lsg. (2 x 20 mL) gewaschen, getrocknet (MgSO₄) und das Lösungsmittel im Vakuum entfernt. So erhielt man das gewünschte Produkt (0.34 g, 97 %).

### Pharmakologische Daten:

Die untersuchten erfindungsgemäßen Benzo[d]isoxazol-3-yl-amin-Verbindungen zeigen eine ausgezeichnete Affinität zu dem Vanilloid-Rezeptor 1 (VR1/TRPV1-Rezeptor). Die Bestimmung erfolgte gemäß der obenstehend beschriebenen Methode I.

| Verbindung | rVR1 [10 µM] Stimulation | rVR1 [10 µM] Inhibierung |
|---|---|---|
| 9 | 0 | 65 |
| 10 | 29 | 99 |

wobei
Verbindung 9 (4-tert-Butyl-benzyl)-(6-pyridin-2-yl-benzo[d]isoxazol-3-yl)-amin und
Verbindung 10 (4-tert-Butyl-benzyl)-[6-(3-methyl-pyridin-2-yl)-benzo[d]isoxazol-3-yl]-amin ist.

## Patentansprüche

1. Substituierte Benzo[d]isoxazol-3-yl-amin-Verbindungen der allgemeinen Formel I, 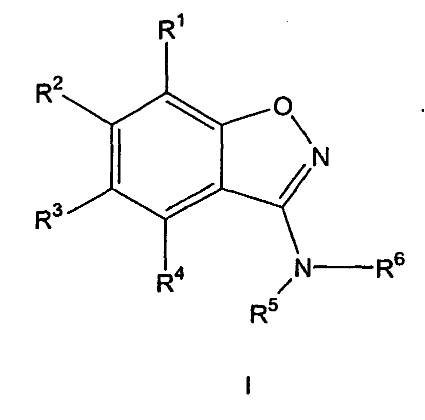 worin
R¹, R², R³ und R⁴, unabhängig voneinander, jeweils für
H; F, Cl, Br, I; -CN; -NC; -NO₂; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷;
für einen linearen oder verzweigten, gesättigten oder ungesättigten, unsubstituierten oder wenigstens einfach substituierten aliphatischen Rest;
für einen gesättigten oder ungesättigten, unsubstituierten oder wenigstens einfach substituierten, ggf. wenigstens ein Heteroatom als Ringglied aufweisenden cycloaliphatischen Rest, der mit einem mono-oder polyzyklischen Ringsystem kondensiert und/oder über eine lineare oder verzweigte Alkylen-, Alkenylen- oder Alkinylen-Gruppe gebunden sein kann;
oder für einen unsubstituierten oder substituierten Acryl- oder Heteroaryl-Rest steht, der mit einem mono- oder polyzyklischen Ringsystem kondensiert und/oder über eine lineare oder verzweigte Alkylen, Alkenylen- oder Alkinylen-Gruppe gebunden sein kann,
wobei wenigstens einer der Reste R¹, R², R³ und R⁴ für einen unsubstituierten oder substituierten Aryl- oder Heteroaryl-Rest steht und
wobei die vorstehend genannten substituierten Aryl- oder Heteroaryl-Reste ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-(C₁₋₅-Alkyl), -N(C₁₋₅-Alkyl)(C₁₋₅-Alkyl), -NH-C(=O)-O-(C₁₋₅-Alkyl), -C(=O)-H, -C(=O)-(C₁₋₅-Alkyl), -C(=O)-N(C₁₋₅-Alkyl)(C₁₋₅-Alkyl), -S(=O)₂NH₂; -S(=O)₂N(H)(C₁₋₅-Alkyl); -S(=O)₂N(C₁₋₅-Alkyl)(C₁₋₅-Alkyl); -S(=O)₂Phenyl; -S(=O)₂-C₁₋₅-Alkyl; -C(=O)-NH-CH₃, -C(=O)-NH-C₂H₅,-C(=O)-N(Cyclopropyl)-C₁₋₅-Alkyl, -N(Cyclopropyl)-S(=O)₂-C₁₋₅-Alkyl,-CH₂-NH-S(=O)₂-C₁₋₅-Alkyl, -CH₂-N(Cyclopropyl)-S(=O)₂-C₁₋₅-Alkyl,-CH₂-N(Cyclopropyl)-C(=O)-C₁₋₅-Alkyl, -CH₂-N(Cyclopropyl)-C(=O)-Phenyl, -O-Phenyl, -O-Benzyl, Phenyl, Benzyl, Thiophenyl (Thienyl), Furanyl und Pyridinyl substituiert sind, wobei jeweils der zyklische Teil der Reste -CH₂-N(Cyclopropyl)-C(=O)-Phenyl, -O-Phenyl, -O-Benzyl, Phenyl, -S(=O)₂Phenyl, Benzyl, Thiophenyl (Thienyl), Furanyl und Pyridinyl ggf. mit 1, 2, 3, 4 oder 5 Substituenten unabhängig voneinander ausgewählt aus der Gruppe bestehend aus F, Cl, Br, -OH; -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
R⁵ und R⁶, unabhängig voneinander, jeweils für
H; -C(=O)R¹⁸; -C(=O)NR¹⁹R²⁰); -C(=S)NR²¹R²²; -S(=O)₂R²³;
für einen linearen oder verzweigten, gesättigten oder ungesättigten, unsubstituierten oder wenigstens einfach substituierten aliphatischen Rest;
für einen gesättigten oder ungesättigten, unsubstituierten oder wenigstens einfach substituierten, ggf. wenigstens ein Heteroatom als Ringglied aufweisenden cycloaliphatischen Rest, der mit einem mono-oder polyzyklischen Ringsystem kondensiert und/oder über eine lineare oder verzweigte Alkylen-Gruppe gebunden sein kann;
oder für einen unsubstituierten oder wenigstens einfach substituierten Aryl- oder Heteroaryl-Rest steht, der mit einem mono- oder polyzyklischen Ringsystem kondensiert und/oder über eine lineare oder verzweigte Alkylen-Gruppe gebunden sein kann,
wobei der vorstehend genannte Aryl-Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -O-CF₃, -S-CF₃, -SH, -S-C₁₋₅-Alkyl, -C₁₋₅-Alkyl, -C(=O)-OH, -C(=O)-O-C₁₋₅-Alkyl, -NH-C₁₋₅-Alkyl, -N(C₁₋₅-Alkyl)₂, -NH-C(=O)-O-C₁₋₅-Alkyl, -C(=O)-H,-C(=O)-C₁₋₅-Alkyl, -C(=O)-NH₂, -C(=O)NH-C₁₋₅-Alkyl, -C(=O)-N(C₁₋₅-Alkyl)₂, -S(=O)₂NH₂; -S(=O)₂N(H)(C₁₋₅-Alkyl); -S(=O)₂N(C₁₋₅-Alkyl)(C₁₋₅-Alkyl); -S(=O)₂Phenyl; -S(=O)₂-C₁₋₅-Alkyl; -CH₂-NH-Cyclopropyl; -CH₂-N(C₁₋₅-Alkyl)-Cyclopropyl; -C(=O)-N(Cyclopropyl)-C₁₋₅-Alkyl,-N(Cyclopropyl)-S(=O)₂-C₁₋₅-Alkyl, -CH₂-NH-S(=O)₂-C₁₋₅-Alkyl, -CH₂-N(Cyclopropyl)-S(=O)₂-C₁₋₅-Alkyl, -CH₂-N(Cyclopropyl)-C(=O)-C₁₋₅-Alkyl, -CH₂-N(Cyclopropyl)-C(=O)-Phenyl, -O-Phenyl, -O-Benzyl, Phenyl, Benzyl, Thiophenyl (Thienyl), Furanyl und Pyridinyl substituiert ist, wobei jeweils der zyklische Teil der Reste -CH₂-N(Cyclopropyl)-C(=O)-Phenyl, -O-Phenyl, -O-Benzyl, Phenyl, -S(=O)₂Phenyl und Benzyl mit 1, 2, 3, 4 oder 5 Substituenten unabhängig voneinander ausgewählt aus der Gruppe bestehend aus F, Cl, Br, -OH, -CF₃, -SF₅,-CN, -NO₂, -C₁₋₅-Alkyl, -O-C₁₋₅-Alkyl, -O-CF₃, -S-CF₃, Phenyl und -O-Benzyl substituiert sein kann,
mit der Maßgabe, dass R⁵ und R⁶ nicht gleichzeitig für einen Wasserstoff-Rest stehen;
oder
R⁵ und R⁶ zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
R⁷ und R⁸, unabhängig voneinander, jeweils für
H, -C(=O)R¹⁵ oder für einen linearen oder verzweigten, gesättigten oder ungesättigten, unsubstituierten oder wenigstens einfach substituierten aliphatischen Rest stehen, oder
R⁷ und R⁸ zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
R⁹, R¹⁰, R¹¹ und R¹⁶ unabhängig voneinander, jeweils für
H;
für einen linearen oder verzweigten, gesättigten oder ungesättigten, unsubstituierten oder wenigstens einfach substituierten aliphatischen Rest;
für einen gesättigten oder ungesättigten, unsubstituierten oder wenigstens einfach substituierten, ggf. wenigstens ein Heteroatom als Ringglied aufweisenden cycloaliphatischen Rest, der mit einem mono-oder polyzyklischen Ringsystem kondensiert und/oder über eine lineare oder verzweigte Alkylen-Gruppe gebunden sein kann;
oder für einen unsubstituierten oder wenigstens einfach substituierten Aryl- oder Heteroaryl-Rest stehen, der mit einem mono- oder polyzyklischen Ringsystem kondensiert und/oder über eine lineare oder verzweigte Alkylen-Gruppe gebunden sein kann;
R¹² und R¹³, unabhängig voneinander, jeweils für
H oder für einen linearen oder verzweigten, gesättigten oder ungesättigten, unsubstituierten oder wenigstens einfach substituierten aliphatischen Rest stehen, oder
R¹² und R¹³ zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
R¹⁴ und R²³, unabhängig voneinander, jeweils für
-NR⁷R⁸;
für einen linearen oder verzweigten, gesättigten oder ungesättigten, unsubstituierten oder wenigstens einfach substituierten aliphatischen Rest,
für einen gesättigten oder ungesättigten, unsubstituierten oder wenigstens einfach substituierten, ggf. wenigstens ein Heteroatom als Ringglied aufweisenden cycloaliphatischen Rest, der mit einem mono-oder polyzyklischen Ringsystem kondensiert und/oder über eine lineare oder verzweigte Alkylen-Gruppe gebunden sein kann,
oder für einen unsubstituierten oder wenigstens einfach substituierten Aryl- oder Heteroaryl-Rest steht, der mit einem mono- oder polyzyklischen Ringsystem kondensiert und/oder über eine lineare oder verzweigte Alkylen-Gruppe gebunden sein kann,
R¹⁵, R¹⁷ und R¹⁸, unabhängig voneinander, jeweils
für einen linearen oder verzweigten, gesättigten oder ungesättigten, unsubstituierten oder wenigstens einfach substituierten aliphatischen Rest,
für einen gesättigten oder ungesättigten, unsubstituierten oder wenigstens einfach substituierten, ggf. wenigstens ein Heteroatom als Ringglied aufweisenden cycloaliphatischen Rest, der mit einem mono-oder polyzyklischen Ringsystem kondensiert und/oder über eine lineare oder verzweigte Alkylen-Gruppe gebunden sein kann,
oder für einen unsubstituierten oder wenigstens einfach substituierten Aryl- oder Heteroaryl-Rest steht, der mit einem mono- oder polyzyklischen Ringsystem kondensiert und/oder über eine lineare oder verzweigte Alkylen-Gruppe gebunden sein kann,
R¹⁹ und R²⁰, unabhängig voneinander, jeweils für
H oder für einen linearen oder verzweigten, gesättigten oder ungesättigten, unsubstituierten oder wenigstens einfach substituierten aliphatischen Rest stehen, oder
R¹⁹ und R²⁰ zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
R²¹ und R²², unabhängig voneinander, jeweils für
H oder für einen linearen oder verzweigten, gesättigten oder ungesättigten, unsubstituierten oder wenigstens einfach substituierten aliphatischen Rest stehen, oder
R²¹ und R²² zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

2. Verbindungen gemäß Anspruch 1, **dadurch gekennzeichnet, dass**
R¹, R², R³ und R⁴, unabhängig voneinander, jeweils für
H; F; Cl; Br; I; -CN; -NC; -NO₂; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl; -(C₁₋₅-Alkylen)-Heteroaryl;
wobei wenigstens einer der Reste R¹, R², R³ und R⁴ für einen Aryl- oder Heteroaryl-Rest steht;
R⁵ und R⁶, unabhängig voneinander, jeweils für
H; -C(=O)R¹⁸; -C(=O)NR¹⁹R²⁰; -C(=S)NR²¹R²²; -S(=O)₂R²³; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl oder -(C₁₋₅-Alkylen)-Heteroaryl stehen;
mit der Maßgabe, dass R⁵ und R⁶ nicht gleichzeitig für einen Wasserstoff-Rest stehen;
oder
R⁵ und R⁶ zusammen mit dem sie verbindenden Stickstoffatom als Ringglied einen Rest bilden, der ausgewählt ist aus der Gruppe H bestehend aus 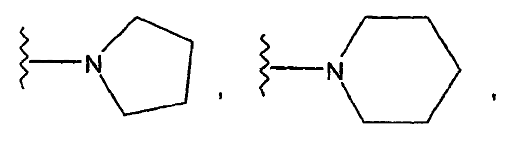 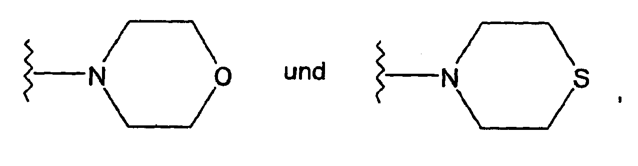
R⁷ und R⁸, unabhängig voneinander, jeweils für
H, -C(=O)R¹⁵ oder C₁₋₁₀-Alkyl stehen, oder
R⁷ und R⁸ zusammen mit dem sie verbindenden Stickstoffatom als Ringglied einen Rest bilden, der ausgewählt ist aus der vorstehend angegebenen Gruppe H;
R⁹, R¹⁰, R¹¹ und R¹⁶ unabhängig voneinander, jeweils für
H; C₁₋₁₀-Alkyl, C₂₋₁₀-Alkenyl, C₂₋₁₀-Alkinyl, C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl, -(C₁₋₅-Alkylen)-Aryl oder -(C₁₋₅-Alkylen)-Heteroaryl stehen;
R¹² und R¹³, unabhängig voneinander, jeweils für
H oder für einen C₁₋₁₀-Alkyl-Rest; oder
R¹² und R¹³ zusammen mit dem sie verbindenden Stickstoffatom als Ringglied einen Rest bilden, der ausgewählt ist aus der vorstehend angegebenen Gruppe H;
R¹⁴ und R²³, unabhängig voneinander, jeweils für
-NR⁷R⁸; C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl oder -(C₁₋₅-Alkylen)-Heteroaryl stehen;
R¹⁵, R¹⁷ und R¹⁸, unabhängig voneinander, jeweils für
C₁₋₁₀-Alkyl, C₂₋₁₀-Alkenyl, C₂₋₁₀-Alkinyl; C₃₋₈-Cycloalkyl; -(C₁₋₅-Alkylen)-C₃₋₈-Cycloalkyl; Heterocycloalkyl; -(C₁₋₅-Alkylen)-Heterocycloalkyl; Aryl; Heteroaryl; -(C₁₋₅-Alkylen)-Aryl oder -(C₁₋₅-Alkylen)-Heteroaryl stehen;
R¹⁹ und R²⁰, unabhängig voneinander, jeweils für
H oder für C₁₋₁₀-Alkyl stehen, oder
R¹⁹ und R²⁰ zusammen mit dem sie verbindenden Stickstoffatom als Ringglied einen Rest bilden, der ausgewählt ist aus der vorstehend angegebenen Gruppe H;
R²¹ und R²², unabhängig voneinander, jeweils für
H oder für C₁₋₁₀-Alkyl stehen, oder
R²¹ und R²² Gruppe H; zusammen mit dem sie verbindenden Stickstoffatom als Ringglied einen Rest bilden, der ausgewählt ist aus der vorstehend angegebenen
wobei
die vorstehend genannten C₁₋₁₀-Alkyl-, C₂₋₁₀-Alkenyl- und C₂₋₁₀-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; -CN; -OH; -SH; -O-C₁₋₂-Alkyl; -S-C₁₋₂-Alkyl und -NH₂ substituiert sein können,
die vorstehend genannten C₃₋₈-Cycloalkyl-Reste jeweils ggf. mit 1, 2, 3, 4 oder 5 Substituenten unabhängig voneinander ausgewählt aus der Gruppe bestehend aus F; Cl; Br; -CN; -OH; -SH; -C₁₋₅-Alkyl; -O-C₁₋₂-Alkyl; -S-C₁₋₂-Alkyl und -NH₂ substituiert sein können,
die vorstehend genannten Heterocycloalkyl-Reste jeweils 4-, 5-, 6- oder 7-gliedrig sind, 1 oder 2 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; -CN; -OH; -SH; -C₁₋₅-Alkyl; -O-C₁₋₂-Alkyl; -S-C₁₋₂-Alkyl und -NH₂ substituiert sein können,
die vorstehend genannten Aryl-Reste jeweils für einen Phenyl- oder Naphthyl-Rest stehen, der mit 1, 2, 3, 4 oder 5 Substituenten unabhängig voneinander ausgewählt aus der Gruppe bestehend aus F; Cl; Br; I; -CF₃; -OCF₃; -SCF₃; -SF₅; -CN; -OH; -SH; -C₁₋₅-Alkyl; -O-C₁₋₅-Alkyl; -S-C₁₋₅-Alkyl; -C(=O)-NH-CH₃, - C(=O)-NH-C₂H₅, -C(=O)-OH; -C(=O)-OC₁₋₅-Alkyl; -N(H)(C₁₋₅-Alkyl); -N(C₁₋₅-Alkyl)(C₁₋₅-Alkyl); -C(=O)-H; -C(=O)-C₁₋₅-Alkyl; -C(=O)N(C₁₋₅-Alkyl)(C₁₋₅-Alkyl); -S(=O)₂NH₂; -S(=O)₂N(H)(C₁₋₅-Alkyl); -S(=O)₂N(C₁₋₅-Alkyl)(C₁₋₅-Alkyl); -S(=O)₂-Phenyl; -S(=O)₂-C₁₋₅-Alkyl; -C(=O)-N(Cyclopropyl)-C₁₋₅-Alkyl, -N(Cyclopropyl)-S(=O)₂-C₁₋₅-Alkyl, -CH₂-NH-S(=O)₂-C₁₋₅-Alkyl, -CH₂-N(Cyclopropyl)-S(=O)₂-C₁₋₅-Alkyl, -CH₂-N(Cyclopropyl)-C(=O)-C₁₋₅-Alkyl, -CH₂-N(Cyclopropyl)-C(=O)-Phenyl, Phenyl; Phenoxy, Benzyl; Thiophenyl (Thienyl), Furanyl und Pyridinyl substituiert sein kann,
die vorstehend genannten Heteroaryl-Reste jeweils 5- oder 6-gliedrig sind, 1, 2 oder 3 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; I; -CF₃; -OCF₃; -SCF₃; - SF₅; -CN; -OH; -SH; -C₁₋₅-Alkyl; -O-C₁₋₅-Alkyl; -S-C₁₋₅-Alkyl; -C(=O)-OH; - C(=O)-OC₁₋₅-Alkyl; -NH₂; -N(H)(C₁₋₅-Alkyl); -N(C₁₋₅-Alkyl)(C₁₋₅-Alkyl), -C(=O)-NH-CH₃, -C(=O)-NH-C₂H₅, -C(=O)N(C₁₋₅-Alkyl)(C₁₋₅-Alkyl), -S(=O)₂NH₂; - S(=O)₂N(H)(C₁₋₅-Alkyl); -S(=O)₂N(C₁₋₅-Alkyl)(C₁₋₅-Alkyl); -S(=O)₂-Phenyl; - S(=O)₂-C₁₋₅-Alkyl; -CH₂-NH-Cyclopropyl; -CH₂-N(C₁₋₅-Alkyl)-Cyclopropyl; - C(=O)-N(Cyclopropyl)-C₁₋₅-Alkyl, -N(Cyclopropyl)-S(=O)₂-C₁₋₅-Alkyl, -CH₂-NH-S(=O)₂-C₁₋₅-Alkyl, -CH₂-N(Cyclopropyl)-S(=O)₂-C₁₋₅-Alkyl, -CH₂-N(Cyclopropyl)-C(=O)-C₁₋₅-Alkyl, -CH₂-N(Cyclopropyl)-C(=O)-Phenyl, Phenyl; Phenoxy, Benzyl; Thiophenyl (Thienyl), Furanyl und Pyridinyl substituiert sein können,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

3. Verbindungen gemäß Anspruch 1 oder 2, **dadurch gekennzeichnet, dass**
R¹, R², R³ und R⁴, unabhängig voneinander, jeweils für
H; F; Cl; Br; I; -CN; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; C₁₋₄-Alkyl, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
wobei wenigstens einer der Reste R¹, R², R³ und R⁴ für einen Aryl- oder Heteroaryl-Rest steht;
R⁵ und R⁶, unabhängig voneinander, jeweils für
H; -C(=O)R¹⁸; -C(=O)NR¹⁹R²⁰; -C(=S)NR²¹R²²; -S(=O)₂R²³; C₁₋₄-Alkyl, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
mit der Maßgabe, dass R⁵ und R⁶ nicht gleichzeitig für einen Wasserstoff-Rest stehen;
oder
R⁵ und R⁶ zusammen mit dem sie verbindenden Stickstoffatom als Ringglied einen Rest bilden, der ausgewählt ist aus der Gruppe H bestehend aus 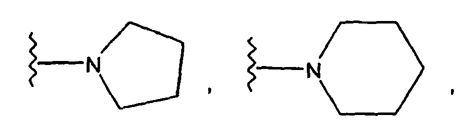 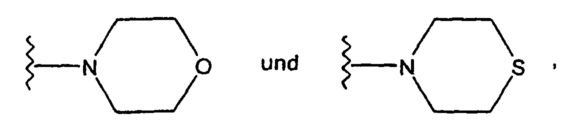
R⁷ und R⁸, unabhängig voneinander, jeweils für
H, -C(=O)R¹⁵ oder C₁₋₄-Alkyl stehen, oder
R⁷ und R⁸ zusammen mit dem sie verbindenden Stickstoffatom als Ringglied einen Rest bilden, der ausgewählt ist aus der vorstehend angegebenen Gruppe H;
R⁹, R¹⁰, R¹¹ und R¹⁶ unabhängig voneinander, jeweils für
H; C₁₋₄-Alkyl, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
R¹² und R¹³, unabhängig voneinander, jeweils für
H oder für C₁₋₄-Alkyl; oder
R¹² und R¹³ zusammen mit dem sie verbindenden Stickstoffatom als Ringglied einen Rest bilden, der ausgewählt ist aus der vorstehend angegebenen Gruppe H;
R¹⁴ und R²³, unabhängig voneinander, jeweils für -NR⁷R⁸; C₁₋₄-Alkyl, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
R¹⁵, R¹⁷ und R¹⁸, unabhängig voneinander, jeweils für
C₁₋₄-Alky/, C₂₋₄-Alkenyl, C₂₋₄-Alkinyl; C₃₋₈-Cycloalkyl; -(C_{1, 2 oder 3}-Alkylen)-C₃₋₈-Cycloalkyl; Heterocycloalkyl; -(C_{1, 2 oder 3}-Alkylen)-Heterocycloalkyl; Aryl; Heteroaryl; -(C_{1, 2 oder 3}-Alkylen)-Aryl oder -(C_{1, 2 oder 3}-Alkylen)-Heteroaryl stehen;
R¹⁹ und R²⁰, unabhängig voneinander, jeweils für
H oder für C₁₋₄-Alkyl stehen, oder
R¹⁹ und R²⁰ zusammen mit dem sie verbindenden Stickstoffatom als Ringglied einen Rest bilden, der ausgewählt ist aus der vorstehend angegebenen Gruppe H;
R²¹ und R²², unabhängig voneinander, jeweils für
H oder für C₁₋₄-Alkyl stehen, oder
R²¹ und R²² zusammen mit dem sie verbindenden Stickstoffatom als Ringglied einen Rest bilden, der ausgewählt ist aus der vorstehend angegebenen Gruppe H;
wobei
die vorstehend genannten C₁₋₄-Alkyl-, C₂₋₄-Alkenyl- und C₂₋₄-Alkinyl-Reste jeweils linear oder verzweigt sind und ggf. mit 1, 2, 3, 4 oder 5 Substituenten unabhängig voneinander ausgewählt aus der Gruppe bestehend aus F; Cl; Br; -CN; -OH; -OCH₃ und -NH₂ substituiert sein können,
die vorstehend genannten C₃₋₈-Cycloalkyl-Reste jeweils ggf. mit 1, 2, 3, 4 oder 5 Substituenten unabhängig voneinander ausgewählt aus der Gruppe bestehend aus F; Cl; Br; -CN; -OH; -CH₃; -C₂H₅; -OCH₃; und -NH₂ substituiert sein können,
die vorstehend genannten Heterocycloalkyl-Reste jeweils 4-, 5-, 6- oder 7-gliedrig sind, 1 oder 2 Heteroatome unabhängig voneinander ausgewählt aus der Gruppe bestehend aus Sauerstoff, Schwefel und Stickstoff als Ringglied(er) aufweisen und ggf. mit 1, 2, 3, 4 oder 5 Substituenten unabhängig voneinander ausgewählt aus der Gruppe bestehend aus F; Cl; Br; -CN; -OH; -CH₃; -C₂H₅; -OCH₃; und -NH₂ substituiert sein können,
die vorstehend genannten Aryl-Reste jeweils für einen Phenyl- oder Naphthyl-Rest stehen, der mit 1, 2, 3, 4 oder 5 Substituenten unabhängig voneinander ausgewählt aus der Gruppe bestehend aus F; Cl; Br; -CF₃; -OCF₃; -SCF₃; -SF₅; -CN; -OH; Methyl; Ethyl; n-Propyl; Isopropyl; n-Butyl; Isobutyl; sec-Butyl; tert-Butyl; Methoxy; Ethoxy; -N(CH₃)₂; -N(C₂H₅)₂; Phenyl; Phenoxy, Benzyl; Thiophenyl (Thienyl), Furanyl, Pyridinyl, -C(=O)-NH-CH₃, -C(=O)-NH-C₂H₅, - C(=O)-H, -C(=O)-CH₃, -C(=O)-C₂H₅, -C(=O)-CH(CH₃)₂, -C(=O)-C(CH₃)₃, - C(=O)-O-CH₃, -C(=O)-O-C₂H₅, -C(=O)-O-C(CH₃)₃, -C(=O)-O-CH(CH₃)₂, - C(=O)-OH, -C(=O)-N(Cyclopropyl)-CH₃, -C(=O)-N(Cyclopropyl)-C₂H₅, -C(=O)-N(Cyclopropyl)-CH(CH₃)₂, -C(=O)-N(Cyclopropyl)-C(CH₃)₃, -CH₂-NH-Cyclopropyl, -CH₂-N(Cyclopropyl)-S(=O)₂-CH₃, -CH₂-N(Cyclopropyl)-S(=O)₂-C₂H₅, -CH₂-N(Cyclopropyl)-C(=O)-CH₃, -CH₂-N(Cyclopropyl)-C(=O)-C₂H₅, - CH₂-N(Cyclopropyl)-C(=O)-CH(CH₃)₂, -CH₂-N(Cyclopropyl)-C(=O)-C(CH₃)₃ und -CH₂-N(Cyclopropyl)-C(=O)-Phenyl substituiert sein kann;
die vorstehend genannten Heteroaryl-Reste jeweils für einen Furanyl-, Thienyl- (Thiophenyl-) oder Pyridinyl-Rest stehen und ggf. mit 1, 2, 3, 4 oder 5 Substituenten unabhängig voneinander ausgewählt aus der Gruppe bestehend F; Cl; Br; -CF₃, -OCF₃; -SCF₃; -SF₅; -CN; -OH; -C(=O)-NH-CH₃, - C(=O)-NH-C₂H₅, Methyl; Ethyl; n-Propyl; Isopropyl; n-Butyl; Isobutyl; sec-Butyl; tert-Butyl; Methoxy; Ethoxy; -NH₂; -N(CH₃)₂; -N(C₂H₅)₂; Phenyl; Phenoxy, Benzyl; Thiophenyl (Thienyl), Furanyl, Pyridinyl, -C(=O)-H, -C(=O)-CH₃, -C(=O)-C₂H₅, -C(=O)-CH(CH₃)₂, -C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(Cyclopropyl)-CH₃, -C(=O)-N(Cyclopropyl)-C₂H₅, -C(=O)-N(Cyclopropyl)-CH(CH₃)₂, -C(=O)-N(Cyclopropyl)-C(CH₃)₃, -CH₂-NH-Cyclopropyl, -CH₂-N(Cyclopropyl)-S(=O)₂-CH₃, -CH₂-N(Cyclopropyl)-S(=O)₂-C₂H₅, -CH₂-N(Cyclopropyl)-C(=O)-CH₃, -CH₂-N(Cyclopropyl)-C(=O)-C₂H₅, -CH₂-N(Cyclopropyl)-C(=O)-CH(CH₃)₂, -CH₂-N(Cyclopropyl)-C(=O)-C(CH₃)₃ und - CH₂-N(Cyclopropyl)-C(=O)-Phenyl substituiert sein können,
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

4. Verbindungen gemäß einem oder mehreren der Ansprüche 1-3 der allgemeinen Formel I', 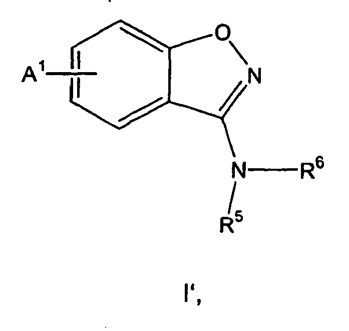 worin
A¹ für einen (Hetero)aryl-Rest steht, der ausgewählt ist aus der Gruppe bestehend aus Phenyl, 1-Naphthyl, 2-Naphthyl, Thiophenyl (Thienyl), Furanyl und Pyridinyl und der ggf. mit 1, 2, 3, 4 oder 5 Substituenten unabhängig voneinander ausgewählt aus der Gruppe bestehend F; Cl; Br; -CF₃; -OCF₃; -SCF₃; -SF₅; -CN; -OH; Methyl; Ethyl; n-Propyl; Isopropyl; n-Butyl; Isobutyl; sec-Butyl; tert-Butyl; Methoxy; Ethoxy;-NH₂; -N(CH₃)₂; -N(C₂H₅)₂; Phenyl; Phenoxy, Benzyl, Thiophenyl (Thienyl), Furanyl, Pyridinyl, -C(=O)-NH-CH₃, -C(=O)-NH-C₂H₅, -C(=O)-H, -C(=O)-CH₃, -C(=O)-C₂H₅, -C(=O)-CH(CH₃)₂, -C(=O)-C(CH₃)₃,-C(=O)-O-CH₃, -C(=O)-O-C₂H₅, -C(=O)-O-C(CH₃)₃, -C(=O)-O-CH(CH₃)₂, -C(=O)-OH, -C(=O)-N(Cyclopropyl)-CH₃, -C(=O)-N(Cyclopropyl)-C₂H₅,-C(=O)-N(Cyclopropyl)-CH(CH₃)₂, -C(=O)-N(Cyclopropyl)-C(CH₃)₃,-CH₂-NH-Cyclopropyl, -CH₂-N(Cyclopropyl)-S(=O)₂-CH₃, -CH₂-N(Cyclopropyl)-S(=O)₂-C₂H₅, -CH₂-N(Cyclopropyl)-C(=O)-CH₃, -CH₂-N(Cyclopropyl)-C(=O)-C₂H₅, -CH₂-N(Cyclopropyl)-C(=O)-CH(CH₃)₂,-CH₂-N(Cyclopropyl)-C(=O)-C(CH₃)₃ und -CH₂-N(Cyclopropyl)-C(=O)-Phenyl substituiert sein kann;
R⁵ und R⁶, unabhängig voneinander, jeweils für
H; einen Alkyl-Rest ausgewählt aus der Gruppe bestehend aus Methyl; Ethyl; n-Propyl; Isopropyl; n-Butyl; Isobutyl; sec-Butyl, tert-Butyl, n-Pentyl und Neopentyl; für -C(=O)-Phenyl; -(CH₂)-Phenyl; -(CH₂)₂-Phenyl; -(CH₂)₃-Phenyl; -(CH₂)-Pyridinyl; -(CH₂)₂-Pyridinyl; -(CH₂)₃-Pyridinyl; wobei die vorstehend genannten Phenyl und Pyridinyl-Reste ggf. mit 1, 2, 3, 4 oder 5 Substituenten unabhängig voneinander ausgewählt aus der Gruppe bestehend F; Cl; Br; -CF₃; -OCF₃; -SCF₃;-SF₅; -CN; -OH; Methyl; Ethyl; n-Propyl; Isopropyl; n-Butyl; Isobutyl; sec-Butyl; tert-Butyl; Methoxy; Ethoxy; -N(CH₃)₂; -N(C₂H₅)₂; Phenyl; Phenoxy, Benzyl; Thiophenyl (Thienyl), Furanyl und Pyridinyl substituiert sein können;
mit der Maßgabe, dass R⁵ und R⁶ nicht gleichzeitig für einen Wasserstoff-Rest stehen;
oder
R⁵ und R⁶ zusammen mit dem sie verbindenden Stickstoffatom als Ringglied einen Rest bilden, der ausgewählt ist aus der Gruppe H bestehend aus 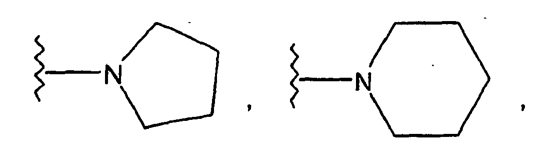 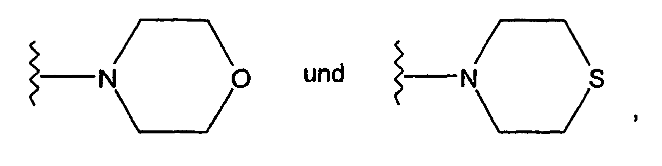
jeweils ggf. in Form entsprechender Salze, insbesondere der Hydrochlorid-Additionssalze, oder jeweils in Form entsprechender Solvate.

5. Verbindungen gemäß einem oder mehreren der Ansprüche 1-4: 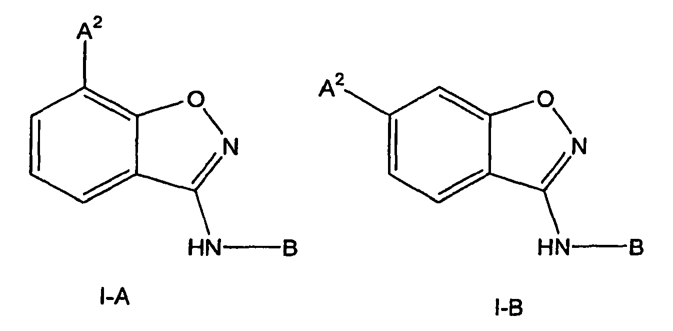 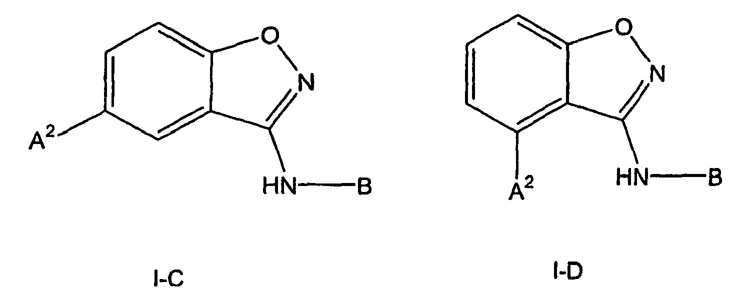 worin jeweils
A² für einen (Hetero)aryl-Rest steht, der ausgewählt ist aus der Gruppe bestehend aus Phenyl, 1-Naphthyl, 2-Naphthyl, Thiophenyl (Thienyl), Furanyl und Pyridinyl und der ggf. mit 1, 2, 3, 4 oder 5 Substituenten unabhängig voneinander ausgewählt aus der Gruppe bestehend F; Cl; Br; -CF₃; -OCF₃; -SCF₃; -SF₅; -CN; -OH; Methyl; Ethyl; n-Propyl; Isopropyl: n-Butyl; Isobutyl; sec-Butyl; tert-Butyl; Methoxy; Ethoxy;-NH₂; -N(CH₃)₂; -N(C₂H₅)₂; Phenyl; Phenoxy, Benzyl; Thiophenyl (Thienyl), Furanyl, Pyridinyl, -C(=O)-NH-CH₃, -C(=O)-NH-C₂H₅,-C(=O)-H, -C(=O)-CH₃, -C(=O)-C₂H₅, -C(=O)-CH(CH₃)₂, -C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(Cyclopropyl)-CH₃, -C(=O)-N(Cyclopropyl)-C₂H₅, -C(=O)-N(Cyclopropyl)-CH(CH₃)₂, -C(=O)-N(Cyclopropyl)-C(CH₃)₃, -CH₂-NH-Cyclopropyl, -CH₂-N(Cyclopropyl)-S(=O)₂-CH₃, -CH₂-N(Cyclopropyl)-S(=O)₂-C₂H₅, -CH₂-N(Cyclopropyl)-C(=O)-CH₃, -CH₂-N(Cyclopropyl)-C(=O)-C₂H₅, -CH₂-N(Cyclopropyl)-C(=O)-CH(CH₃)₂, -CH₂-N(Cyclopropyl)-C(=O)-C(CH₃)₃ und -CH₂-N(Cyclopropyl)-C(=O)-Phenyl substituiert sein kann;
B für einen Alkyl-Rest ausgewählt aus der Gruppe bestehend aus Methyl; Ethyl; n-Propyl; Isopropyl; n-Butyl; Isobutyl; sec-Butyl, tert-Butyl, n-Pentyl und Neopentyl; für -C(=O)-Phenyl; -(CH₂)-Phenyl;-(CH₂)₂-Phenyl; -(CH₂)₃-Phenyl; -(CH₂)-Pyridinyl; -(CH₂)₂-Pyridinyl;-(CH₂)₃-Pyridinyl; wobei die vorstehend genannten Phenyl und Pyridinyl-Reste ggf. mit 1, 2, 3, 4 oder 5 Substituenten unabhängig voneinander ausgewählt aus der Gruppe bestehend F; Cl; Br; -CF₃; -OCF₃; -SCF₃;-SF₅; -CN; -OH; Methyl; Ethyl; n-Propyl; Isopropyl; n-Butyl; Isobutyl; sec-Butyl; tert-Butyl; Methoxy; Ethoxy; -N(CH₃)₂; -N(C₂H₅)₂; Phenyl; Phenoxy, Benzyl; Thiophenyl (Thienyl), Furanyl und Pyridinyl substituiert sein können,
jeweils ggf. in Form entsprechender Salze, insbesondere der Hydrochlorid-Additionssalze, oder jeweils in Form entsprechender Solvate.

6. Verbindungen gemäß Anspruch 5, **dadurch gekennzeichnet, dass** A² für einen der folgenden Reste A²¹ oder A²² steht: 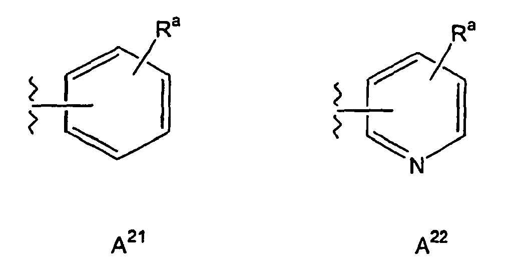 worin
R^{a} jeweils für einen Rest steht, der ausgewählt ist aus der Gruppe bestehend aus H, F, Cl, Br, I, -CN; -CF₃, -OCF₃, -SCF₃, -SF₅, -OH, -SH, Methyl; Ethyl; n-Propyl; Isopropyl; n-Butyl; Isobutyl; sec-Butyl; tert-Butyl; -OCH₃; -OC₂H₅; -S-CH₃, -S-C₂H₅, -C(=O)-NH-CH₃, -C(=O)-NH-C₂H₅,-C(=O)-H, -C(=O)-O-C₂H₅, -C(=O)-OH, -CH₂-NH-Cyclopropyl, -CH₂-N(Cyclopropyl)-S(=O)₂-CH₃, -CH₂-N(Cyclopropyl)-C(=O)-CH₃ und -CH₂-N(Cyclopropyl)-C(=O)-Phenyl steht und
B für einen Alkyl-Rest ausgewählt aus der Gruppe bestehend aus Methyl, Ethyl, n-Propyl, Isopropyl, n-Butyl, Isobutyl, sec-Butyl, tert-Butyl, n-Pentyl und Neopentyl steht;
oder für einen der folgenden Reste B¹, B² oder B³ steht, 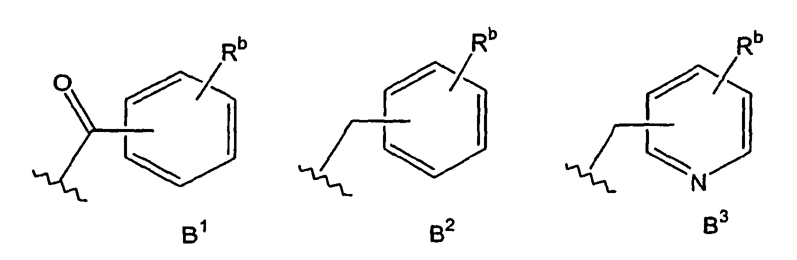 worin
R^{b} jeweils für einen Rest steht, der ausgewählt ist aus der Gruppe bestehend aus H, F, Cl, Br, I, -CN; -CF₃, -OCF₃, -SCF₃, -SF₅, -OH, -SH, Methyl; Ethyl; n-Propyl; Isopropyl; n-Butyl; Isobutyl; sec-Butyl; tert-Butyl; -OCH₃; -OC₂H₅; -S-CH₃ und -S-C₂H₅.

7. Verbindungen gemäß einem oder mehreren der Ansprüche 1-6 ausgewählt aus der Gruppe bestehend aus
[1] Benzyl-[6-(3-chlor-phenyl)-benzo[d]isoxazol-3-yl]-amin,
[2] Benzyl-[6-(4-chlor-phenyl)-benzo[d]isoxazol-3-yl]-amin,
[3] Benzyl-(6-p-tolyl-benzo[d]isoxazol-3-yl)-amin,
[4] Benzyl-[6-(2-methoxy-phenyl)-benzo[d]isoxazol-3-yl]-amin,
[5] N-[6-(3-Chlor-phenyl)-benzo[d]isoxazol-3-yl]-benzamid,
[6] N-[6-(4-Chlor-phenyl)-benzo[d]isoxazol-3-yl]-benzamid,
[7] N-(6-p-Tolyl-benzo[d]isoxazol-3-yl)-benzamid,
[8] N-[6-(2-Methoxy-phenyl)-benzo[d]isoxazol-3-yl]-benzamid,
[9] (4-tert-Butyl-benzyl)-(6-pyridin-2-yl-benzo[d]isoxazol-3-yl)-amin,
[10] (4-tert-Butyl-benzyl)-[6-(3-methyl-pyridin-2-yl)-benzo[d]isoxazol-3-yl]-amin,
[11] (4-tert-Butyl-benzyl)-[6-(6-methyl-pyridin-2-yl)-benzo[d]isoxazol-3-yl]-amin,
[12] (4-tert-Butyl-benzyl)-(6-phenyl-benzo[d]isoxazol-3-yl)-amin,
[13] [6-(4-Chlor-phenyl)-benzo[d]isoxazol-3-yl]-pyridin-2-ylmethyl-amin,
[14] (6-Phenyl-benzo[d]isoxazol-3-yl)-pyridin-2-ylmethyl-amin,
[15] Pyridin-2-ylmethyl-(6-o-tolyl-benzo[d]isoxazol-3-yl)-amin,
[16] [6-(4-Chlor-phenyl)-benzo[d]isoxazol-3-yl]-pyridin-3-ylmethyl-amin,
[17] (6-Phenyl-benzo[d]isoxazol-3-yl)-pyridin-3-ylmethyl-amin,
[18] Pyridin-3-ylmethyl-(6-o-tolyl-benzo[d]isoxazol-3-yl)-amin,
[19] 2-{3-[(Pyridin-3-ylmethyl)-amino]-benzo[d]isoxazol-6-yl}-phenol,
[20] [6-(4-Chlor-phenyl)-benzo[d]isoxazol-3-yl]-pyridin-4-ylmethyl-amin,
[21] (6-Phenyl-benzo[d]isoxazol-3-yl)-pyridin-4-ylmethyl-amin,
[22] Pyridin-4-ylmethyl-(6-o-totyl-benzo[d]isoxazol-3-yl)-amin,
[23] 4-{[6-(4-Chlor-phenyl)-benzo[d]isoxazol-3-ylamino]-methyl}-benzonitril,
[24] 4-[(6-Phenyl-benzo[d]isoxazol-3-ylamino)-methyl]-benzonitril,
[25] 4-[(6-o-Tolyl-benzo[d]isoxazol-3-ylamino)-methyl]-benzonitril,
[26] 4-{[6-(2-Hydroxy-phenyl)-benzo[d]isoxazol-3-ylamino]-methyl}-benzonitril,
[27] [6-(4-Chlor-phenyl)-benzo[d]isoxazol-3-yl]-(3-methyl-benzyl)-amin,
[28] (3-Methyl-benzyl)-(6-phenyl-benzo[d]isoxazol-3-yl)-amin,
[29] (3-Methyl-benzyl)-(6-o-tolyl-benzo[d]isoxazol-3-yl)-amin,
[30] 2-[3-(3-Methyl-benzylamino)-benzo[d]isoxazol-6-yl]-phenol,
[31] (3-Chlor-benzyl)-[6-(4-chlor-phenyl)-benzo[d]isoxazol-3-yl]-amin,
[32] (3-Chlor-benzyl)-(6-phenyl-benzo[d]isoxazol-3-yl)-amin,
[33] (3-Chlor-benzyl)-(6-o-tolyl-benzo[d]isoxazol-3-yl)-amin,
[34] 2-[3-(3-Chlor-benzylamino)-benzo[d]isoxazol-6-yl]-phenol,
[35] 2-(3-(Neopentylamino)benzo[d]isoxazol-5-yl)benzoesäure,
[36] Ethyl 4-(3-(neopentylamino)benzo[d]isoxazol-5-yl)benzoat,
[37] N-Neopentyl-5-(3-(trifluormethyl)phenyl)benzo[d]isoxazol-3-amin,
[38] 3-(3-(Neopentylamino)benzo[d]isoxazol-5-yl)benzonitril,
[39] N-Methyl-3-(3-(neopentylamino)benzo[d]isoxazol-5-yl)benzamid,
[40] 3-(3-(Neopentylamino)benzo[d]isoxazol-5-yl)benzoesäure,
[41] Ethyl 3-(3-(neopentylamino)benzo[d]isoxazol-5-yl)benzoat,
[42] N-Cyclopropyl-N-(2-methoxy-5-(3-(neopentylamino)benzo[d]isoxazol-5-yl)benzyl)methansulfonamid,
[43] N-Cyclopropyl-N-(2-methoxy-5-(3-(neopentylamino)benzo[d]isoxazol-5-yl)benzyl)acetamid,
[44] N-Cyclopropyl-N-(2-fluor-5-(3-(neopentylamino)benzo[d]isoxazol-5-yl)benzyl)acetamid,
[45] 5-(3-((Cyclopropylamino)methyl)-4-methoxyphenyl)-N-neopentylbenzo[d]isoxazol-3-amin,
[46] 5-(3-((Cyclopropylamino)methyl)-4-fluorphenyl)-N-neopentylbenzo[d]isoxazol-3-amin,
[47] 2-Methoxy-5-(3-(neopentylamino)benzo[d]isoxazol-5-yl)benzaldehyd,
[48] 2-Fluor-5-(3-(neopentylamino)benzo[d]isoxazol-5-yl)benzaldehyd,
[49] N-Cyclopropyl-N-(3-(3-(neopentylamino)benzo[d]isoxazol-5-yl)benzyl)benzamid,
[50] N-Cyclopropyl-N-(3-(3-(neopentylamino)benzo[d]isoxazol-5-yl)benzyl)acetamid,
[51] 5-(3-((Cyclopropylamino)methyl)phenyl)-N-neopentylbenzo[d]isoxazol-3-amin und
[52] 3-(3-(Neopentylamino)benzo[d]isoxazol-5-yl)benzaldehyd;
sowie jeweils deren entsprechende Salze, insbesondere deren Hydrochlorid-Additionssalze, und ggf. jeweils deren entsprechende Solvate.

8. Verbindungen gemäß einem oder mehreren der Ansprüche 1-7, **dadurch gekennzeichnet, dass** sie im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM aufweisen.

9. Verfahren zur Herstellung von Verbindungen gemäß einem oder mehreren der Ansprüche 1-8, **dadurch gekennzeichnet, dass** eine ggf. substituierte 2-Fluor-benzonitril-verbindung der allgemeinen Formel II, 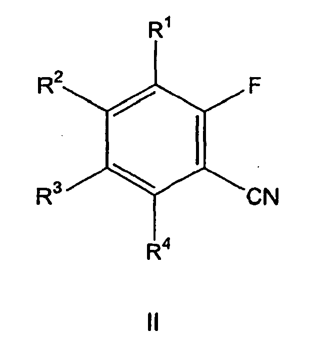 worin die Reste R¹, R², R³ und R⁴ die Bedeutung gemäß einem oder mehreren der Ansprüche 1-8 haben, in einem Reaktionsmedium in Gegenwart einer Base mit Acethydroxamsäure der Formel III 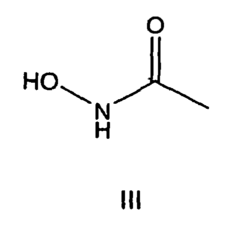 zu einer Verbindung der allgemeinen Formel I, 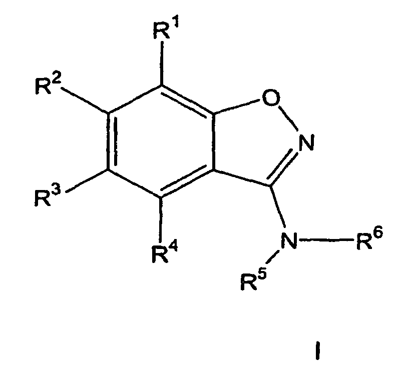 worin die Reste R¹-R⁴ die vorstehend genannte Bedeutung haben sowie die Reste R⁵ und R⁶ jeweils für einen Wasserstoff-Rest stehen, umgesetzt wird, diese ggf. gereinigt und/oder ggf. isoliert wird,
und diese ggf. anschließend in einem Reaktionsmedium mit wenigstens einem Isocyanat der allgemeinen Formel R¹⁹-N=C=O, worin R¹⁹ die Bedeutung gemäß einem oder mehreren der Ansprüche 1 bis 8 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹, R², R³, R⁴ und R⁵ die vorstehend genannte Bedeutung haben und R⁶ für -C(=O)-NR¹⁹R²⁰ steht, wobei R¹⁹ die vorstehend genannte Bedeutung hat und R²⁰ für einen Wasserstoff-Rest steht, und diese ggf. gereinigt und/oder ggf. isoliert wird
oder
diese ggf. anschließend in einem Reaktionsmedium mit wenigstens einem Isothiocyanat der allgemeinen Formel S=C=N-R²¹, worin R²¹ die Bedeutung gemäß einem oder mehreren der Ansprüche 1 bis 8 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹, R², R³, R⁴ und R⁵ die vorstehend genannte Bedeutung haben und R⁶ für -C(=S)-NR²¹R²² steht, wobei R²¹ die vorstehend genannte Bedeutung hat und R²² für einen Wasserstoff-Rest steht, und diese ggf. gereinigt und/oder ggf. isoliert wird,
und ggf. wenigstens eine Verbindung der allgemeinen Formel I, worin R¹, R², R³, R⁴ und R⁵ die vorstehend genannte Bedeutung haben und R⁶ für -C(=O)-NR¹⁹R²⁰ oder für -C(=S)-NR²¹R²² steht, wobei R¹⁹ und R²¹ die vorstehend genannte Bedeutung haben und R²⁰ und R²² jeweils für einen Wasserstoff-Rest stehen, in einem Reaktionsmedium, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R²⁰ oder der allgemeinen Formel LG-R²², worin LG für eine Abgangsgruppe, bevorzugt für ein Halogen-Atom, besonders bevorzugt für ein Chloratom steht, und R²² und R²⁴ jeweils die Bedeutung gemäß einem oder mehreren der Ansprüche 1-8 mit Ausnahme von Wasserstoff haben, zu wenigstens einer Verbindung der allgemeinen Formel I umgesetzt wird, worin R¹ bis R⁶ die vorstehend genannte Bedeutung haben, und diese ggf. gereinigt und/oder ggf. isoliert wird,
oder
ggf. wenigstens eine Verbindung der allgemeinen Formel I, worin R¹, R², R³, R⁴ die vorstehend genannte Bedeutung haben und R⁵ und R⁶ jeweils für H stehen, in einem Reaktionsmedium, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R⁵ oder der allgemeinen Formel LG-R⁶, worin LG für eine Abgangsgruppe, bevorzugt für ein Halogen-Atom, besonders bevorzugt für ein Chloratom steht, und R⁵ und R⁶ jeweils die Bedeutung gemäß einem oder mehreren der Ansprüche 1-8 mit Ausnahme von Wasserstoff haben, zu wenigstens einer Verbindung der allgemeinen Formel I umgesetzt wird, worin R¹ bis R⁶ die vorstehend genannte Bedeutung haben, und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen in einem Reaktionsmedium, in Gegenwart wenigstens einer Säure und wenigstens eines Reduktionsmittels, bevorzugt in Gegenwart von Trifluoressigsäure und Triethylsilan, mit wenigstens einer Verbindung der allgemeinen Formel R⁵-(C=O)-H, worin R⁵ die Bedeutung gemäß einem oder mehreren der Ansprüche 1 bis 8 mit Ausnahme von H, -C(=O)R¹⁸, -C(=O)-NR¹⁹R²⁰, -C(=S)-NR²¹R²² und -S(=O)₂R²³ hat, zu wenigstens einer Verbindung der allgemeinen Formel I ungesetzt wird, worin R¹ bis R⁵ die vorstehend genannte Bedeutung haben und R⁶ für H steht, und diese ggf. gereinigt und/oder ggf. isoliert wird
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium mit wenigstens einer Verbindung der allgemeinen Formel R¹⁸-C(=O)-LG, worin R¹⁸ die Bedeutung gemäß einem oder mehreren der Ansprüche 1 bis 8 hat und LG für eine Abgangsgruppe, vorzugsweise für ein Halogenatom, besonders bevorzugt für ein Chloratom steht zu wenigstens einer Verbindung der allgemeinen Formel I umgesetzt wird, worin R¹ bis R⁴ die vorstehend genannte Bedeutung haben, R⁵ für einen -C(=O)R¹⁸-Rest und R⁶ für einen Wasserstoff-Rest steht; und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium in Gegenwart wenigstens eines Kupplungsreagenzes ggf. in Gegenwart wenigstens einer Base mit wenigstens einer Verbindung der allgemeinen Formel R¹⁸-C(=O)-OH, worin R¹⁸ die Bedeutung gemäß einem oder mehreren der Ansprüche 1 bis 8 hat zu wenigstens einer Verbindung der allgemeinen Formel I umgesetzt wird, worin R¹ bis R⁴ die vorstehend genannte Bedeutung haben, R⁵ für einen -C(=O)R¹⁸-Rest und R⁶ für einen Wasserstoff-Rest steht; und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium, ggf. in Gegenwart wenigstens einer Base, mit wenigstens einer Verbindung der allgemeinen Formel R²³-S(=O)₂-LG, worin R²³ die Bedeutung gemäß einem oder mehreren der Ansprüche 1 bis 8 hat und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ bis R⁴ die vorstehend genannte Bedeutung haben, R⁵ für eine -S(=O)₂-R²³-Gruppe und R⁶ für einen Wasserstoff-Rest steht, und diese ggf. gereinigt und/oder ggf. isoliert wird.

10. Verfahren zur Herstellung von Verbindungen gemäß einem oder mehreren der Ansprüche 1-8, **dadurch gekennzeichnet, dass** eine ggf. substituierte 2-Fluor-benzonitril-verbindung der allgemeinen Formel II, 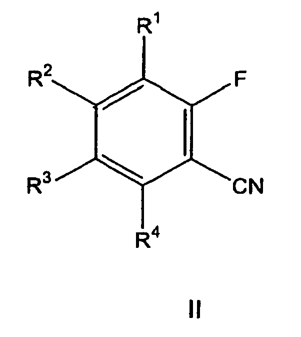 worin wenigstens einer der Reste R¹, R², R³ und R⁴ für eine Abgangsgruppe, vorzugsweise für einen Halogen-Rest oder einen Sulfonsäureester, besonders bevorzugt für eine Abgangsgruppe ausgewählt aus der Gruppe bestehend aus Chlor, Brom, Iod, Triflat, Mesylat und Tosylat, ganz besonders bevorzugt für ein Bromatom steht, und die übrigen Reste die Bedeutung gemäß einem oder mehreren der Ansprüche 1-8 haben und nicht für einen ggf. substituierten Aryl- oder Heteroaryl-Rest stehen, in einem Reaktionsmedium in Gegenwart einer Base mit Acethydroxamsäure der Formel III 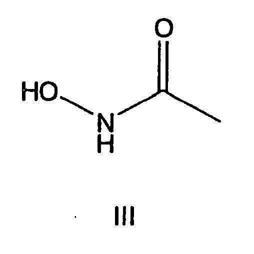 zu einer Verbindung der allgemeinen Formel I, 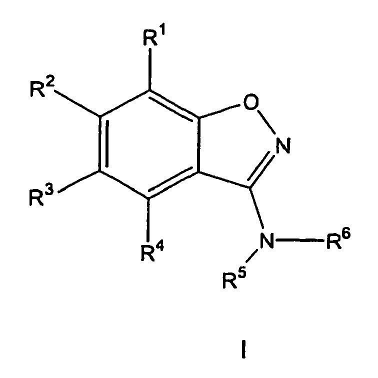 worin die Reste R¹-R⁴ die vorstehend genannte Bedeutung haben sowie die Reste R⁵ und R⁶ jeweils für einen Wasserstoff-Rest stehen, umgesetzt wird, diese ggf. gereinigt und/oder ggf. isoliert wird,
und diese ggf. anschließend in einem Reaktionsmedium mit wenigstens einem Isocyanat der allgemeinen Formel R¹⁹-N=C=O, worin R¹⁹ die Bedeutung gemäß einem oder mehreren der Ansprüche 1 bis 8 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹, R², R³, R⁴ und R⁵ die vorstehend genannte Bedeutung haben und R⁶ für -C(=O)-NR¹⁹R²⁰ steht, wobei R¹⁹ die vorstehend genannte Bedeutung hat und R²⁰ für einen Wasserstoff-Rest steht, und diese ggf. gereinigt und/oder ggf. isoliert wird
oder
diese ggf. anschließend in einem Reaktionsmedium mit wenigstens einem Isothiocyanat der allgemeinen Formel S=C=N-R²¹, worin R²¹ die Bedeutung gemäß einem oder mehreren der Ansprüche 1 bis 8 hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel I umgesetzt wird, worin R¹, R², R³, R⁴ und R⁵ die vorstehend genannte Bedeutung haben und R⁶ für -C(=S)-NR²¹R²² steht, wobei R²¹ die vorstehend genannte Bedeutung hat und R²² für einen Wasserstoff-Rest steht, und diese ggf. gereinigt und/oder ggf. isoliert wird,
und ggf. wenigstens eine Verbindung der allgemeinen Formel I, worin R¹, R², R³, R⁴ und R⁵ die vorstehend genannte Bedeutung haben und R⁶ für -C(=O)-NR¹⁹R²⁰ oder für -C(=S)-NR²¹R²² steht, wobei R¹⁹ und R²¹ die vorstehend genannte Bedeutung haben und R²⁰ und R²² jeweils für einen Wasserstoff-Rest stehen, in einem Reaktionsmedium, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R²⁰ oder der allgemeinen Formel LG-R²², worin LG für eine Abgangsgruppe, bevorzugt für ein Halogen-Atom, besonders bevorzugt für ein Chloratom steht, und R²² und R²⁴ jeweils die Bedeutung gemäß einem oder mehreren der Ansprüche 1-8 mit Ausnahme von Wasserstoff haben, zu wenigstens einer Verbindung der allgemeinen Formel I umgesetzt wird, worin R¹ bis R⁶ die vorstehend genannte Bedeutung haben, und diese ggf. gereinigt und/oder ggf. isoliert wird,
oder
ggf. wenigstens eine Verbindung der allgemeinen Formel I, worin R¹, R², R³, R⁴ die vorstehend genannte Bedeutung haben und R⁵ und R⁶ jeweils für H stehen, in einem Reaktionsmedium, in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R⁵ oder der allgemeinen Formel LG-R⁶, worin LG für eine Abgangsgruppe, bevorzugt für ein Halogen-Atom, besonders bevorzugt für ein Chloratom steht, und R⁵ und R⁶ jeweils die Bedeutung gemäß einem oder mehreren der Ansprüche 1-8 mit Ausnahme von Wasserstoff haben, zu wenigstens einer Verbindung der allgemeinen Formel I umgesetzt wird, worin R¹ bis R⁶ die vorstehend genannte Bedeutung haben, und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen in einem Reaktionsmedium, in Gegenwart wenigstens einer Säure und wenigstens eines Reduktionsmittels, bevorzugt in Gegenwart von Trifluoressigsäure und Triethylsilan, mit wenigstens einer Verbindung der allgemeinen Formel R⁵-(C=O)-H, worin R⁵ die Bedeutung gemäß einem oder mehreren der Ansprüche 1 bis 8 mit Ausnahme von H, -C(=O)R¹⁸, -C(=O)-NR¹⁹R²⁰, -C(=S)-NR²¹R²² und -S(=O)₂R²³ hat, zu wenigstens einer Verbindung der allgemeinen Formel I umgesetzt wird, worin R¹ bis R⁵ die vorstehend genannte Bedeutung haben und R⁶ für H steht, und diese ggf. gereinigt und/oder ggf. isoliert wird
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium mit wenigstens einer Verbindung der allgemeinen Formel R¹⁸-C(=O)-LG, worin R¹⁸ die Bedeutung gemäß einem oder mehreren der Ansprüche 1 bis 8 hat und LG für eine Abgangsgruppe, vorzugsweise für ein Halogenatom, besonders bevorzugt für ein Chloratom steht zu wenigstens einer Verbindung der allgemeinen Formel I umgesetzt wird, worin R¹ bis R⁴ die vorstehend genannte Bedeutung haben, R⁵ für einen -C(=O)R¹⁸-Rest und R⁶ für einen Wasserstoff-Rest steht; und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium in Gegenwart wenigstens eines Kupplungsreagenzes ggf. in Gegenwart wenigstens einer Base mit wenigstens einer Verbindung der allgemeinen Formel R¹⁸-C(=O)-OH, worin R¹⁸ die Bedeutung gemäß einem oder mehreren der Ansprüche 1 bis 8 hat zu wenigstens einer Verbindung der allgemeinen Formel I umgesetzt wird, worin R¹ bis R⁴ die vorstehend genannte Bedeutung haben, R⁵ für einen -C(=O)R¹⁸-Rest und R⁶ für einen Wasserstoff-Rest steht; und diese ggf. gereinigt und/oder ggf. isoliert wird,
oder
wenigstens eine Verbindung der allgemeinen Formel I, worin die Reste R¹-R⁴ die vorstehend genannte Bedeutung haben sowie die Reste R⁵ und R⁶ jeweils für H stehen, in einem Reaktionsmedium, ggf. in Gegenwart wenigstens einer Base, mit wenigstens einer Verbindung der allgemeinen Formel R²³-S(=O)₂-LG, worin R²³ die Bedeutung gemäß einem oder mehreren der Ansprüche 1 bis 8 hat und LG für eine Abgangsgruppe, bevorzugt für ein Halogen-Atom, besonders bevorzugt für ein Chloratom steht, zu wenigstens einer Verbindung der allgemeinen Formel I umgesetzt wird, worin R¹ bis R⁴ die vorstehend genannte Bedeutung haben, R⁵ für eine -S(=O)₂-R²³-Gruppe und R⁶ für einen Wasserstoff-Rest steht, und diese ggf. gereinigt und/oder ggf. isoliert wird,
und jeweils die so erhaltene Verbindung der allgemeinen Formel I, in der wenigstens einer der Reste R¹ bis R⁴ für eine Abgangsgruppe, vorzugsweise für einen Halogen-Rest oder einen Sulfonsäureester, besonders bevorzugt für eine Abgangsgruppe ausgewählt aus der Gruppe bestehend aus Chlor, Brom, Iod, Triflat, Mesylat und Tosylat, ganz besonders bevorzugt für ein Bromatom, steht, und die übrigen Reste die vorstehend genannte Bedeutung haben, ggf. in einem Reaktionsmedium, ggf. in Gegenwart wenigstens einer Base und eines Katalysators, der polymergebunden sein kann, bevorzugt in Gegenwart wenigstens einer Base ausgewählt aus der Gruppe bestehend aus Kaliumcarbonat, Natriumcarbonat, Kaliumphosphat, Natriumhydrogenphosphat, Cäsiumcarbonat, Triethylamin, [1,4]-Diazabicyclo-[2.2.2]-octan, Diisopropylamin, Diisopropylethylamin und N-Methylmorpholin und eines Katalysators, der polymergebunden sein kann, ausgewählt aus der Gruppe bestehend aus Palladium(II)acetat, Tris(dibenzylidenaceton)-dipalladium, Palladium(0)bis(dibenzylidenaceton), Tetrakis(triphenylphosphin)palladium(0), (1,1'-Bis(diphenylphosphino)-ferrocen)dichloro-palladium(II), Bis(acetonitril)dichloropalladium(II), Palladium(II)chlorid, Dichlorobis(triphenyl-phosphin)palladium(II), Dichloro(tricyclohexylphosphin)palladium(II), Bis(acetato)bis(triphenylphosphin)-palladium(II), Bistriphenylphosphinpalladium(II)dichlorid, Bistriphenyl-phosphinpalladium(II)acetat und Eisen(III)chlorid, besonders bevorzugt in Gegenwart von einem Alkalicarbonat und einem Palladiumkatalysator, ganz besonders bevorzugt in Gegenwart von Natriumcarbonat und Tetrakis(triphenylphosphin)-palladium (0), ggf. in Gegenwart wenigstens eines Liganden, der polymergebunden sein kann, bevorzugt in Gegenwart wenigstens eines Liganden, der polymergebunden sein kann, ausgewählt aus der Gruppe bestehend aus 2-Dicyclohexylphosphino-2',6'-dimethoxybiphenyl (S-Phos), 2-Dicyclohexylphosphino-2',4',6'-triisopropylbiphenyl (X-Phos), Tricyclohexylphosphin, Tricyclohexylphosphin Tetrafluoroborat, Tri-tert-butylphosphin Tetrafluoroborat, Triphenylphosphin und Imidazolium-Salzen, ggf. unter Verwendung von Mikrowellenstrahlung, mit wenigstens einer Boronsäure-Verbindung der folgenden Formel umsetzt, 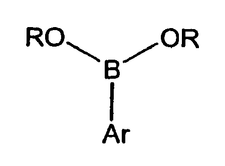 worin Ar für einen unsubstituierten oder wenigstens einfach substituierten Aryl- oder Heteroaryl-Rest gemäß einem oder mehreren der Ansprüche 1-8 steht und R für Wasserstoff oder einen organischen Rest, vorzugsweise für Wasserstoff oder einen Alkyl-Rest steht oder zwei Reste R zusammen mit der sie verbindenden -O-B-O-Gruppe einen Heterocycloalkyl-Rest bilden, und die so erhaltene Verbindung der allgemeinen Formel I, in der wenigstens einer der Reste R¹ bis R⁴ für einen unsubstituierten oder wenigstens einfach substituierten Aryl- oder Heteroaryl-Rest steht, ggf. reinigt und/oder ggf. isoliert,
oder jeweils die so erhaltene Verbindung der allgemeinen Formel I, in der wenigstens einer der Reste R¹ bis R⁴ für eine Abgangsgruppe, vorzugsweise für einen Halogen-Rest oder einen Sulfonsäureester, besonders bevorzugt für eine Abgangsgruppe ausgewählt aus der Gruppe bestehend aus Chlor, Brom, Iod, Triflat, Mesylat und Tosylat, ganz besonders bevorzugt für ein Bromatom, steht, und die übrigen Reste die vorstehend genannte Bedeutung haben, mit einer Organometall-Verbindung der allgemeinen Formel R'-M-X, worin R' für einen unsubstituierten oder wenigstens einfach substituierten Aryl- oder Heteroaryl-Rest gemäß einem oder mehreren der Ansprüche 1-8 steht, M für ein Übergangsmetall-Ion, vorzugsweise für ein Magnesium- oder Zink-Ion, und X für eine Abgangsgruppe, vorzugsweise für einen Halogen-Rest oder einen Sulfonsäureester, besonders bevorzugt für eine Abgangsgruppe ausgewählt aus der Gruppe bestehend aus Chlor, Brom, Iod, Triflat, Mesylat und Tosylat, ganz besonders bevorzugt für ein Bromatom, steht ggf. in wenigstens einem Reaktionsmedium, ggf. in Gegenwart wenigstens eines Katalysators, der polymergebunden sein kann, bevorzugt in Gegenwart eines Katalysators, der polymergebunden sein kann, ausgewählt aus der Gruppe bestehend aus Palladium(II)acetat, Tris(dibenzylidenaceton)dipalladium, Palladium(0)bis(dibenzylidenaceton), Tetrakis(triphenylphosphin)palladium(0), (1,1'-Bis(diphenylphosphino)-ferrocen)dichloropalladium(II), Bis(acetonitril)dichloropalladium(II), Palladium(II)chlorid, Dichlorobis(triphenylphosphin)palladium(II), Dichloro(tricyclohexylphosphin)palladium(II), Bis(acetato)bis(triphenylphosphin)-palladium(II), Bistriphenyl-phosphinpalladium(II)dichlorid, Bistriphenyl-phosphinpalladium(II)acetat und Eisen(III)chlorid, besonders bevorzugt in Gegenwart von Tetrakis(triphenylphosphin)-Palladium(0), umsetzt und die so erhaltene Verbindung der allgemeinen Formel I, in der wenigstens einer der Reste R¹ bis R⁴ für einen unsubstituierten oder wenigstens einfach substituierten Aryl-oder Heteroaryl-Rest steht, ggf. reinigt und/oder isoliert.

11. Arzneimittel enthaltend wenigstens eine Verbindung gemäß einem oder mehreren der Ansprüche 1 bis 8 und ggf. einen oder mehrere physiologisch verträgliche Hilfsstoffe.

12. Arzneimittel gemäß Anspruch 11 zur Behandlung und/oder Prophylaxe von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil.

13. Verwendung wenigstens einer Verbindung gemäß einem oder mehreren der Ansprüche 1 bis 8 zur Herstellung eines Arzneimittels zur Behandlung und/oder Prophylaxe von einer oder mehreren Erkrankungen ausgewählt aus der Gruppe bestehend Schmerz, vorzugsweise von Schmerz ausgewählt aus der Gruppe bestehend aus akutem Schmerz, chronischem Schmerz, neuropathischem Schmerz und visceralem Schmerz; Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil (DA-5018).

14. Verwendung gemäß Anspruch 13 zur Herstellung eines Arzneimittels zur Behandlung und/oder Prophylaxe von Schmerz ausgewählt aus der Gruppe bestehend aus akutem Schmerz, chronischem Schmerz, neuropathischem Schmerz und visceralem Schmerz.

## Claims

1. Substituted benzo[d]isoxazol-3-ylamine compounds of the general formula I, 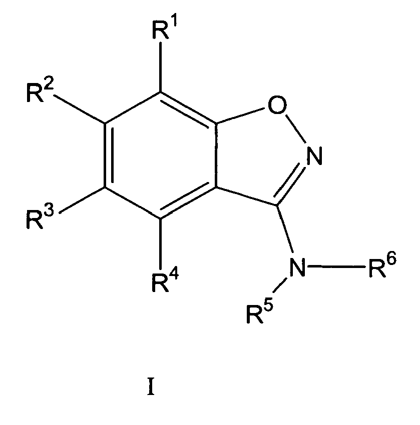 wherein
R¹, R², R³ and R⁴, independently of one another, respectively stand for
H; F, Cl, Br, I; -CN; -NC; -NO₂; -SF₅; -NR⁷R⁸; -OR⁹; -SR¹⁰; -C(=O)OR¹¹_{;} -(C=O)NR¹²R¹³; -S(=O)₂R¹⁴; -C(=O)R¹⁵; -NR¹⁶-S(=O)₂R¹⁷;
for a linear or branched, saturated or unsaturated, unsubstituted or at least mono-substituted aliphatic residue;
for a saturated or unsaturated, unsubstituted or at least mono-substituted cycloaliphatic residue, which possibly has at least one heteroatom as ring member, condenses with a monocyclic or polycyclic ring system and/or can be bonded via a linear or branched alkylene, alkenylene or alkinylene group;
or for an unsubstituted or substituted aryl or heteroaryl residue, which condenses with a mono- or polycyclic ring system and/or can be bonded via a linear or branched alkylene, alkenylene or alkinylene group,
wherein at least one of residues R¹, R², R³ and R⁴ stands for an unsubstituted or substituted aryl or heteroaryl residue and
wherein the above-mentioned substituted aryl or heteroaryl residues are possibly substituted respectively with 1, 2, 3, 4 or 5 substituents selected independently of one another from the group comprising F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-OH, -C(=O)-O-C₁₋₅-alkyl, -NH-(C₁₋₅-alkyl), -N(C₁₋₅-alkyl)(C₁₋₅-alkyl), -NH-C(=O)-O-(C₁₋₅-alkyl), -C(=O)-H, -C(=O)-(C₁₋₅-alkyl), -C(=O)-N(C₁₋₅-alkyl)(C₁₋₅-alkyl), -S(=O)₂NH₂; -S(=O)₂N(H)(C₁₋₅-alkyl); -S(=O)₂N(C₁₋₅-alkyl)(C₁₋₅-alkyl); -S(=O)₂phenyl; -S(=O)₂-C₁₋₅-alkyl; -C(=O)-NH-CH₃, -C(=O)-NH-C₂H₅, -C(=O)-N(cyclopropyl)-C₁₋₅-alkyl, -N(cyclopropyl)-S(=O)₂-C₁₋₅-alkyl, -CH₂-NH-S(=O)₂-C₁₋₅-alkyl, -CH₂-N(cyclopropyl)-S(=O)₂-C₁₋₅-alkyl, -CH₂-N(cyclopropyl)-C(=O)-C₁₋₅-alkyl, -CH₂-N(cyclopropyl)-C(=O)-phenyl, -O-phenyl, -O-benzyl, phenyl, benzyl, thiophenyl (thienyl), furanyl and pyridinyl, wherein the cyclic part of residues -CH₂-N(cyclopropyl)-C(=O)-phenyl, -O-phenyl, -O-benzyl, phenyl, -S(=O)₂phenyl, benzyl, thiophenyl (thienyl), furanyl and pyridinyl can possibly be substituted respectively with 1, 2, 3, 4 or 5 substituents selected independently of one another from the group comprising F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
R⁵ and R⁶, independently of one another, respectively stand for
H; -C(=O)R¹⁸; -C(=O)NR¹⁹R²⁰; -C(=S)NR²¹R²²; -S(=O)₂R²³;
for a linear or branched, saturated or unsaturated, unsubstituted or at least mono-substituted aliphatic residue;
for a saturated or unsaturated, unsubstituted or at least mono-substituted cycloaliphatic residue which possibly has at least one heteroatom as ring member, condenses with a mono- or polycyclic ring system and/or can be bonded via a linear or branched alkylene group;
or for an unsubstituted or at least mono-substituted aryl or heteroaryl residue which condenses with a mono- or polycyclic ring system and/or can be bonded via a linear or branched alkylene group,
wherein the abovementioned aryl residue is possibly substituted respectively with 1, 2, 3, 4 or 5 substituents selected independently of one another from the group comprising F, Cl, Br, I, -CN, -CF₃, -SF₅, -OH, -O-C₁₋₅-alkyl, -O-CF₃, -S-CF₃, -SH, -S-C₁₋₅-alkyl, -C₁₋₅-alkyl, -C(=O)-OH, -C(=O)-O-C₁₋₅-alkyl, -NH-C₁₋₅-alkyl, -N(C₁₋₅-alkyl)₂, -NH-C(=O)-O-C₁₋₅-alkyl, -C(=O)-H, -C(=O)-C₁₋₅-alkyl, -C(=O)-NH₂, -C(=O)NH-C₁₋₅-alkyl, -C(=O)-N(C₁₋₅-alkyl)₂, -S(=O)₂NH₂; -S(=O)₂N(H)(C₁₋₅-alkyl); -S(=O)₂N(C₁₋₅-alkyl)(C₁₋₅-alkyl); -S(=O)₂phenyl; -S(=O)₂-C₁₋₅-alkyl; -CH₂-NH-cyclopropyl; -CH₂-N(C₁₋₅-alkyl)-cyclopropyl; -C(=O)-N(cyclopropyl)-C₁₋₅-alkyl, -N(cyclopropyl)-S(=O)₂-C₁₋₅-alkyl, -CH₂-NH-S(=O)₂-C₁₋₅-alkyl, -CH₂-N(cyclopropyl)-S(=O)₂-C₁₋₅-alkyl, -CH₂-N(cyclopropyl)-C(=O)-C₁₋₅-alkyl, -CH₂-N(cyclopropyl)-C(=O)-phenyl, -O-phenyl, -O-benzyl, phenyl, benzyl, thiophenyl (thienyl), furanyl and pyridinyl, wherein the cyclic part of residues -CH₂-N(cyclopropyl)-C(=O)-phenyl, -O-phenyl, -O-benzyl, phenyl, -S(=O)₂phenyl and benzyl can be respectively substituted with 1, 2, 3, 4 or 5 substituents selected independently of one another from the group comprising F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
on condition that R⁵ and R⁶ do not simultaneously stand for a hydrogen residue;
or
R⁵ and R⁶ together with the nitrogen atom which connects them form as ring member a saturated or unsaturated, unsubstituted or at least mono-substituted heterocycloaliphatic residue which possibly has at least one further heteroatom as ring member,
R⁷ and R⁸, independently of one another, respectively stand for
H, -C(=O)R¹⁵ or for a linear or branched, saturated or unsaturated, unsubstituted or at least mono-substituted aliphatic residue, or
R⁷ and R⁸ together with the nitrogen atom which connects them form as ring member a saturated or unsaturated, unsubstituted or at least mono-substituted heterocycloaliphatic residue which possibly has at least one further heteroatom as ring member,
R⁹, R¹⁰, R¹¹ and R¹⁶, independently of one another, respectively stand for
H;
for a linear or branched, saturated or unsaturated, unsubstituted or at least mono-substituted aliphatic residue;
for a saturated or unsaturated, unsubstituted or at least mono-substituted cycloaliphatic residue which possibly has at least one heteroatom as ring member, condenses with a mono- or polycyclic ring system and/or can be bonded via a linear or branched alkylene group;
or for an unsubstituted or at least mono-substituted aryl or heteroaryl residue which condenses with a mono- or polycyclic ring system and/or can be bonded via a linear or branched alkylene group;
R¹² and R¹³, independently of one another, respectively stand for
H or for a linear or branched, saturated or unsaturated, unsubstituted or at least mono-substituted aliphatic residue, or
R¹² and R¹³ together with the nitrogen atom which connects them form as ring member a saturated or unsaturated, unsubstituted or at least mono-substituted heterocycloaliphatic residue which possibly has at least one further heteroatom as ring member,
R¹⁴ and R²³, independently of one another, respectively stand for
-NR⁷R⁸;
for a linear or branched, saturated or unsaturated, unsubstituted or at least mono-substituted aliphatic residue,
for a saturated or unsaturated, unsubstituted or at least mono-substituted cycloaliphatic residue, which possibly has at least one heteroatom as ring member, condenses with a mono- or polycyclic ring system and/or can be bonded via a linear or branched alkylene group,
or for an unsubstituted or at least mono-substituted aryl or heteroaryl residue which condenses with a mono- or polycyclic ring system and/or can be bonded via a linear or branched alkylene group,
R¹⁵, R¹⁷ and R¹⁸, independently of one another, respectively stand
for a linear or branched, saturated or unsaturated, unsubstituted or at least mono-substituted aliphatic residue,
for a saturated or unsaturated, unsubstituted or at least mono-substituted cycloaliphatic residue, which possibly has at least one heteroatom as ring member, condenses with a mono- or polycyclic ring system and/or can be bonded via a linear or branched alkylene group,
or for an unsubstituted or at least mono-substituted aryl or heteroaryl residue, which condenses with a mono- or polycyclic ring system and/or can be bonded via a linear or branched alkylene group,
R¹⁹ and R²⁰, independently of one another, respectively stand for
H or for a linear or branched, saturated or unsaturated, unsubstituted or at least mono-substituted aliphatic residue, or
R¹⁹ and R²⁰ together with the nitrogen atom which connects them form as ring member a saturated or unsaturated, unsubstituted or at least mono-substituted heterocycloaliphatic residue which possibly has at least one further heteroatom as ring member,
R²¹ and R²², independently of one another, respectively stand for
H or for a linear or branched, saturated or unsaturated, unsubstituted or at least mono-substituted aliphatic residue, or
R²¹ and R²² together with the nitrogen atom which connects them form as ring member a saturated or unsaturated, unsubstituted or at least mono-substituted heterocycloaliphatic residue which possibly has at least one further heteroatom as ring member,
where applicable, respectively in the form of one of their pure stereoisomers, in particular enantiomers or diastereomers, their racemates or in the form of a mixture of stereoisomers, in particular the enantiomers and/or diastereomers, in any desired mixture ratio, or respectively in the form of corresponding salts, or respectively in the form of corresponding solvates.

2. Compounds according to claim 1, **characterised in that**
R¹, R², R³ and R⁴, independently of one another, respectively stand for
H; F; Cl; Br; I; -CN; -NC; -NO₂; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(-O)₂R¹⁷; C₁₋₁₀-alkyl, C₂₋₁₀-alkenyl, C₂₋₁₀-alkinyl; C₃₋₈-cycloalkyl; -(C₁₋₅-alkylene)-C₃₋₈-cycloalkyl; heterocycloalkyl; -(C₁₋₅-alkylene)-heterocycloalkyl; aryl; heteroaryl; -(C₁₋₅-alkylene)-aryl; -(C₁₋₅-alkylene)-heteroaryl;
wherein at least one of the residues R¹, R², R³ and R⁴ stands for an aryl or heteroaryl residue;
R⁵ and R⁶, independently of one another, respectively stand for H; -C(=O)R¹⁸; -C(=O)NR¹⁹R²⁰; -C(=S)NR²¹R²²; -S(=O)₂R²³; C₁₋₁₀-alkyl, C₂₋₁₀-alkenyl, C₂₋₁₀-alkinyl; C₃₋₈-cycloalkyl; -(C₁₋₅-alkylene)-C₃₋₈-cycloalkyl; heterocycloalkyl; -(C₁₋₅-alkylene)-heterocycloalkyl; aryl; heteroaryl; -(C₁₋₅-alkylene)-aryl or -(C₁₋₅-alkylene)-heteroaryl;
on condition that R⁵ and R⁶ do not simultaneously stand for a hydrogen residue;
or
R⁵ and R⁶ together with the nitrogen atom which connects them form as ring member a residue which is selected from group H comprising 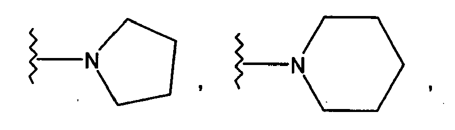 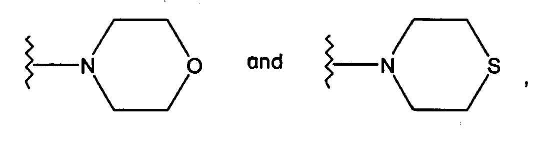 R⁷ and R⁸, independently of one another, respectively stand for
H, -C(=O)R¹⁵ or C₁₋₁₀-alkyl, or
R⁷ and R⁸ together with the nitrogen atom which connects them form as ring member a residue which is selected from group H indicated above;
R⁹, R¹⁰, R¹¹ and R¹⁶, independently of one another, respectively stand for
H; C₁₋₁₀-alkyl, C₂₋₁₀-alkenyl, C₂₋₁₀-alkinyl; C₃₋₈-cycloalkyl; -(C₁₋₅-alkylene)-C₃₋₈-cycloalkyl; heterocycloalkyl; -(C₁₋₅-alkylene)-heterocycloalkyl; aryl; heteroaryl; -(C₁₋₅-alkylene)-aryl or -(C₁₋₅-alkylene)-heteroaryl;
R¹² and R¹³, independently of one another, respectively stand for H or for a C₁₋₁₀-alkyl residue; or
R¹² and R¹³ together with the nitrogen atom which connects them form as ring member a residue which is selected from group H indicated above;
R¹⁴ and R²³, independently of one another, respectively stand for
-NR⁷R⁸; C₁₋₁₀-alkyl, C₂₋₁₀-alkenyl, C₂₋₁₀-alkinyl; C₃₋₈-cycloalkyl; -(C₁₋₅-alkylene)-C₃₋₈-cycloalkyl; heterocycloalkyl; -(C₁₋₅-alkylene)-heterocycloalkyl; aryl; heteroaryl; -(C₁₋₅-alkylene)-aryl or -(C₁₋₅-alkylene)-heteroaryl;
R¹⁵, R¹⁷ and R¹⁸, independently of one another, respectively stand for
C₁₋₁₀-alkyl, C₂₋₁₀-alkenyl, C₂₋₁₀-alkinyl; C₃₋₈-cycloalkyl; -(C₁₋₅-alkylene)-C₃₋₈-cycloalkyl; heterocycloalkyl; -(C₁₋₅-alkylene)-heterocycloalkyl; aryl; heteroaryl; -(C₁₋₅-alkylene)-aryl or -(C₁₋₅-alkylene)-heteroaryl;
R¹⁹ and R²⁰, independently of one another, respectively stand for
H or for C₁₋₁₀-alkyl, or
R¹⁹ and R²⁰ together with the nitrogen atom which connects them form as ring member a residue which is selected from group H indicated above;
R²¹ and R²², independently of one another, respectively stand for
H or for C₁₋₁₀-alkyl, or
R²¹ and R²² together with the nitrogen atom which connects them form as ring member a residue which is selected from group H indicated above;
wherein
the above-mentioned C₁₋₁₀-alkyl, C₂₋₁₀-alkenyl and C₂₋₁₀-alkinyl residues are respectively linear or branched and, where applicable, can be substituted with 1, 2, 3, 4 or 5 substituents selected independently of one another from the group comprising F; Cl; Br; -CN; -OH; -SH; -O-C₁₋₂-alkyl; -S-C₁₋₂-alkyl and -NH₂,
the above-mentioned C₃₋₈-cycloalkyl residues can possibly be substituted respectively with 1, 2, 3, 4 or 5 substituents selected independently of one another from the group comprising F; Cl; Br; -CN; -OH; -SH; -C₁₋₅-alkyl; -O-C₁₋₂-alkyl; -S-C₁₋₂-alkyl and -NH₂,
the above-mentioned heterocycloalkyl residues respectively have 4, 5, 6 or 7 members, have 1 or 2 heteroatoms selected independently of one another from the group comprising oxygen, sulphur and nitrogen as ring member(s) and can possibly be substituted with 1, 2, 3, 4 or 5 substituents selected independently of one another from the group comprising F; Cl; Br; -CN; -OH; -SH; -C₁₋₅-alkyl; -O-C₁₋₂-alkyl; -S-C₁₋₂-alkyl and -NH₂,
the above-mentioned aryl residues respectively stand for a phenyl or naphthyl residue which can be substituted with 1, 2, 3, 4 or 5 substituents selected independently of one another from the group comprising F; Cl; Br; I; -CF₃; -OCF₃; -SCF₃; -SF₅; -CN; -OH; -SH; -C₁₋₅-alkyl; -O-C₁₋₅-alkyl; -S-C₁₋₅-alkyl; -C(=O)-NH-CH₃, -C(=O)-NH-C₂H₅, -C(=O)-OH; -C(=O)-OC₁₋₅-alkyl; -N(H)(C₁₋₅-alkyl); -N(C₁₋₅-alkyl)(C₁₋₅-alkyl); -C(=O)-H; -C(=O)-C₁₋₅-alkyl; -C(=O)N(C₁₋₅-alkyl)(C₁₋₅-alkyl); -S(=O)₂NH₂; -S(=O)₂N(H)(C₁₋₅-alkyl); -S(=O)₂N(C₁₋₅-alkyl)(C₁₋₅-alkyl); -S(=O)₂-phenyl; -S(=O)₂-C₁₋₅-alkyl; -C(=O)-N(cyclopropyl)-C₁₋₅-alkyl, -N(cyclopropyl)-S(=O)₂-C₁₋₅-alkyl, -CH₂-NH-S(=O)₂-C₁₋₅-alkyl, -CH₂-N(cyclopropyl)-S(=O)₂-C₁₋₅-alkyl, -CH₂-N(cyclopropyl)-C(=O)-C₁₋₅-alkyl, -CH₂-N(cyclopropyl)-C(=O)-phenyl, phenyl; phenoxy, benzyl; thiophenyl (thienyl), furanyl and pyridinyl,
the above-mentioned heteroaryl residues area respectively have 5 or 6 members, have 1, 2 or 3 heteroatoms selected independently of one another from the group comprising oxygen, sulphur and nitrogen as ring member(s) and can possibly be substituted with 1, 2, 3, 4 or 5 substituents selected independently of one another from the group comprising F; Cl; Br; I; -CF₃; -OCF₃; -SCF₃; -SF₅; -CN; -OH; -SH; -C₁₋₅-alkyl; -O-C₁₋₅-alkyl; -S-C₁₋₅-alkyl; -C(=O)-OH; -C(=O)-OC₁₋₅-alkyl; -NH₂; -N(H)(C₁₋₅-alkyl); -N(C₁₋₅-alkyl)(C₁₋₅-alkyl); -C(=O)-NH-CH₃, -C(=O)-NH-C₂H₅, -C(=O)N(C₁₋₅-alkyl)(C₁₋₅-alkyl); -S(=O)₂NH₂; -S(=O)₂N(H)(C₁₋₅-alkyl); -S(=O)₂N(C₁₋₅-alkyl)(C₁₋₅-alkyl); -S(=O)₂-phenyl; -S(=O)₂-C₁₋₅-alkyl; -CH₂-NH-cyclopropyl; -CH₂-N(C₁₋₅-alkyl)-cyclopropyl; -C(=O)-N(cyclopropyl)-C₁₋₅-alkyl, -N(cyclopropyl)-S(=O)₂-C₁₋₅-alkyl, -CH₂-NH-S(=O)₂-C₁₋₅-alkyl, -CH₂-N(cyclopropyl)-S(=O)₂-C₁₋₅-alkyl, -CH₂-N(cyclopropyl)-C(=O)-C₁₋₅-alkyl, -CH₂-N(cyclopropyl)-C(=O)-phenyl, phenyl; phenoxy, benzyl; thiophenyl (thienyl), furanyl and pyridinyl,
where applicable, respectively in the form of one of their pure stereoisomers, in particular enantiomers or diastereomers, their racemates or in the form of a mixture of stereoisomers, in particular of enantiomers and/or diastereomers, in any desired mixture ratio, or respectively in the form of corresponding salts, or respectively in the form of corresponding solvates.

3. Compounds according to claim 1 or 2, **characterised in that**
R¹, R², R³ and R⁴, independently of one another, respectively stand for
H; F; Cl; Br; I; -CN; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; C₁₋₄-alkyl, C₂₋₄-alkenyl, C₂₋₄-alkinyl; C₃₋₈-cycloalkyl; -(C_{1, 2 or 3}-alkylene)-C₃₋₈-cycloalkyl; heterocycloalkyl; -(C_{1, 2 or 3}-alkylene)-heterocycloalkyl; aryl; heteroaryl; -(C_{1, 2 or 3}-alkylene)-aryl or -(C_{1, 2 or 3}-alkylene)-heteroaryl;
wherein at least one of residues R¹, R², R³ and R⁴ stands for an aryl or heteroaryl residue;
R⁵ and R⁶, independently of one another, respectively stand for
H; -C(=O)R¹⁸; -C(=O)NR¹⁹R²⁰; -C(=S)NR²¹R²²; -S(=O)₂R²³; C₁₋₄-alkyl, C₂₋₄-alkenyl, C₂₋₄-alkinyl; C₃₋₈-cycloalkyl; -(C_{1, or 3}-alkylene)-C₃₋₈-cycloalkyl; heterocycloalkyl; -(C_{1, 2 or 3}-alkylene)-heterocycloalkyl; aryl; heteroaryl; -(C_{1, 2 or 3}-alkylene)-aryl or -(C_{1, 2 or 3}-alkylene)-heteroaryl;
on condition that R⁵ and R⁶ do not simultaneously stand for a hydrogen residue;
or
R⁵ and R⁶ together with the nitrogen atom which connects them form as ring member a residue which is selected from group H comprising 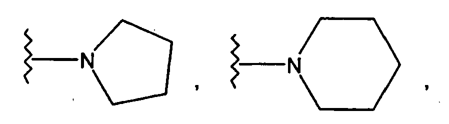 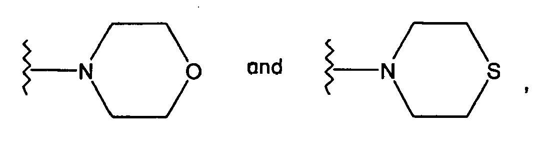 R⁷ and R⁸, independently of one another, respectively stand for
H, -C(=O)R¹⁵ or C₁₋₄-alkyl, or
R⁷ and R⁸ together with the nitrogen atom which connects them form as ring member a residue which is selected from group H indicated above;
R⁹, R¹⁰, R¹¹ and R¹⁶, independently of one another, respectively stand for
H; C₁₋₄-alkyl, C₂₋₄-alkenyl, C₂₋₄-alkinyl; C₃₋₈-cycloalkyl; -(C_{1, 2 or 3}-alkylene)-C₃₋₈-cycloalkyl; heterocycloalkyl; -(C_{1, 2 or 3}-alkylene)-heterocycloalkyl; aryl; heteroaryl; -(C_{1, 2 or 3}-alkylene)-aryl or -(C_{1, 2 or 3}-alkylene)-heteroaryl;
R¹² and R¹³, independently of one another, respectively stand for
H or for C₁₋₄-alkyl; or
R¹² and R¹³ together with the nitrogen atom which connects them form as ring member a residue which is selected from group H indicated above;
R¹⁴ and R²³, independently of one another, respectively stand for
-NR⁷R⁸; C₁₋₄-alkyl, C₂₋₄-alkenyl, C₂₋₄-alkinyl; C₃₋₈-cycloalkyl; -(C_{1, 2 or 3}-alkylene)-C₃₋₈-cycloalkyl; heterocycloalkyl; -(C_{1, 2 or 3}-alkylene)-heterocycloalkyl; aryl; heteroaryl; -(C_{1, 2 or 3}-alkylene)-aryl or -(C_{1, 2 or 3}-alkylene)-heteroaryl;
R¹⁵, R¹⁷ and R¹⁸, independently of one another, respectively stand for
C₁₋₄-alkyl, C₂₋₄-alkenyl, C₂₋₄-alkinyl; C₃₋₈-cycloalkyl; -(C_{1, 2 or 3}-alkylene)-C₃₋₈-cycloalkyl; heterocycloalkyl; -(C_{1, 2 or 3}-alkylene)-heterocycloalkyl; aryl; heteroaryl; -(C_{1, 2 or 3}-alkylene)-aryl or -(C_{1, 2 or 3}-alkylene)-heteroaryl;
R¹⁹ and R²⁰, independently of one another, respectively stand for
H or for C₁₋₄-alkyl, or
R¹⁹ and R²⁰ together with the nitrogen atom which connects them form as ring member a residue which is selected from group H indicated above;
R²¹ and R²², independently of one another, respectively stand for
H or for C₁₋₄-alkyl, or
R²¹ and R²² together with the nitrogen atom which connects them form as ring member a residue which is selected from group H indicated above;
wherein
the above-mentioned C₁₋₄-alkyl-, C₂₋₄-alkenyl- and C₂₋₄-alkinyl residues are respectively linear or branched and, where applicable, can be substituted with 1, 2, 3, 4 or 5 substituents selected independently of one another from the group comprising F; Cl; Br; -CN; -OH; -OCH₃ and -NH₂,
the above-mentioned C₃₋₈-cycloalkyl residues can possibly be substituted respectively with 1, 2, 3, 4 or 5 substituents selected independently of one another from the group comprising F; Cl; Br; -CN; -OH; -CH₃; -C₂H₅; -OCH₃; and -NH₂,
the above-mentioned heterocycloalkyl residues respectively have 4, 5, 6 or 7 members, have 1 or 2 heteroatoms selected independently of one another from the group comprising oxygen, sulphur and nitrogen as ring member(s) and, where applicable, can be substituted with 1, 2, 3, 4 or 5 substituents selected independently of one another from the group comprising F; Cl; Br; -CN; -OH; -CH₃; -C₂H₅; -OCH₃; and -NH₂,
the above-mentioned aryl residues respectively stand for a phenyl or naphthyl residue which can be substituted with 1, 2, 3, 4 or 5 substituents selected independently of one another from the group comprising F; Cl; Br; -CF₃; -OCF₃; -SCF₃; -SF₅; -CN; -OH; methyl; ethyl; n-propyl; isopropyl; n-butyl; isobutyl; sec-butyl; tert-butyl; methoxy; ethoxy; -N(CH₃)₂; -N(C₂H₅)₂; phenyl; phenoxy, benzyl; thiophenyl (thienyl), furanyl, pyridinyl, -C(=O)-NH-CH₃, -C(=O)-NH-C₂H₅, -C(=O)-H, -C(=O)-CH₃, -C(=O)-C₂H₅, -C(=O)-CH(CH₃)₂, -C(-O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(cyclopropyl)-CH₃, -C(=O)-N(cyclopropyl)-C₂H₅, -C(=O)-N(cyclopropyl)-CH(CH₃)₂, -C(=O)-N(cyclopropyl)-C(CH₃)₃, -CH₂-NH-cyclopropyl, -CH₂-N(cyclopropyl)-S(=O)₂-CH₃, -CH₂-N(cyclopropyl)-S(=O)₂-C₂H₅, -CH₂-N(cyclopropyl)-C(=O)-CH₃, -CH₂-N(cyclopropyl)-C(=O)-C₂H₅, -CH₂-N(cyclopropyl)-C(=O)-CH(CH₃)₂, -CH₂-N(cyclopropyl)-C(=O)-C(CH₃)₃ and -CH₂-N(cyclopropyl)-C(=O)-phenyl;
the above-mentioned heteroaryl residues respectively stand for a furanyl, thienyl (thiophenyl) or pyridinyl residue and, where applicable, can be substituted with 1, 2, 3, 4 or 5 substituents selected independently of one another from the group comprising F; Cl; Br; -CF₃; -OCF₃; -SCF₃; -SF₅; -CN; -OH; -C(=O)-NH-CH₃, -C(=O)-NH-C₂H₅, methyl; ethyl; n-propyl; isopropyl; n-butyl; isobutyl; sec-butyl; tert-butyl; methoxy; ethoxy; -NH₂; -N(CH₃)₂; -N(C₂H₅)₂; phenyl; phenoxy, benzyl; thiophenyl (thienyl), furanyl, pyridinyl, -C(=O)-H, -C(=O)-CH₃, -C(=O)-C₂H₅, -C(=O)-CH(CH₃)₂, -C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(cyclopropyl)-CH₃, -C(=O)-N(cyclopropyl)-C₂H₅, -C(=O)-N(cyclopropyl)-CH(CH₃)₂, -C(=O)-N(cyclopropyl)-C(CH₃)₃, -CH₂-NH-cyclopropyl, -CH₂-N(cyclopropyl)-S(=O)₂-CH₃, -CH₂-N(cyclopropyl)-S(=O)₂-C₂H₅, -CH₂-N(cyclopropyl)-C(=O)-CH₃, -CH₂-N(cyclopropyl)-C(=O)-C₂H₅, -CH₂-N(cyclopropyl)-C(=O)-CH(CH₃)₂, -CH₂-N(cyclopropyl)-C(=O)-C(CH₃)₃ and -CH₂-N(cyclopropyl)-C(=O)-phenyl,
respectively, where applicable, in the form of one of their pure stereoisomers, in particular enantiomers or diastereomers, their racemates or in the form of a mixture of stereoisomers, in particular enantiomers and/or diastereomers, in any desired mixture ratio, or respectively in the form of corresponding salts, or respectively in the form of corresponding solvates.

4. Compounds according to one or more of claims 1-3 of the general formula I', 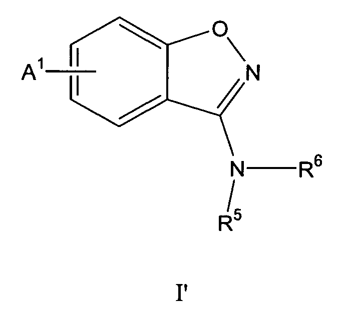 wherein
A¹ stands for a (hetero)aryl residue which is selected from the group comprising phenyl, 1-naphthyl, 2-naphthyl, thiophenyl (thienyl), furanyl and pyridinyl and which, where applicable, can be substituted with 1, 2, 3, 4 or 5 substituents selected independently of one another from the group comprising F; Cl; Br; -CF₃; -OCF₃; -SCF₃; -SF₅; -CN; -OH; methyl; ethyl; n-propyl; isopropyl; n-butyl; isobutyl; sec-butyl; tert-butyl; methoxy; ethoxy; -NH₂; -N(CH₃)₂; -N(C₂H₅)₂; phenyl; phenoxy, benzyl; thiophenyl (thienyl), furanyl, pyridinyl, -C(=O)-NH-CH₃, -C(=O)-NH-C₂H₅, -C(=O)-H, -C(=O)-CH₃, -C(=O)-C₂H₅, -C(=O)-CH(CH₃)₂, -C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(cyclopropyl)-CH₃, -C(=O)-N(cyclopropyl)-C₂H₅, -C(=O)-N(cyclopropyl)-CH(CH₃)₂, -C(=O)-N(cyclopropyl)-C(CH₃)₃, -CH₂-NH-cyclopropyl, -CH₂-N(cyclopropyl)-S(=O)₂-CH₃, -CH₂-N(cyclopropyl)-S(=O)₂-C₂H₅, -CH₂-N(cyclopropyl)-C(=O)-CH₃, -CH₂-N(cyclopropyl)-C(=O)-C₂H₅, -CH₂-N(cyclopropyl)-C(=O)-CH(CH₃)₂, -CH₂-N(cyclopropyl)-C(=O)-C(CH₃)₃ and -CH₂-N(cyclopropyl)-C(=O)-phenyl;
R⁵ and R⁶, independently of one another, respectively stand for
H; an alkyl residue selected from the group comprising methyl; ethyl; n-propyl; isopropyl; n-butyl; isobutyl; sec-butyl, tert-butyl, n-pentyl and neopentyl; for -C(=O)-phenyl; -(CH₂)-phenyl; -(CH₂)₂-phenyl; -(CH₂)₃-phenyl; -(CH₂)-pyridinyl; -(CH₂)₂-pyridinyl; -(CH₂)₃-pyridinyl; wherein the above-mentioned phenyl and pyridinyl residues, where applicable, can be substituted with 1, 2, 3, 4 or 5 substituents selected independently of one another from the group comprising F; Cl; Br; -CF₃; -OCF₃; -SCF₃; -SF₅; -CN; -OH; methyl; ethyl; n-propyl; isopropyl; n-butyl; isobutyl; sec-butyl; tert-butyl; methoxy; ethoxy; -N(CH₃)₂; -N(C₂H₅)₂; phenyl; phenoxy, benzyl; thiophenyl (thienyl), furanyl and pyridinyl;
on condition that R⁵ and R⁶ do not simultaneously stand for a hydrogen residue;
or
R⁵ and R⁶ together with the nitrogen atom which connects them form as ring member a residue which is selected from group H comprising 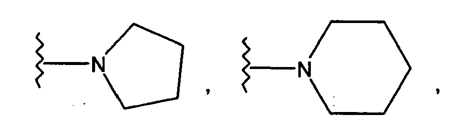 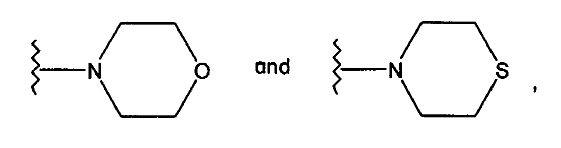 respectively, where applicable, in the form of corresponding salts, in particular hydrochloride addition salts, or respectively in the form of corresponding solvates.

5. Compounds according to one or more of claims 1-4: 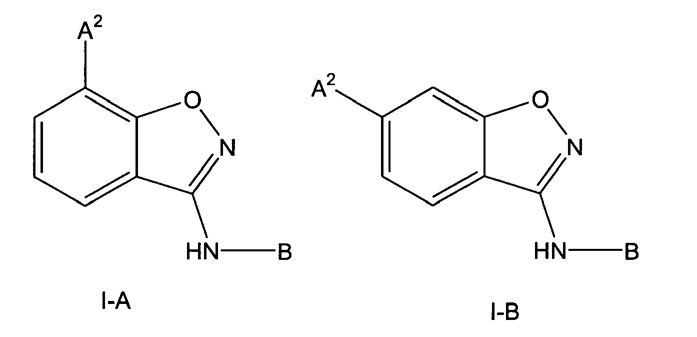 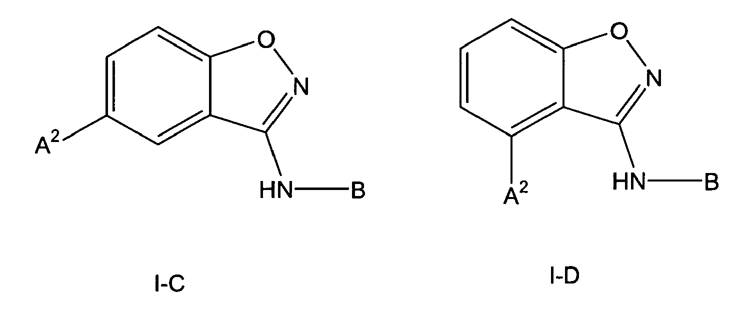 wherein respectively
A² stands for a (hetero)aryl residue which is selected from the group comprising phenyl, 1-naphthyl, 2-naphthyl, thiophenyl (thienyl), furanyl and pyridinyl and which can be substituted, where applicable, with 1, 2, 3, 4 or 5 substituents selected independently of one another from the group comprising F; Cl; Br; -CF₃; -OCF₃; -SCF₃; -SF₅; -CN; -OH; methyl; ethyl; n-propyl; isopropyl; n-butyl; isobutyl; sec-butyl; tert-butyl; methoxy; ethoxy; -NH₂; -N(CH₃)₂; -N(C₂H₅)₂; phenyl; phenoxy, benzyl; thiophenyl (thienyl), furanyl, pyridinyl, -C(=O)-NH-CH₃, -C(=O)-NH-C₂H₅, -C(=O)-H, -C(=O)-CH₃, -C(=O)-C₂H₅, -C(=O)-CH(CH₃)₂, -C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(cyclopropyl)-CH₃, -C(=O)-N(cyclopropyl)-C₂H₅, -C(=O)-N(cyclopropyl)-CH(CH₃)₂, -C(=O)-N(cyclopropyl)-C(CH₃)₃, -CH₂-NH-cyclopropyl, -CH₂-N(cyclopropyl)-S(=O)₂-CH₃, -CH₂-N(cyclopropyl)-S(=O)₂-C₂H₅, -CH₂-N(cyclopropyl)-C(=O)-CH₃, -CH₂-N(cyclopropyl)-C(=O)-C₂H₅, -CH₂-N(cyclopropyl)-C(=O)-CH(CH₃)₂, -CH₂-N(cyclopropyl)-C(=O)-C(CH₃)₃ and -CH₂-N(cyclopropyl)-C(=O)-phenyl;
B stands for an alkyl residue selected from the group comprising methyl; ethyl; n-propyl; isopropyl; n-butyl; isobutyl; sec-butyl, tert-butyl, n-pentyl and neopentyl; for -C(=O)-phenyl; -(CH₂)-phenyl; -(CH₂)₂-phenyl; -(CH₂)₃-phenyl; -(CH₂)-pyridinyl; -(CH₂)₂-pyridinyl; -(CH₂)₃-pyridinyl; wherein the above-mentioned phenyl and pyridinyl residues can, where applicable, be substituted with 1, 2, 3, 4 or 5 substituents selected independently of one another from the group comprising F; Cl; Br; -CF₃; -OCF₃; -SCF₃; -SF₅; -CN; -OH; methyl; ethyl; n-propyl; isopropyl; n-butyl; isobutyl; sec-butyl; tert-butyl; methoxy; ethoxy; -N(CH₃)₂; -N(C₂H₅)₂; phenyl; phenoxy, benzyl; thiophenyl (thienyl), furanyl and pyridinyl,
respectively, where applicable, in the form of corresponding salts, in particular hydrochloride addition salts, or respectively in the form of corresponding solvates.

6. Compounds according to claim 5, **characterised in that** A² stands for one of the following residues A²¹ or A²²: 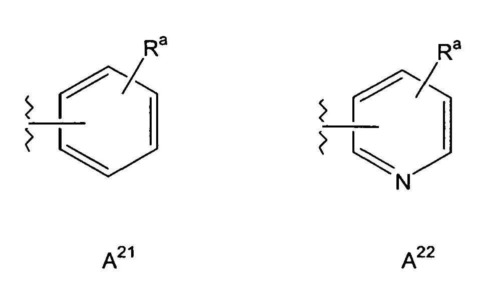 wherein
R^{a} respectively stands for a residue which is selected from the group comprising H, F, Cl, Br, I, -CN; -CF₃, -OCF₃, -SCF₃, -SF₅, -OH, -SH, methyl; ethyl; n-propyl; isopropyl; n-butyl; isobutyl; sec-butyl; tert-butyl; -OCH₃; -OC₂H₅; -S-CH₃, -S-C₂H₅, -C(=O)-NH-CH₃, -C(=O)-NH-C₂H₅, -C(=O)-H, -C(=O)-O-C₂H₅, -C(=O)-OH, -CH₂-NH-cyclopropyl, -CH₂-N(cyclopropyl)-S(=O)₂-CH₃, -CH₂-N(cyclopropyl)-C(=O)-CH₃ and -CH₂-N(cyclopropyl)-C(=O)-phenyl and
B stands for an alkyl residue selected from the group comprising methyl, ethyl, n-propyl, isopropyl, n-butyl, isobutyl, sec-butyl, tert-butyl, n-pentyl and neopentyl;
or for one of the following residues B¹, B² or B³, 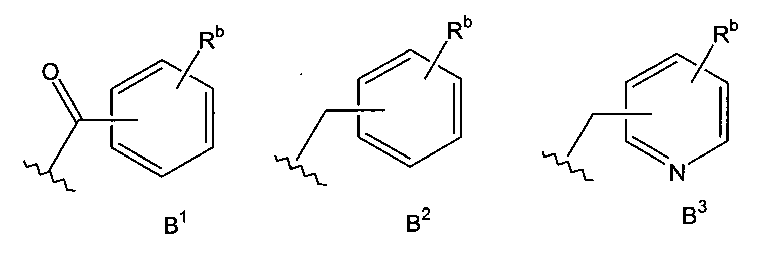 wherein
R^{b} respectively stands for a residue which is selected from the group comprising H, F, Cl, Br, I, -CN; -CF₃, -OCF₃, -SCF₃, -SF₅, -OH, -SH, methyl; ethyl; n-propyl; isopropyl; n-butyl; isobutyl; sec-butyl; tert-butyl; -OCH₃; -OC₂H₅; -S-CH₃ and -S-C₂H₅.

7. Compounds according to one or more of claims 1-6 selected from the group comprising
[1] Benzyl-[6-(3-chloro-phenyl)-benzo[d]isoxazol-3-yl]-amine,
[2] Benzyl-[6-(4-chloro-phenyl)-benzo[d]isoxazol-3-yl]-amine,
[3] Benzyl-(6-p-tolyl-benzo[d]isoxazol-3-yl)-amine,
[4] Benzyl-[6-(2-methoxy-phenyl)-benzo[d]isoxazol-3-yl]-amine,
[5] N-[6-(3-chloro-phenyl)-benzo[d]isoxazol-3-yl]-benzamide,
[6] N-[6-(4-chloro-phenyl)-benzo[d]isoxazol-3-yl]-benzamide,
[7] N-(6-p-tolyl-benzo[d]isoxazol-3-yl)-benzamide,
[8] N-[6-(2-methoxy-phenyl)-benzo[d]isoxazol-3-yl]-benzamide,
[9] (4-tert-butyl-benzyl)-(6-pyridine-2-yl-benzo[d]isoxazol-3-yl)-amine,
[10] (4-tert-butyl-benzyl)-[6-(3-methyl-pyridine-2-yl)-benzo[d]isoxazol-3-yl]-amine,
[11] (4-tert-butyl-benzyl)-[6-(6-methyl-pyridine-2-yl)-benzo[d]isoxazol-3-yl]- amine,
[12] (4-tert-butyl-benzyl)-(6-phenyl-benzo[d]isoxazol-3-yl)-amine,
[13] [6-(4-chloro-phenyl)-benzo[d]isoxazol-3-yl]-pyridine-2-ylmethyl-amine,
[14] (6-phenyl-benzo[d]isoxazol-3-yl)-pyridine-2-ylmethyl-amine,
[15] Pyridine-2-ylmethyl-(6-o-tolyl-benzo[d]isoxazol-3-yl)-amine,
[16] [6-(4-chloro-phenyl)-benzo[d]isoxazol-3-yl]-pyridine-3-ylmethyl-amine,
[17] (6-phenyl-benzo[d]isoxazol-3-yl)-pyridine-3-ylmethyl-amine,
[18] Pyridine-3-ylmethyl-(6-o-tolyl-benzo[d]isoxazol-3-yl)-amine,
[19] 2-{3-[(pyridine-3-ylmethyl)-amino]-benzo[d]isoxazol-6-yl}-phenol,
[20] [6-(4-chloro-phenyl)-benzo[d]isoxazol-3-yl]-pyridine-4-ylmethyl-amine,
[21] (6-phenyl-benzo[d]isoxazol-3-yl)-pyridine-4-ylmethyl-amine,
[22] Pyridine-4-ylmethyl-(6-o-tolyl-benzo[d]isoxazol-3-yl)-amine,
[23] 4-{[6-(4-chloro-phenyl)-benzo[d]isoxazol-3-ylamino]-methyl}-benzonitrile,
[24] 4-[(6-phenyl-benzo[d]isoxazol-3-ylamino)-methyl]-benzonitrile,
[25] 4-[(6-o-tolyl-benzo[d]isoxazol-3-ylamino)-methyl]-benzonitrile,
[26] 4-{[6-(2-hydroxy-phenyl)-benzo[d]isoxazol-3-ylamino]-methyl}-benzonitrile,
[27] [6-(4-chloro-phenyl)-benzo[d]isoxazol-3-yl]-(3-methyl-benzyl)-amine,
[28] (3-methyl-benzyl)-(6-phenyl-benzo[d]isoxazol-3-yl)-amine,
[29] (3-methyl-benzyl)-(6-o-tolyl-benzo[d]isoxazol-3-yl)-amine,
[30] 2-[3-(3-methyl-benzylamino)-benzo[d]isoxazol-6-yl]-phenol,
[31] (3-chloro-benzyl)-[6-(4-chloro-phenyl)-benzo[d]isoxazol-3-yl]-amine,
[32] (3-chloro-benzyl)-(6-phenyl-benzo[d]isoxazol-3-yl)-amine,
[33] (3-chloro-benzyl)-(6-o-tolyl-benzo[d]isoxazol-3-yl)-amine,
[34] 2-[3-(3-chloro-benzylamino)-benzo[d]isoxazol-6-yl]-phenol,
[35] 2-(3-(neopentylamino)benzo[d]isoxazol-5-yl)benzoic acid,
[36] Ethyl 4-(3-(neopentylamino)benzo[d]isoxazol-5-yl)benzoate,
[37] N-neopentyl-5-(3-(trifluoromethyl)phenyl)benzo[d]isoxazol-3-amine,
[38] 3-(3-(neopentylamino)benzo[d]isoxazol-5-yl)benzonitrile,
[39] N-methyl-3-(3-(neopentylamino)benzo[d]isoxazol-5-yl)benzamide,
[40] 3-(3-(neopentylamino)benzo[d]isoxazol-5-yl)benzoic acid,
[41] Ethyl 3-(3-(neopentylamino)benzo[d]isoxazol-5-yl)benzoate,
[42] N-cyclopropyl-N-(2-methoxy-5-(3-(neopentylamino)benzo[d]isoxazol-5-yl)benzyl)methanesulphonamide,
[43] N-cyclopropyl-N-(2-methoxy-5-(3-(neopentylamino)benzo[d]isoxazol-5-yl)benzyl)acetamide,
[44] N-cyclopropyl-N-(2-fluoro-5-(3-(neopentylamino)benzo[d]isoxazol-5-yl)benzyl)acetamide,
[45] 5-(3-((cyclopropylamino)methyl)-4-methoxyphenyl)-N-neopentylbenzo[d]isoxazol-3-amine,
[46] 5-(3-((cyclopropylamino)methyl)-4-fluorophenyl)-N-neopentylbenzo[d]isoxazol-3-amine,
[47] 2-methoxy-5-(3-(neopentylamino)benzo[d]isoxazol-5-yl)benzaldehyde,
[48] 2-fluoro-5-(3-(neopentylamino)benzo[d]isoxazol-5-yl)benzaldehyde,
[49] N-cyclopropyl-N-(3-(3-(neopentylamino)benzo[d]isoxazol-5-yl)benzyl)benzamide,
[50] N-cyclopropyl-N-(3-(3-(neopentylamino)benzo[d]isoxazol-5-yl)benzyl)acetamide,
[51] 5-(3-((cyclopropylamino)methyl)phenyl)-N-neopentylbenzo[d]isoxazol-3-amine and
[52] 3-(3-(neopentylamino)benzo[d]isoxazol-5-yl)benzaldehyde;
and respectively their corresponding salts, in particular their hydrochloride addition salts, and respectively, where applicable, their corresponding solvates.

8. Compounds according to one or more of claims 1-7, **characterised in that** in the FLIPR assay in a concentration of 10 µM they exhibit an inhibition of the Ca²⁺ ion influx in dorsal root ganglia of rats of at least 10%, preferably of at least 30%, particularly preferred of at least 50%, most particularly preferred of at least 70%, still further preferred of at least 90%, in comparison to the maximum achievable inhibition of the Ca²⁺ ion influx with capsaicin in a concentration of 10 µM.

9. Method for producing compounds according to one or more of claims 1-8, **characterised in that** a possibly substituted 2-fluoro-benzonitrile compound of the general formula II, 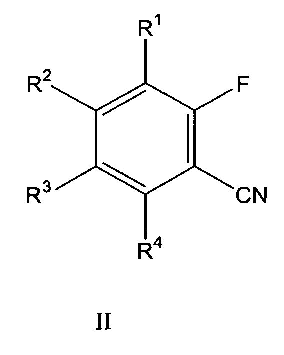 wherein residues R¹, R², R³ and R⁴ have the meaning according to one or more of claims 1-8, is transformed in a reaction medium in the presence of a base with acetohydroxamic acid of the formula III 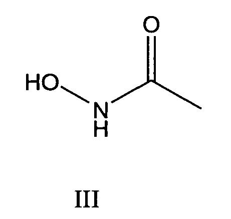 into a compound of the general formula I, 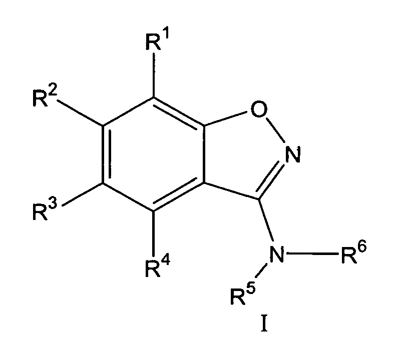 wherein residues R¹-R⁴ have the meaning indicated above and residues R⁵ and R⁶ respectively stand for a hydrogen residue, the latter compound is purified where applicable and/or isolated where applicable,
and the latter compound is subsequently transformed, where applicable, in a reaction medium with at least one isocyanate of the general formula R¹⁹-N=C=O, wherein R¹⁹ has the meaning according to one or more of claims 1 to 8, where applicable, in the presence of a base, preferably in the presence of at least one base selected from the group comprising triethylamine, 4,4-dimethylaminopyridine and diisopropylethylamine, into at least one compound of the general formula I, wherein R¹, R², R³, R⁴ and R⁵ have the meaning indicated above and R⁶ stands for -C(=O)-NR¹⁹R²⁰, wherein R¹⁹ has the meaning indicated above and R²⁰ stands for a hydrogen residue, and the latter compound is purified where applicable and/or isolated where applicable
or
and the latter compound is, where applicable, subsequently transformed in a reaction medium with at least one isothiocyanate of the general formula S=C=N-R²¹, wherein R²¹ has the meaning according to one or more of claims 1 to 8, where applicable, in the presence of at least one base, preferably in the presence of at least one base selected from the group comprising triethylamine, 4,4-dimethylaminopyridine and diisopropylethylamine, into at least one compound of the general formula I, wherein R¹, R², R³, R⁴ and R⁵ have the meaning indicated above and R⁶ stands for -C(=S)-NR²¹R²², wherein R²¹ has the meaning indicated above and R²² stands for a hydrogen residue, and the latter compound is purified where applicable and/or isolated where applicable,
and, where applicable, at least one compound of the general formula I, wherein R¹, R², R³, R⁴ and R⁵ have the meaning indicated above and R⁶ stands for -C(=O)-NR¹⁹R²⁰ or for -C(=S)-NR²¹R²², wherein R¹⁹ and R²¹ have the meaning indicated above and R²⁰ and R²² respectively stand for a hydrogen residue, is transformed in a reaction medium, in the presence of at least one base, preferably in the presence of at least one metal hydride salt or a metal alcoholate salt, particularly preferred in the presence of a metal hydride salt or a metal alcoholate salt selected from the group comprising sodium hydride, potassium hydride, potassium t-butoxide, sodium t-butoxide, potassium methoxide, sodium methoxide, sodium methoxide and potassium methoxide, with at least one compound of the general formula LG-R²⁰ or the general formula LG-R²², wherein LG stands for a leaving group, preferably for a halogen atom, particularly preferred for a chlorine atom, and R²² and R²⁴ respectively have the meaning according to one or more of claims 1-8 with the exception of hydrogen, into at least one compound of the general formula I, wherein R¹ to R⁶ have the meaning indicated above, and the latter compound is purified where applicable and/or isolated where applicable,
or
where applicable, at least one compound of the general formula I, wherein R¹, R², R³, R⁴ have the meaning indicated above and R⁵ and R⁶ respectively stand for H, is transformed in a reaction medium, in the presence of at least one base, preferably in the presence of at least one metal hydride salt or a metal alcoholate salt, particularly preferred in the presence of a metal hydride salt or a metal alcoholate salt selected from the group comprising sodium hydride, potassium hydride, potassium t-butoxide, sodium t-butoxide, potassium methoxide, sodium methoxide, sodium methoxide and potassium methoxide, with at least one compound of the general formula LG-R⁵ or the general formula LG-R⁶, wherein LG stands for a leaving group, preferably for a halogen atom, particularly preferably for a chlorine atom, and R⁵ and R⁶ respectively have the meaning according to one or more of claims 1-8 with the exception of hydrogen, into at least one compound of the general formula I, wherein R¹ to R⁶ have the meaning indicated above, and the latter compound is purified where applicable and/or isolated where applicable,
or
at least one compound of the general formula I, wherein residues R¹-R⁴ have the meaning indicated above and residues R⁵ and R⁶ respectively stand for H, is transformed in a reaction medium, in the presence of at least one acid and at least one reducing agent, preferably in the presence of trifluoroacetic acid and triethylsilane, with at least one compound of the general formula R⁵-(C=O)-H, wherein R⁵ has the meaning according to one or more of claims 1 to 8 with the exception of H, -C(=O)R¹⁸, -C(=O)-NR¹⁹R²⁰, -C(=S)-NR²¹R²² and -S(=O)₂R²³, into at least one compound of the general formula I, wherein R¹ to R⁵ have the meaning indicated above and R⁶ stands for H, and the latter compound is purified where applicable and/or isolated where applicable
or
at least one compound of the general formula I, wherein residues R¹-R⁴ have the meaning indicated above and residues R⁵ and R⁶ respectively stand for H, is transformed in a reaction medium with at least one compound of the general formula R¹⁸-C(=O)-LG, wherein R¹⁸ has the meaning according to one or more of claims 1 to 8 and LG stands for a leaving group, preferably for a halogen atom, particularly preferred for a chlorine atom, into at least one compound of the general formula I, wherein R¹ to R⁴ have the meaning indicated above, R⁵ stands for a -C(=O)R¹⁸ residue and R⁶ stands for a hydrogen residue; and the latter compound is purified where applicable and/or isolated where applicable,
or
at least one compound of the general formula I, wherein residues R¹-R⁴ have the meaning indicated above and residues R⁵ and R⁶ respectively stand for H, is transformed in a reaction medium in the presence of at least one coupling reagent, where applicable in the presence of at least one base with at least one compound of the general formula R¹⁸-C(=O)-OH, wherein R¹⁸ has the meaning according to one or more of claims 1 to 8, into at least one compound of the general formula I, wherein R¹ to R⁴ have the meaning indicated above, R⁵ stands for a -C(=O)R¹⁸ residue and R⁶ stands for a hydrogen residue; and the latter compound is purified where applicable and/or isolated where applicable,
or
at least one compound of the general formula I, wherein residues R¹-R⁴ have the meaning indicated above and residues R⁵ and R⁶ respectively stand for H, is transformed in a reaction medium, where applicable in the presence of at least one base, with at least one compound of the general formula R²³-S(=O)₂-LG, wherein R²³ has the meaning according to one or more of claims 1 to 8 and LG stands for a leaving group, preferably for a halogen atom, particularly preferred for a chlorine atom, into at least one compound of the general formula I, wherein R¹ to R⁴ have the meaning indicated above, R⁵ stands for a -S(=O)₂-R²³ group and R⁶ for a hydrogen residue, and the latter compound is purified where applicable and/or is isolated where applicable.

10. Method for producing compounds according to one or more of claims 1-8, **characterised in that** a possibly substituted 2-fluoro-benzonitrile compound of the general formula II, 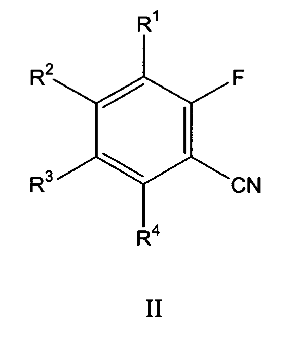 wherein at least one of residues R¹, R², R³ and R⁴ stands for a leaving group, preferably for a halogen residue or a sulphonic acid ester, particularly preferred for a leaving group selected from the group comprising chlorine, bromine, iodine, triflate, mesylate and tosylate, most particularly preferred for a bromine atom, and the remaining residues have the meaning according to one or more of claims 1-8 and do not stand for a possibly substituted aryl or heteroaryl residue, is transformed in a reaction medium in the presence of a base with acetohydroxamic acid of formula III 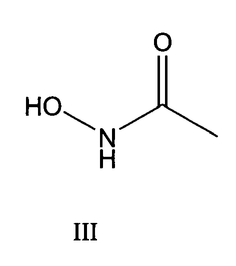 into a compound of the general formula I, 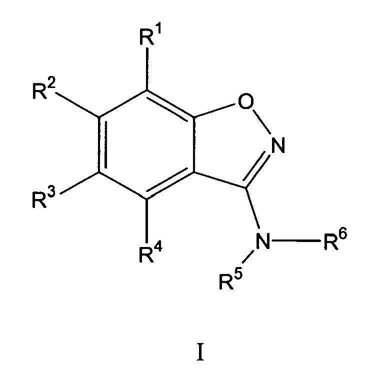 wherein residues R¹-R⁴ have the meaning indicated above and residues R⁵ and R⁶ respectively stand for a hydrogen residue, the latter compound is purified where applicable and/or is isolated where applicable,
and the latter compound, where applicable, is subsequently transformed in a reaction medium with at least one isocyanate of the general formula R¹⁹-N=C=O, wherein R¹⁹ has the meaning according to one or more of claims 1 to 8, where applicable in the presence of at least one base, preferably in the presence of at least one base selected from the group comprising triethylamine, 4,4-dimethylaminopyridine and diisopropylethylamine, into at least one compound of the general formula I, wherein R¹, R², R³, R⁴ and R⁵ have the meaning indicated above and R⁶ stands for -C(=O)-NR¹⁹R²⁰, wherein R¹⁹ has the meaning indicated above and R²⁰ stands for a hydrogen residue, and the latter compound is purified where applicable and/or isolated where applicable
or
the latter compound, where applicable, is subsequently transformed in a reaction medium with at least one isothiocyanate of the general formula S=C=N-R²¹, wherein R²¹ has the meaning according to one or more of claims 1 to 8, where applicable in the presence of at least one base, preferably in the presence of at least one base selected from the group comprising triethylamine, 4,4-dimethylaminopyridine and diisopropylethylamine, into at least one compound of the general formula I, wherein R¹, R², R³, R⁴ and R⁵ have the meaning indicated above and R⁶ stands for -C(=S)-NR²¹R²², wherein R²¹ has the meaning indicated above and R²² stands for a hydrogen residue, and the latter compound is purified where applicable and/or isolated where applicable,
and where applicable at least one compound of the general formula I, wherein R¹, R², R³, R⁴ and R⁵ have the meaning indicated above and R⁶ stands for -C(=O)-NR¹⁹R²⁰ or for -C(=S)-NR²¹R²², wherein R¹⁹ and R²¹ have the meaning indicated above and R²⁰ and R²² respectively stand for a hydrogen residue, is transformed in a reaction medium, in the presence of at least one base, preferably in the presence of at least one metal hydride salt or a metal alcoholate salt, particularly preferred in the presence of a metal hydride salt or a metal alcoholate salt selected from the group comprising sodium hydride, potassium hydride, potassium t-butoxide, sodium t-butoxide, potassium methoxide, sodium methoxide, sodium methoxide and potassium methoxide, with at least one compound of the general formula LG-R²⁰ or the general formula LG-R²², wherein LG stands for a leaving group, preferably for a halogen atom, particularly preferred for a chlorine atom, and R²² and R²⁴ respectively have the meaning according to one or more of claims 1-8 with the exception of hydrogen, into at least one compound of the general formula I, wherein R¹ to R⁶ have the meaning indicated above, and the latter compound is purified where applicable and/or isolated where applicable,
or
where applicable at least one compound of the general formula I, wherein R¹, R², R³, R⁴ have the meaning indicated above and R⁵ and R⁶ respectively stand for H, is transformed in a reaction medium, in the presence of at least one base, preferably in the presence of at least one metal hydride salt or a metal alcoholate salt, particularly preferred in the presence of a metal hydride salt or a metal alcoholate salt selected from the group comprising sodium hydride, potassium hydride, potassium t-butoxide, sodium t-butoxide, potassium methoxide, sodium methoxide, sodium methoxide and potassium methoxide, with at least one compound of the general formula LG-R⁵ or the general formula LG-R⁶, wherein LG stands for a leaving group, preferably for a halogen atom, particularly preferred for a chlorine atom, and R⁵ and R⁶ respectively have the meaning according to one or more of claims 1-8 with the exception of hydrogen, into at least one compound of the general formula I, wherein R¹ to R⁶ have the meaning indicated above, and the latter compound is purified where applicable and/or isolated where applicable,
or
at least one compound of the general formula I, wherein residues R¹-R⁴ have the meaning indicated above and residues R⁵ and R⁶ respectively stand for H, is transformed in a reaction medium, in the presence of at least one acid and at least one reducing agent, preferably in the presence of trifluoroacetic acid and triethylsilane, with at least one compound of the general formula R⁵-(C=O)-H, wherein R⁵ has the meaning according to one or more of claims 1 to 8 with the exception of H, -C(=O)R¹⁸, -C(=O)-NR¹⁹R²⁰, -C(=S)-NR²¹R²² and -S(=O)₂R²³, into at least compound of the general formula I, wherein R¹ to R⁵ have the meaning indicated above and R⁶ stands for H, and the latter compound is purified where applicable and/or isolated where applicable
or
at least one compound of the general formula I, wherein residues R¹-R⁴ have the meaning indicated above and residues R⁵ and R⁶ respectively stand for H, is transformed in a reaction medium with at least one compound of the general formula R¹⁸-C(=O)-LG, wherein R¹⁸ has the meaning according to one or more of claims 1 to 8 and LG stands for a leaving group, preferably for a halogen atom, particularly preferably for a chlorine atom, into at least compound of the general formula I, wherein R¹ to R⁴ have the meaning indicated above, R⁵ stands for a -C(=O)R¹⁸ residue and R⁶ stands for a hydrogen residue; and the latter compound is purified where applicable and/or isolated where applicable,
or
at least one compound of the general formula I, wherein residues R¹-R⁴ have the meaning indicated above and residues R⁵ and R⁶ respectively stand for H, is transformed in a reaction medium in the presence of at least one coupling reagent, where applicable in the presence of at least one base with at least one compound of the general formula R¹⁸-C(=O)-OH, wherein R¹⁸ has the meaning according to one or more of claims 1 to 8, into at least one compound of the general formula I, wherein R¹ to R⁴ have the meaning indicated above, R⁵ stands for a -C(=O)R¹⁸ residue and R⁶ stands for a hydrogen residue; and the latter compound is purified where applicable and/or isolated where applicable,
or
at least one compound of the general formula I, wherein residues R¹-R⁴ have the meaning indicated above and residues R⁵ and R⁶ respectively stand for H, is transformed in a reaction medium, where applicable in the presence of at least one base, with at least one compound of the general formula R²³-S(=O)₂-LG, wherein R²³ has the meaning according to one or more of claims 1 to 8 and LG stands for a leaving group, preferably for a halogen atom, particularly preferred for a chlorine atom, into at least one compound of the general formula I, wherein R¹ to R⁴ have the meaning indicated above, R⁵ stands for a -S(=O)₂-R²³ group and R⁶ stands for a hydrogen residue, and the latter compound is purified where applicable and/or isolated where applicable,
and respectively the compound obtained in this manner of the general formula I, in which at least one of residues R¹ to R⁴ stands for a leaving group, preferably for a halogen residue or a sulphonic acid ester, particularly preferred for a leaving group selected from the group comprising chlorine, bromine, iodine, triflate, mesylate and tosylate, most particularly preferred for a bromine atom, and the remaining residues have the meaning indicated above, where applicable in a reaction medium, where applicable in the presence of at least one base and one catalyst which can be polymer-bonded, preferably in the presence of at least one base selected from the group comprising potassium carbonate, sodium carbonate, potassium phosphate, sodium hydrogen phosphate, caesium carbonate, triethylamine, [1,4]-diazabicyclo-[2.2.2]-octane, diisopropylamine, diisopropylethylamine and N-methylmorpholine and a catalyst which can be polymer-bonded selected from the group comprising palladium(II)acetate, tris(dibenzylideneacetone)dipalladium, palladium(0)bis(dibenzylideneacetone), tetrakis(triphenylphosphine)palladium(0), (1, 1 '-bis(diphenylphosphino)-ferrocene)dichloropalladium(II), bis(acetonitrile)dichloropalladium(II), palladium(II)chloride, dichlorobis(triphenylphosphine)palladium(II), dichloro(tricyclohexylphosphine)palladium(II), bis(acetato)bis(triphenylphosphine)-palladium(II), bistriphenyl-phosphinepalladium(II)dichloride, bistriphenyl-phosphinepalladium(II)acetate and iron(III)chloride, particularly preferred in the presence of an alkali carbonate and a palladium catalyst, most particularly preferred in the presence of sodium carbonate and tetrakis(triphenylphosphine)-palladium (0), where applicable in the presence of at least one ligand which can be polymer-bonded, preferably in the presence of at least one ligand which can be polymer-bonded selected from the group comprising 2-dicyclohexylphosphino-2',6'-dimethoxybiphenyl (S-Phos), 2-dicyclohexylphosphino-2',4',6'-triisopropylbiphenyl (X-Phos), tricyclohexylphosphine, tricyclohexylphosphine tetrafluoroborate, tri-tert-butylphosphine tetrafluoroborate, triphenylphosphine and imidazolium salts, where applicable using microwave radiation, transforms with at least one boric acid compound of the following formula, 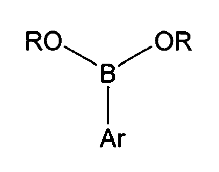 wherein Ar stands for an unsubstituted or at least mono-substituted aryl or heteroaryl residue according to one or more of claims 1-8 and R for hydrogen or an organic residue, preferably for hydrogen or an alkyl residue, or two residues R together with the -O-B-O- group which connects them form a heterocycloalkyl residue, and purifies
where applicable and/or isolates where applicable the compound obtained in this manner of the general formula I, in which at least one of residues R¹ to R⁴ stands for an unsubstituted or at least mono-substituted aryl or heteroaryl residue,
or respectively the compound obtained in this manner of the general formula I, in which at least one of residues R¹ to R⁴ stands for a leaving group, preferably for a halogen residue or a sulphonic acid ester, particularly preferred for a leaving group selected from the group comprising chlorine, bromine, iodine, triflate, mesylate and tosylate, most particularly preferred for a bromine atom, and the remaining residues have the meaning indicated above, transforms with an organometallic compound of the general formula R'-M-X, wherein R' stands for an unsubstituted or at least mono-substituted aryl or heteroaryl residue according to one or more of claims 1-8, M for a transition metal ion, preferably for a magnesium or zinc ion, and X for a leaving group, preferably for a halogen residue or a sulphonic acid ester, particularly preferred for a leaving group selected from the group comprising chlorine, bromine, iodine, triflate, mesylate and tosylate, most particularly preferred for a bromine atom, where applicable in at least one reaction medium, where applicable in the presence of at least one catalyst which can be polymer-bonded, preferably in the presence of a catalyst which can be polymer-bonded, selected from the group comprising palladium(II)acetate, tris(dibenzylideneacetone)dipalladium, palladium(0)bis-(dibenzylideneacetone), tetrakis(triphenylphosphine)palladium(0), (1,1'-bis(diphenylphosphino)-ferrocene)dichloropalladium(II), bis(acetonitrile)-dichloropalladium(II), palladium(II)chloride, dichlorobis(triphenylphosphine)palladium(II), dichloro(tricyclohexylphosphine)palladium(II), bis(acetato)bis(triphenylphosphine)-palladium(II), bistriphenyl-phosphinepalladium(II)dichloride, bistriphenyl-phosphinepalladium(II)acetate and iron(III)chloride, particularly preferably in the presence of tetrakis(triphenylphosphine)-palladium(0), and purifies where applicable and/or isolates where applicable the compound obtained in this manner of the general formula I, in which at least one of residues R¹ to R⁴ stands for an unsubstituted or at least mono-substituted aryl or heteroaryl residue.

11. Medicament containing at least one compound according to one or more of claims 1 to 8 and where applicable one or more physiologically compatible adjuvants.

12. Medicament according to claim 11 for the treatment and/or prophylaxis of one of more illnesses selected from the group comprising pain, preferably pain selected from the group comprising acute pain, chronic pain, neuropathic pain and visceral pain; joint pain; migraines; depression; nervous complaints; nerve injuries; neurodegenerative illnesses, preferably selected from the group comprising multiple sclerosis, Alzheimer's disease, Parkinson's disease and Huntington's disease; cognitive dysfunctions, preferably cognitive deficiencies, particularly preferred memory disorders; epilepsy; airway illnesses, preferably selected from the group comprising asthma and lung inflammation; coughs; urinary incontinence; overactive bladder (OAB); stomach ulcers; irritable bowel syndrome; strokes; eye irritations; skin irritations; neurotic skin complaints; inflammatory illnesses, preferably inflammation of the bowel; diarrhoea; pruritus; eating disorders, preferably selected from the group comprising bulimia, cachexia, anorexia and obesity; medicament dependency; medicament abuse; withdrawal symptoms in the case of medicament dependency; development of tolerance to medicaments, preferably to natural or synthetic opioids; drug dependency; drug abuse; withdrawal symptoms in the case of drug dependency; alcohol dependency; alcohol abuse and withdrawal symptoms in the case of alcohol dependency; for diuresis; for antinatriuresis; to influence the cardiovascular system; to improve vigilance; to increase libido; to modulate movement activity; for anxiolysis; for local anaesthesia and/or to inhibit undesirable side effects, preferably selected from the group comprising hyperthermia, high blood pressure and narrowing of the bronchia, triggered by the administration of vanilloid receptor 1 (VR1/TRPV1 receptor) agonists, preferably selected from the group comprising capsaicin, resiniferatoxin, olvanil, arvanil, SDZ-249665, SDZ-249482, nuvanil and capsavanil.

13. Use of at least one compound according to one or more of claims 1 to 8 to produce a medicament for the treatment and/or prophylaxis of one of more illnesses selected from the group comprising pain, preferably pain selected from the group comprising acute pain, chronic pain, neuropathic pain and visceral pain; joint pain; migraines; depression; nervous complaints; nerve injuries; neurodegenerative illnesses, preferably selected from the group comprising multiple sclerosis, Alzheimer's disease, Parkinson's disease and Huntington's disease; cognitive dysfunctions, preferably cognitive deficiencies, particularly preferred memory disorders; epilepsy; airway illnesses, preferably selected from the group comprising asthma and lung inflammation; coughs; urinary incontinence; overactive bladder; stomach ulcers; irritable bowel syndrome; strokes; eye irritations; skin irritations; neurotic skin complaints; inflammatory illnesses, preferably inflammation of the bowel; diarrhoea; pruritus; eating disorders, preferably selected from the group comprising bulimia, cachexia, anorexia and obesity; medicament dependency; medicament abuse; withdrawal symptoms in the case of medicament dependency; development of tolerance to medicaments, preferably to natural or synthetic opioids; drug dependency; drug abuse; withdrawal symptoms in the case of drug dependency; alcohol dependency; alcohol abuse and withdrawal symptoms in the case of alcohol dependency; for diuresis; for antinatriuresis; to influence the cardiovascular system; to improve vigilance; to increase libido; to modulate movement activity; for anxiolysis; for local anaesthesia and/or to inhibit undesirable side effects, preferably selected from the group comprising hyperthermia, high blood pressure and narrowing of the bronchia, triggered by the administration of vanilloid receptor 1 (VR1/TRPV1 receptor) agonists, preferably selected from the group comprising capsaicin, resiniferatoxin, olvanil, arvanil, SDZ-249665, SDZ-249482, nuvanil and capsavanil (DA-5018).

14. Use according to claim 13 for the production of a medicament for the treatment and/or prophylaxis of pain selected from the group comprising acute pain, chronic pain, neuropathic pain and visceral pain.

## Revendications

1. Composés benzo[d]isoxazol-3-yl-amines substituées de formule générale I 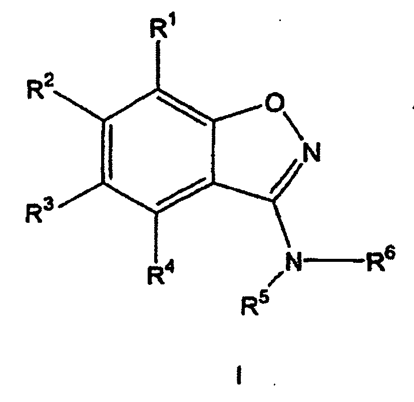 dans laquelle
R¹, R², R³ et R⁴ représentent chacun indépendamment des autres H; F, Cl, Br, I; -CN; -NC; -NO₂; -SF₅; -NR⁷R⁸; -OR⁹; -SR¹⁰; -C(=O) OR¹¹; -(C=O)NR¹²R¹³; -S(=O)₂R¹⁴; -C(=O)R¹⁵; -NR¹⁶-S(=O)₂R¹⁷;
un radical aliphatique à chaîne droite ou ramifiée, saturé ou insaturé, non substitué ou au moins monosubstitué ;
un radical cycloaliphatique saturé ou insaturé, non substitué ou au moins monosubstitué, comportant éventuellement au moins un hétéroatome en tant que chaînon, radical qui peut être condensé à un système cyclique monocyclique ou polycyclique et/ou être lié par l'intermédiaire d'un groupe alkylène, alcénylène ou alcynylène à chaîne droite ou ramifiée ;
ou représente un radical aryle ou hétéroaryle non substitué ou substitué, qui peut être condensé à un système cyclique monocyclique ou polycyclique et/ou peut être lié par l'intermédiaire d'un groupe alkylène, alcénylène ou alcynylène à chaîne droite ou ramifiée, au moins l'un des radicaux R¹, R², R³ et R⁴ représentant un radical aryle ou hétéroaryle non substitué ou substitué,
et
où les radicaux aryle ou hétéroaryle substitués mentionnés ci-dessus sont éventuellement substitués chacun par 1, 2, 3, 4 ou 5 substituants choisis indépendamment les uns des autres dans le groupe consistant en F, Cl, Br, I, -CN, -CF₃, -SF₅, -OH, les radicaux -O-(alkyle en C₁₋₅), -NH₂, -NO₂, -O-CF₃, -S-CF₃, -SH, -S-(alkyle en C₁₋₅), -C-(alkyle en C₁₋₅), -C(=O)-OH, -C(=O)-O-(alkyle en C₁₋₅), -NH-(alkyle en C₁₋₅), -N(alkyle en C₁₋₅)(alkyle en C₁₋₅), -NH-C(=O)-O-(alkyle en C₁₋₅), -C(=O)-H, -C(=O)-(alkyle en C₁₋₅), -C(=O)-N(alkyle en C₁₋₅)(alkyle en C₁₋₅), -S(=O)₂NH₂; -S(=O)₂N(H) (alkyle en C₁₋₅); -S(=O)2N (alkyle en C₁₋₅)(alkyle en C₁₋₅) ; -S(=O)₂phényle; -S(=O)₂-(alkyle en C₁₋₅); -C(=O)-NH-CH₃, -C(=O)-NH-C₂H₅, -C(=O)-N(cyclopropyl)-(alkyle en C₁₋₅), -N(cyclopropyl) -S(=O)2-(alkyle en C₁₋₅), -CH₂-NH-S(=O)₂-(alkyle en C₁₋₅), -CH₂-N(cyclopropyl)-S(=O)₂-(alkyle en C₁₋₅), -CH₂-N(cyclopropyl)-C(=O)- (alkyle en C₁₋₅), -CH₂-N(cyclopropyl)-C(=O)-phényle, -O-phényle, -O-benzyle, phényle, benzyle, thiophényle (thiényle), furannyle et pyridinyle, la partie cyclique de chacun des radicaux -CH₂-N(cyclopropyl)-C(=O)-phényle, -O-phényle, -O-benzyle, phényle, -S(=O)₂phényle, benzyle, thiophényle (thiényle), furannyle et pyridinyle pouvant être substituée éventuellement par 1, 2, 3, 4 ou 5 substituants choisis indépendamment les uns des autres dans le groupe consistant en F, Cl, Br, -OH, -CF₃, -SF₅, -CN, -NO₂, les radicaux -(alkyle en C₁₋₅), -O-(alkyle en C₁₋₅), -O-CF₃, -S-CF₃, phényle et -O-benzyle,
R⁵ et R⁶ représentent chacun indépendamment des autres H; -C(=O)R¹⁸; -C(=O)NR¹⁹R²⁰; -C(=S)NR²¹R²²; -S(=O)₂R²³;
un radical aliphatique à chaîne droite ou ramifiée, saturé ou insaturé, non substitué ou au moins monosubstitué ;
un radical cycloaliphatique saturé ou insaturé, non substitué ou au moins monosubstitué, comportant éventuellement au moins un hétéroatome en tant que chaînon, radical qui peut être condensé à un système cyclique monocyclique ou polycyclique et/ou peut être lié par l'intermédiaire d'un groupe alkylène à chaîne droite ou ramifiée ;
ou un radical aryle ou hétéroaryle non substitué ou au moins monosubstitué, qui peut être condensé à un système cyclique monocyclique ou polycyclique, et/ou peut être lié par l'intermédiaire d'un groupe alkylène à chaîne droite ou ramifiée,
où chaque radical aryle mentionné ci-dessus est éventuellement substitué par 1, 2, 3, 4 ou 5 substituants choisis indépendamment les uns des autres dans le groupe consistant en F, Cl, Br, I, -CN, -CF₃, -SF₅, -OH, les radicaux -O-(alkyle en C₁₋₅), -O-CF₃, -S-CF₃, -SH, -S-(alkyle en C₁₋₅), -(alkyle en C₁₋₅), -C(=OH)-OH, -C(=O)-O-(alkyle en C₁₋₅), -NH- (alkyle en C₁₋₅), -N (alkyle en C₁₋₅)₂, -NH-C(=O)-O-(alkyle en C₁₋₅), -C(=O)-H, -C(=O)-(alkyle en C₁₋₅), -C(=O)-NH₂, -C(=O)NH-(alkyle en C₁₋₅), -C(=O)-N(alkyle en C₁₋₅)₂, -S(=O)₂HN₂; -S(=O)₂N(H) (alkyle en C₁₋₅); -S(=O)₂N(alkyle en C₁₋₅)(alkyle en C₁₋₅); -S(=O)₂phényle; -S(=O)₂-(alkyle en C₁₋₅); -CH₂-NH-cyclopropyle; -CH₂-N(alkyle en C₁₋₅)-cyclopropyle; -C(=O)-N(cyclopropyl)-(alkyle en C₁₋₅), -N(cyclopropyl) -S(=O)₂- (alkyle en C₁₋₅), -CH₂-NH-S(=O)₂-(alkyle en C₁₋₅), -CH₂-N(cyclopropyl)-S(=O)₂-(alkyle en C₁₋₅), -CH₂-N(cyclopropyl)-C(=O)-(alkyle en C₁₋₅), -CH₂-N(cyclopropyl)-C(=O)-phényle, -O-phényle, -O-benzyle, phényle, benzyle, thiophényle (thiényle), furannyle et pyridinyle, la partie cyclique de chacun des radicaux -CH₂-N(cycloprolyl)-C(=O)-phényle, -O-phényle, -O-benzyle, phényle, -S(=O)₂ phényle et benzyle pouvant être substituée par 1, 2, 3, 4 ou 5 substituants choisis indépendamment les uns des autres dans le groupe consistant en F, Cl, Br, -OH, -CF₃, -SF₅, -CN, -NO₂, les radicaux -(alkyle en C₁₋₅), -O-(alkyle en C₁₋₅), -O-CF₃, -S-CF₃, phényle et -O-benzyle,
à la condition que R⁵ et R⁶ ne représentent pas simultanément un résidu hydrogène ;
ou
R⁵ et R⁶, avec l'atome d'azote qui les relie servant de chaînon, forment un radical hétérocycloaliphatique saturé ou insaturé, non substitué ou au moins monosubstitué, comportant éventuellement au moins un hétéroatome supplémentaire en tant que chaînon,
R⁷ et R⁸ représentent chacun indépendamment de l'autre H, -C(=O)R¹⁵, ou un radical aliphatique à chaîne droite
ou ramifiée, saturé ou insaturé, non substitué ou au moins monosubstitué, ou
R⁷ et R⁸, avec l'atome d'azote qui les relie en tant que chaînon, forment un radical hétérocycloaliphatique saturé ou insaturé, non substitué ou au moins monosubstitué, comportant éventuellement au moins un hétéroatome supplémentaire en tant que chaînon,
R⁹, R¹⁰, R¹¹ et R¹⁶ représentent chacun indépendamment des autres
H ;
un radical aliphatique à chaîne droite ou ramifiée, saturé ou insaturé, non substitué ou au moins monosubstitué ;
un radical cycloaliphatique saturé ou insaturé, non substitué ou au moins monosubstitué, comportant éventuellement au moins un hétéroatome en tant que chaînon, radical qui peut être condensé à un système cyclique monocyclique ou polycyclique et/ou peut être lié par l'intermédiaire d'un groupe alkylène à chaîne droite ou ramifiée ;
ou un radical aryle ou hétéroaryle non substitué ou au moins monosubstitué, qui peut être condensé à un système cyclique monocyclique ou polycyclique et/ou peut être lié par l'intermédiaire d'un groupe alkylène à chaîne droite ou ramifiée ;
R¹² et R¹³ représentent chacun indépendamment de l'autre H ou un radical aliphatique à chaîne droite ou ramifiée, saturé ou insaturé, non substitué ou au moins monosubstitué, ou
R¹² et R¹³, avec l'atome d'azote qui les relie en tant que chaînon, forment un radical hétérocycloaliphatique saturé ou insaturé, non substitué ou au moins monosubstitué, comportant éventuellement au moins un hétéroatome supplémentaire en tant que chaînon,
R¹⁴ et R²³ représentent chacun indépendamment de l'autre -NR⁷R⁸ ;
un radical aliphatique à chaîne droite ou ramifiée, saturé ou insaturé, non substitué ou au moins monosubstitué,
un radical cycloaliphatique saturé ou insaturé, non substitué ou au moins monosubstitué, comportant éventuellement au moins un hétéroatome en tant que chaînon, radical qui peut être condensé à un système cyclique monocyclique ou polycyclique et/ou peut être lié par l'intermédiaire d'un groupe alkylène à chaîne droite ou ramifiée,
ou représente un radical aryle ou hétéroaryle non substitué ou au moins monosubstitué, qui peut être condensé à un système cyclique monocyclique ou polycyclique et/ou peut être lié par l'intermédiaire d'un groupe alkylène à chaîne droite ou ramifiée,
R¹⁵, R¹⁷ et R¹⁸ représentent chacun indépendamment des autres
un radical aliphatique à chaîne droite ou ramifiée, saturé ou insaturé, non substitué ou au moins monosubstitué,
un radical cycloaliphatique saturé ou insaturé, non substitué ou au moins monosubstitué, comportant éventuellement au moins un hétéroatome en tant que chaînon, radical qui peut être condensé à un système cyclique monocyclique ou polycyclique et/ou peut être lié par l'intermédiaire d'un groupe alkylène à chaîne droite ou ramifiée,
ou un radical aryle ou hétéroaryle non substitué ou au moins monosubstitué, qui peut être condensé à un système cyclique monocyclique ou polycyclique et/ou peut être lié par l'intermédiaire d'un groupe alkylène à chaîne droite ou ramifiée,
R¹⁹ et R²⁰ représentent chacun indépendamment de l'autre H ou un radical aliphatique à chaîne droite ou ramifiée, saturé ou insaturé, non substitué ou au moins monosubstitué, ou
R¹⁹ et R²⁰, avec l'atome d'azote qui les relie en tant que chaînon, forment un radical hétérocycloaliphatique saturé ou insaturé, non substitué ou au moins monosubstitué, comportant éventuellement au moins un hétéroatome supplémentaire en tant que chaînon,
R²¹ et R²² représentent chacun indépendamment de l'autre H ou un radical aliphatique à chaîne droite ou ramifiée, saturé ou insaturé, non substitué ou au moins monosubstitué, ou
R²¹ et R²², avec l'atome d'azote qui les relie en tant que chaînon, forment un radical hétérocycloaliphatique saturé ou insaturé, non substitué ou au moins monosubstitué, comportant éventuellement au moins un hétéroatome supplémentaire en tant que chaînon,
dans chaque cas éventuellement sous forme de l'un de leurs stéréoisomères purs, en particulier de leurs énantiomères ou diastéréoisomères, de leurs racémates,
ou sous forme d'un mélange de diastéréoisomères, éventuellement des énantiomères et/ou diastéréoisomères, selon un rapport de mélange quelconque, ou dans chaque cas sous forme des sels correspondants, ou dans chaque cas sous forme des produits de solvatation correspondants.

2. Composés selon la revendication 1, **caractérisés en ce que**
R¹, R², R³ et R⁴ représentent chacun indépendamment des autres
H; F; Cl; Br; I, -CN; -NC; -NO₂; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; un radical alkyle en C₁₋₁₀, alcényle en C₂₋₁₀, alcynyle en C₂₋₁₀; cycloalkyle en C₃₋₈; -(alkylène en C₁₋₅)-(cycloalkyle en C₃₋₈); hétérocycloalkyle; -(alkylène en C₁₋₅)-hétérocycloalkyle; aryle; hétéroaryle; -(alkylène en C₁₋₅)-aryle; -(alkylène en C₁₋₅)-hétéroaryle ;
au moins l'un d'un radicaux R¹, R², R³ et R⁴ étant un radical aryle ou hétéroaryle ;
R⁵ et R⁶ représentent chacun indépendamment de l'autre H; -C(=O)R¹⁸; -C(=O)NR¹⁹R²⁰; -C(=S)NR²¹R²²; -S(=O)₂R²³; un radical alkyle en C₁₋₁₀, alcényle en C₂₋₁₀, alcynyle en C₂₋₁₀, cycloalkyle en C₃₋₈; -(alkylène en C₁₋₅)-(cycloalkyle en C₃₋₈); hétérocycloalkyle; -(alkylène en C₁₋₅)-hétérocycloalkyle; aryle; hétéroaryle; -(alkylène en C₁₋₅)-aryle ou (alkylène en C₁₋₅)-hétéroaryle ;
à la condition que R⁵ et R⁶ ne représentent pas simultanément un résidu hydrogène ;
ou
R⁵ et R⁶, avec l'atome d'azote qui les relie en tant que chaînon, forment un radical qui est choisi dans le groupe H, consistant en 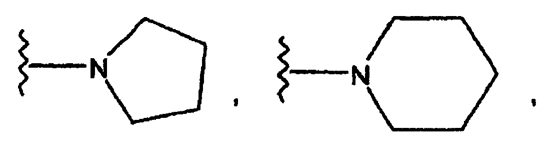 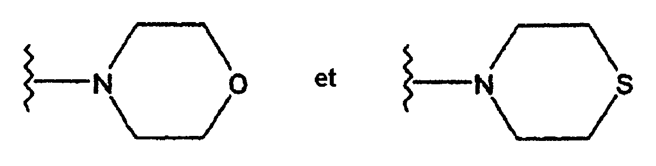 R⁷ et R⁸ représentent chacun indépendamment de l'autre H, -C(=O)R¹⁵ ou un radical alkyle en C₁₋₁₀, ou
R⁷ et R⁸, avec l'atome d'azote qui les relie en tant que chaînon, forment un radical qui est choisi dans le groupe H indiqué ci-dessus ;
R⁹, R¹⁰, R¹¹ et R¹⁶ représentent chacun indépendamment des autres H; un radical alkyle en C₁₋₁₀, alcényle en C₂₋₁₀, alcynle en C₂₋₁₀, cycloalkyle en C₃₋₈; -(alkylène en C₁₋₅)-(cycloalkyle en C₃₋₈); hétérocycloalkyle; -(alkylène en C₁₋₅)-hétérocycloalkyle; aryle; hétéroaryle; -(alkylène en C₁₋₅)-aryle ou -(alkylène en C₁₋₅)-hétéroaryle;
R¹² et R¹³ représentent chacun indépendamment de l'autre H ou un radical alkyle en C₁₋₁₀ ; ou
R¹⁴ et R²³, avec l'atome d'azote qui les relie en tant que chaînon, forment un radical qui est choisi dans le groupe H indiqué ci-dessus ; représentent chacun indépendamment de l'autre -NR⁷R⁸; un radical alkyle en C₁₋₁₀, alcényle en C₂₋₁₀, alcynyle en C₂₋₁₀, cycloalkyle en C₃₋₈; -(alkylène en C₁₋₅)-(cycloalkyle en C₃₋₈); hétérocycloalkyle; -(alkylène en C₁₋₅)-hétérocycloalkyle; aryle; hétéroaryle; -(alkylène en C₁₋₅)-aryle ou (alkylène en C₁₋₅)-hétéroaryle ;
R¹⁵, R¹⁷ et R¹⁸ représentent chacun indépendamment des autres un radical alkyle en C₁₋₁₀, alcényle en C₂₋₁₀, alcynyle en C₂₋₁₀, cycloalkyle en C₃₋₈, -(alkylène en C₁₋₅)-(cycloalkyle en C₃₋₈); hétérocycloalkyle; -(alkylène en C₁₋₅)-hétérocycloalkyle; aryle; hétéroaryle; -(alkylène en C₁₋₅)-aryle ou -(alkylène en C₁₋₅)-hétéroaryle ;
R¹⁹ et R²⁰ représentent chacun indépendamment de l'autre H ou un radical alkyle en C₁₋₁₀, ou
R¹⁹ et R²⁰, avec l'atome d'azote qui les relie en tant que chaînon, forment un radical qui est choisi dans le groupe H indiqué ci-dessus ;
R²¹ et R²² représentent chacun indépendamment de l'autre H ou un radical alkyle en C₁₋₁₀, ou
R²¹ et R²², avec l'atome d'azote qui les relie en tant que chaînon, forment un radical qui est choisi dans le groupe H indiqué ci-dessus ;
où
les radicaux alkyle en C₁₋₁₀, alcényle en C₂₋₁₀ et alcynyle en C₂₋₁₀ mentionnés ci-dessus ont chacun une chaîne droite ou ramifiée, et peuvent être éventuellement substitués par 1, 2, 3, 4 ou 5 substituants choisis indépendamment les uns des autres dans le groupe consistant en F; Cl; Br; -CN; -OH; -SH; un radical -O-(alkyle en C₁₋₂); S-(alkyle en C₁₋₂) et -NH₂,
les radicaux cycloalkyle en C₃₋₈ mentionnés ci-dessus peuvent être chacun substitués éventuellement par 1, 2, 3, 4 ou 5 substituants choisis indépendamment les uns des autres dans le groupe consistant en F; Cl; Br; -CN; -OH; -SH; les radicaux alkyle en C₁₋₅; -O-(alkyle en C₁₋₂); -S-(alkyle en C₁₋₂) et -NH₂,
les radicaux hétérocycloalkyle mentionnés ci-dessus sont chacun à 4, 5, 6 ou 7 chaînons, comportent chacun un ou deux hétéroatomes choisis indépendamment l'un de l'autre dans le groupe consistant en l'oxygène, le soufre et l'azote en tant que chaînon(s), et peuvent chacun être substitués éventuellement par 1, 2, 3, 4 ou 5 substituants choisis indépendamment les uns des autres dans le groupe consistant en F; Cl; Br; -CN; -OH; -SH; les radicaux alkyle en C₁₋₅; -O-(alkyle en C₁₋₂); -S-(alkyle en C₁₋₂) et -NH₂,
chacun des radicaux aryle mentionnés ci-dessus représente un radical phényle ou naphtyle, qui peut être substitué par 1, 2, 3, 4 ou 5 substituants choisis indépendamment les uns des autres dans le groupe consistant en F; Cl; Br; I; -CF₃; -OCF₃; -SCF₃; -SF₅; -CN; -OH; -SH; les radicaux -(alkyle en C₁₋₅); -O-(alkyle en C₁₋₅); -S-(alkyle en C₁₋₅); -C(=O)-NH-CH₃, -C(=O)-NH-C₂H₅, -C(=O)-OH; -C(=O)-O(alkyle en C₁₋₅); -N(H)(alkyle en C₁₋₅); -N (alkyle en C₁₋₅) (alkyle en C₁₋₅); -C(=O)-H; -C(=O)-(alkyle en C₁₋₅); -C(=O)N(alkyle en C₁₋₅) (alkyle en C₁₋₅); -S(=O)₂NH₂; -S(=O)₂N(H)alkyle en C₁₋₅); -S(=O)₂N(alkyle en C₁₋₅) (alkyle en C₁₋₅); -S(=O)₂-phényle; -S(=O)₂-(alkyle en C₁₋₅); -C(=O)-N(cyclopropyl) (alkyle en C₁₋₅), -N(cyclopropyl)-S(=O)2-(alkyle en C₁₋₅), -CH₂-NH-S(=O)₂-(alkyle en C₁₋₅), -CH₂-N(cyclopropyl)-S(=O)₂-(alkyle en C₁₋₅), -CH₂-N(cyclopropyl)-C(=O)-(alkyle en C₁₋₅), -CH₂-N(cyclopropyl)-C(=O)-phényle, phényle; phénoxy, benzyle; thiophényle (thiényle), furannyle et pyridinyle,
les radicaux hétéroaryle mentionnés ci-dessus ont chacun 5 ou 6 chaînons, comportent chacun 1, 2 ou 3 hétéroatomes choisis indépendamment les uns des autres dans le groupe consistant en l'oxygène, le soufre et l'azote en tant que chaînon(s) et peuvent chacun être substitués éventuellement par 1, 2, 3, 4 ou 5 substituants choisis indépendamment les uns des autres dans le groupe consistant en F; Cl; Br; I; -CF₃; -OCF₃; -SCF₃; -SF₅; -CN; -OH; -SH; les radicaux -(alkyle en C₁₋₅); -O-(alkyle en C₁₋₅); -S-(alkyle en C₁₋₅); -C(=O)-OH; -C(=O)-O(alkyle en C₁₋₅); -NH₂; -N (H) (alkyle en C₁₋₅); -N (alkyle en C₁₋₅) (alkyle en C₁₋₅); -C(=O)-NH-CH₃, -C(=O)-NH-C₂H₅, -C(=O)N(alkyle en C₁₋₅) (alkyle en C₁₋₅); -S(=O)₂NH₂; -S(=O)₂N(H) (alkyle en C₁₋₅); -S(=O)₂N (alkyle en C₁₋₅) (alkyle en C₁₋₅); -S(=O)₂-phényle; -S(=O)₂-(alkyle en C₁₋₅); -CH₂-NH-cyclopropyle; -CH₂-N (alkyle en C₁₋₅) cyclopropyle; -C(=O)-N(cyclopropyl)-(alkyle en C₁₋₅) -N(cyclopropyl)-S(=O)₂-(alkyle en C₁₋₅), -CH₂-NH-S(=O)₂-(alkyle en C₁₋₅), -CH₂-N(cyclopropyl)-S(=O)₂-(alkyle en C₁₋₅), -CH₂-N(cyclopropyl)-C(=O)-(alkyle en C₁₋₅), -CH₂-N(cyclopropyl)-C(=O)-phényle, phényle; phénoxy, benzyle; thiophényle (thiényle), furannyle et pyridinyle,
dans chaque cas éventuellement sous forme de l'un de leurs stéréoisomères purs, en particulier de leurs énantiomères ou diastéréoisomères, de leurs racémates, ou sous forme d'un mélange de diastéréoisomères, éventuellement des énantiomères et/ou diastéréoisomères, selon un rapport de mélange quelconque, ou dans chaque cas sous forme des sels correspondants, ou dans chaque cas sous forme des produits de solvatation correspondants.

3. Composés selon la revendication 1 ou 2, **caractérisés en ce que**
R¹, R², R³ et R⁴ représentent chacun indépendamment des autres
H; F; Cl; Br; I; -CN; -SF₅; -NR⁷R⁸; -OR⁹; -SR¹⁰; -C(=O)OR¹¹; -(C=O)NR¹²R¹³; -S(=O)₂R¹⁴; -C(=O)R¹⁵; -NR¹⁶-S(=O)₂R¹⁷; un radical alkyle en C₁₋₄, alcényle en C₁₋₉, alcynyle en C₂₋₄; cycloalkyle en C₃₋₈; -(alkylène en C_{1,2 ou 3})-(cycloalkyle en C₃₋₈); hétérocycloalkyle; -(alkylène en C_{1,2 ou 3})-hétérocycloalkyle; aryle; hétéroaryle; -(alkylène en C_{1,2 ou 3})-aryle ou -(alkylène en C_{1,2 ou 3})-hétéroaryle ;
au moins l'un des radicaux R¹, R², R³ et R⁴ représentant un radical aryle ou hétéroaryle ;
R⁵ et R⁶ représentent chacun indépendamment de l'autre H; -C(=O)R¹⁸; -C(=O)NR¹⁹R²⁰; -C(=S)NR²¹R²²; -S(=O)₂R²³; un radical alkyle en C₁₋₄, alcényle en C₂₋₄, alcynyle en C₂₋₄; cycloalkyle en C₃₋₈; -(alkylène en C_{1,2 ou 3})-(cycloalkyle en C₃₋₈); hétérocycloalkyle; -(alkylène en C_{1,2 ou 3})-hétérocycloalkyle; aryle; hétéroaryle; -(alkylène en C_{1,2 ou 3})-aryle ou -(alkylène en C_{1,2 ou 3})-hétéroaryle ;
à la condition que R⁵ et R⁶ ne représentent pas simultanément un résidu hydrogène ; ou
R⁵ et R⁶, avec l'atome d'azote qui les relie en tant que chaînon, forment un radical qui est choisi dans le groupe H consistant en 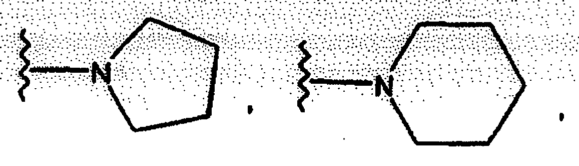 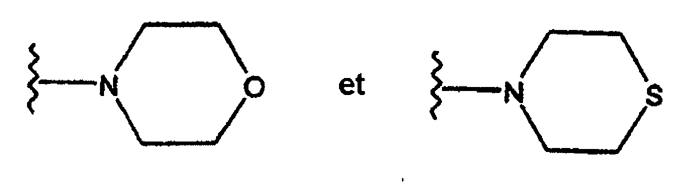 R⁷ et R⁸ représentent chacun indépendamment de l'autre H, -C(=O)R¹⁵ ou un radical alkyle en C₁₋₄, ou
R⁷ et R⁸, avec l'atome d'azote qui les relie en tant que chaînon, forment un radical qui est choisi dans le groupe H indiqué ci-dessus ;
R⁹, R¹⁰, R¹¹ et R¹⁶ représentent chacun indépendamment des autres H ; un radical alkyle en C₁₋₄, alcényle en C₂₋₄, alcynyle en C₂₋₄; cycloalkyle en C₃₋₈; -(alkylène en C₁,_{2 ou 3})-(cycloalkyle en C₃₋₈) ; hétérocycloalkyle; -(alkylène en C_{1,2 ou 3})-hétérocycloalkyle; aryle; hétéroaryle; -(alkylène en C_{1,2 ou 3})-aryle ou -(alkylène en C_{1,2 ou 3})-hétéroaryle ;
R¹² et R¹³ représentent chacun indépendamment de l'autre H ou un radical alkyle en C₁₋₄ ; ou
R¹² et R¹³, avec l'atome d'azote qui les relie en tant que chaînon, forment un radical qui est choisi dans le groupe H indiqué ci-dessus ;
R¹⁴ et R²³ représentent chacun indépendamment de l'autre -NR⁷R⁸; un radical alkyle en C₁₋₄, alcényle en C₂₋₄, alcynyle en C₂₋₄; cycloalkyle en C₃₋₈; -(alkylène en C_{1,2 ou 3})-(cycloalkyle en C₃₋₈); hétérocycloalkyle; -(alkylène en C_{1,2 ou 3})-hétérocycloalkyle; aryle; hétéroaryle; -(alkyle en C_{1,2 ou 3})-aryle ou -(alkylène en C_{1,2 ou 3})-hétéroaryle ;
R¹⁵, R¹⁷ et R¹⁸ représentent chacun indépendamment des autres un radical alkyle en C₁₋₄, alcényle en C₂₋₄; alcynyle en C₂₋₄; cycloalkyle en C₃₋₈; -(alkylène en C_{1,2 ou 3})-(cycloalkyle en C₃₋₈); hétérocycloalkyle; -(alkylène en C_{1,2 ou 3}) -hétérocycloalkyle; aryle; hétéroaryle; -(alkyle en C_{1,2 ou 3})-aryle ou -(alkylène en C_{1,2 ou 3})-hétéroaryle ;
R¹⁹ et R²⁰ représentent chacun indépendamment de l'autre H ou un radical alkyle en C₁₋₄, ou
R¹⁹ et R²⁰, avec l'atome d'azote qui les relie en tant que chaînon, forment un radical qui est choisi dans le groupe H indiqué ci-dessus ;
R²¹ et R²² représentent chacun indépendamment de l'autre H ou un radical alkyle en C₁₋₄, ou
R²¹ et R²², avec l'atome d'azote qui les relie en tant que chaînon, forment un radical qui est choisi dans le groupe H indiqué ci-dessus ;
où
les radicaux alkyle en C₁₋₄, alcényle en C₂₋₄ et alcynyle en C₂₋₄ mentionnés ci-dessus ont chacun une chaîne droite ou ramifiée et peuvent être éventuellement substitués par 1, 2, 3, 4 ou 5 substituants choisis indépendamment les uns des autres dans le groupe consistant en F; Cl; Br; -CN; -OH; -OCH₃ et -NH₂,
les radicaux cycloalkyle en C₃₋₈ mentionnés ci-dessus peuvent être chacun substitués éventuellement par 1, 2, 3, 4 ou 5 substituants choisis indépendamment les uns des autres dans le groupe consistant en F; Cl; Br; -CN; -OH; -CH₃; -C₂H₅; -OCH₃ et -NH₂,
les radicaux hétérocycloalkyle mentionnés ci-dessus ont chacun 4, 5, 6 ou 7 chaînons, comportent chacun 1 ou 2 hétéroatomes choisis indépendamment les uns des autres dans le groupe consistant en l'oxygène, le soufre et l'azote en tant que chaînon(s) et peuvent chacun être éventuellement substitués par 1, 2, 3, 4 ou 5 substituants choisis indépendamment les uns des autres dans le groupe consistant en F; Cl; Br; -CN; -OH; -CH₃; C₂H₅; -OCH₃ et -NH₂,
chacun des radicaux aryle mentionnés ci-dessus représente un radical phényle ou naphtyle, qui peut être substitué par 1, 2, 3, 4 ou 5 substituants choisis indépendamment les uns des autres dans le groupe consistant en F; Cl; Br; -CF₃; -OCF₃; -SCF₃; -SF₅; -CN; -OH; les radicaux méthyle, éthyle, n-propyle, isopropyle, n-butyle, isobutyle, sec-butyle, tert-butyle, méthoxy, éthoxy; -N(CH₃)₂; -N(C₂H₅)₂; phényle; phénoxy, benzyle; thiophényle (thiényle), furannyle, pyridinyle, -C(=O)-NH-CH₃, -C(=O)-NH-C₂H₅, -C(=O)-H, -C(=O)-CH₃, -C(=O)-C₂H₅, -C(=O)-CH(CH₃)₂, -C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(cyclopropyl)-CH₃, -C(=O)-N(cyclopropyl) -C₂H₅, -C(=O)-N(cycloprolyl) -CH(CH₃)₂, -C(=O)-N(cyclopropyl)-C(CH₃)₃, -CH₂-NH-cyclopropyl, -CH₂-N(cyclopropyl)-S(=O)₂-CH₃, -CH₂-N(cyclopropyl)-S(=O)₂-C₂H₅, -CH₂-N(cyclopropyl)-C(=O)-CH₃, -CH₂-N(cyclopropyl)-C(=O)-C₂H₅, -CH₂-N(cyclopropyl)-C(=O) -CH (CH₃)₂, -CH₂-N-cyclopropyl)-C(=O)-C(CH₃)₃ et -CH₂-N(cyclopropyl)-C(=O)-phényle ;
chacun des radicaux hétéroaryle mentionnés ci-dessus représente un radical furannyle, thiényle (thiophényle) ou pyridinyle et peut être éventuellement substitué par 1, 2, 3, 4 ou 5 substituants choisis indépendamment les uns des autres dans le groupe consistant en F; Cl; Br; -CF₃; -OCF₃; -SCF₃; -SF₅; -CN; -OH; -C(=O)-NH-CH₃, -C(=O)-NH-C₂H₅, les radicaux méthyle, éthyle, n-propyle, isopropyle, n-butyle, isobutyle, sec-butyle, tert-butyle, méthoxy, éthoxy; -NH₂; -N(CH₃)₂; -N(C₂H₅)₂; phényle; phénoxy, benzyle; thiophényle (thiényle), furannyle, pyridinyle, -C(=O)-H, -C(=O)-CH3, -C(=O)-C₂H₅, -C(=O)-CH(CH₃)₂, -C(=O)-C(CH₃)₃, -C(=O)-O-CH₃, -C(=O)-O-C₂H₅, -C(=O)-O-C(CH₃)₃, -C(=O)-O-CH(CH₃)₂, -C(=O)-OH, -C(=O)-N(cyclopropyl)-CH₃, -C(=O)-N (cyclopropyl) -C₂H₅, -C(=O)-N (cyclopropyl) -CH(CH₃)₂, -C(=O)-N(cyclopropyl)-C(CH₃)₃, -CH₂-NH-cyclopropyl, -CH₂-N(cyclopropyl)-S(=O)₂-CH₃, -CH₂-N(cyclopropyl)-S(=O)₂-C₂H₅, -CH₂-N(cyclopropyl)-C(=O)-CH3, -CH₂-N(cyclopropyl)-C(=O)-C₂H₅, -CH₂-N(cyclopropyl)-C(=O)-CH(CH₃)₂, -CH₂-N(cyclopropyl)-C(=O)-C(CH₃)₃ et -CH₂-N(cyclopropyl)-C(=O)-phényle,
dans chaque cas éventuellement sous forme de l'un de leurs stéréoisomères purs, en particulier de leurs énantiomères ou diastéréoisomères, de leurs racémates,
ou sous forme d'un mélange de diastéréoisomères, éventuellement des énantiomères et/ou diastéréoisomères, selon un rapport de mélange quelconque, ou dans chaque cas sous forme des sels correspondants, ou dans chaque cas sous forme des produits de solvatation correspondants.

4. Composés selon l'une ou plusieurs des revendications 1-3 de formule générale I' 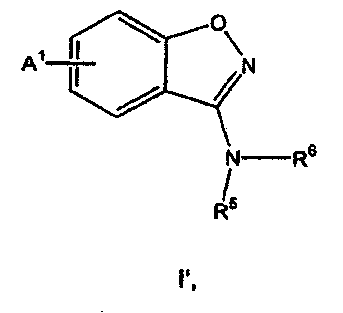 dans laquelle
A¹ est un radical (hétéro) aryle, qui est choisi dans le groupe consistant en les radicaux phényle, 1-naphtyle, 2-naphtyle, thiophényle (thiényle), furannyle et pyridinyle, et qui peut être éventuellement substitué par 1, 2, 3, 4 ou 5 substituants choisis indépendamment les uns des autres dans le groupe consistant en F; Cl; Br; -CF₃; -OCF₃; -SCF₃; -SF₅; -CN; -OH; les radicaux méthyle, éthyle, n-propyle, isopropyle; n-butyle; isobutyle; sec-butyle; tert-butyle; méthoxy; éthoxy; -NH₂; -N(CH₃)₂; -N(C₂H₅)₂; phényle; phénoxy; benzyle; thiophényle (thiényle), furannyle, pyridinyle, -C(=O)-NH-CH₃, -C(=O)-NH-C₂H₅, -C(=O)-H, -C(=O)-CH₃, -C(=O)-C₂H₅, -C(=O)-CH(CH₃)₂, -C(=O)-C(CH₃)₃, -C(=O)-O-CH₃, -C(=O)-OC₂H₅, -C(=O)-O-C(CH₃)₃, -C(=O)-O-CH(CH₃)₂, -C(=O)-OH, -C(=O)-N(cyclopropyl)-CH₃, -C(=O)-N(cyclopropyl)-C₂H₅, -C(=O)-N(cyclopropyl)-CH(CH₃)₂, -C(=O)-N(cyclopropyl)-C(CH₃)₃, -CH₂-NH-cyclopropyl, -CH₂-N(cyclopropyl)-S(=O)₂-CH₃, -CH₂-N(cyclopropyl)-S(=O)₂-C₂H₅, -CH₂-N(cyclopropyl)-C(=O)-CH₃, CH₂-N(cyclopropyl)-C(=O)-C₂H₅, -CH₂-N(cyclopropyl)-C(=O)-CH(CH₃)₂, -CH₂-N(cyclopropyl)-C(=O)-C(CH₃)₃ et -CH₂-N(cyclopropyl)-C(=O)-phényle ;
R⁵ et R⁶ représentent chacun indépendamment de l'autre H ; un radical alkyle choisi dans le groupe consistant en les radicaux méthyle, éthyle, n-propyle, isopropyle, n-butyle, isobutyle, sec-butyle, tert-butyle, n-pentyle et néopentyle; -C(=O)-phényle; -(CH₂)phényle, -(CH₂)₂-phényle, -(CH₂)₃-phényle; -(CH₂)-pyridinyle; -(CH₂)₂-pyridinyle, -(CH₂)₃-pyridinyle; les radicaux phényle et pyridinyle mentionnés ci-dessus pouvant être éventuellement substitués par 1, 2, 3, 4 ou 5 substituants choisis indépendamment les uns des autres dans le groupe consistant en F; Cl; Br; -CF₃; -OCF₃; -SCF₃; -SF₅; -CN; -OH; les radicaux méthyle, éthyle, n-propyle, isopropyle, n-butyle, isobutyle, sec-butyle, tert-butyle, méthoxy, éthoxy, -N(CH₃)₂; -N(C₂H₅)₂; phényle; phénoxy; benzyle; thiophényle (thiényle), furannyle et pyridinyle ;
à la condition que R⁵ et R⁶ ne représentent pas simultanément un résidu hydrogène ;
ou
R⁵ et R⁶, avec l'atome d'azote qui les relie en tant que chaînon, forment un radical qui est choisi dans le groupe H consistant en 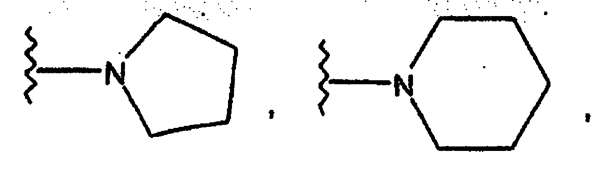 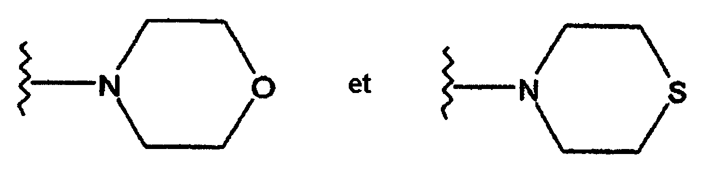 éventuellement chacun sous forme des sels correspondants, en particulier des sels d'addition chlorhydrates, ou chacun sous forme des produits de solvatation correspondants.

5. Composés selon l'une ou plusieurs des revendications 1-4 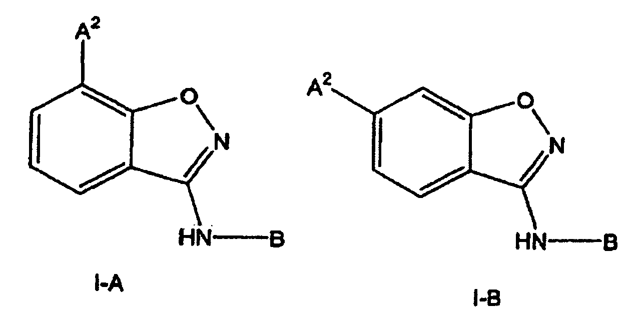 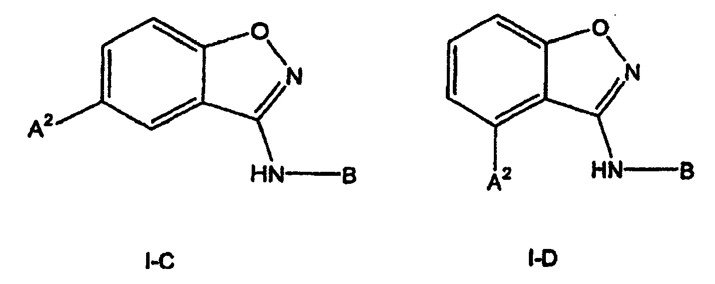 dans chacun desquels
A² représente un radical (hétéro)aryle, qui est choisi dans le groupe consistant en les radicaux phényle, 1-naphtyle, 2-naphtyle, thiophényle (thiényle), furannyle et pyridinyle, et qui peut être éventuellement substitué par 1, 2, 3, 4 ou 5 substituants choisis indépendamment les uns des autres dans le groupe consistant en F; Cl; Br; CF₃; -OCF₃; -SCF₃; -SF₅; -CN; -OH; les radicaux méthyle, éthyle, n-propyle, isopropyle, n-butyle, isobutyle, sec-butyle, tert-butyle, méthoxy, éthoxy; -NH₂; -N(CH₃)₂; -N (C₂H₅)₂; phényle; phénoxy, benzyle; thiophényle (thiényle), furannyle, pyridinyle, -C(=O)-NH-CH₃, -C(=O)-NH-C₂H₅, -C(=O)-H, -C(=O)-CH₃, -C(=O)-C₂H₅, -C (=O) -CH (CH₃)₂, -C(=O)-C(CH₃)₃, -C(=O)-O-CH₃, -C(=O)-O-C₂H₅, -C(=O)-OC(CH₃)₃, -C(=O)-O-CH(CH₃)₂, -C(=O)-OH, -C(=O)-N(cyclopropyl)-CH₃, -C(=O)-N(cyclopropyl)-C₂H₅, -C(=O)-N(cyclopropyl)-CH(CH₃)₂, -C(=O)-N(cyclopropyl)-C(CH₃)₃, -CH₂-NH-cyclopropyle, -CH₂-N(cyclopropyl)-S(=O)₂-CH₃, -CH₂-N(cyclopropyl)-S(=O)₂-C₂H₅, -CH₂-N(cyclopropyl)-C(=O)-CH₃, -CH₂-N(cyclopropyl)-C(=O)-C₂H₅, -CH₂-N(cyclopropyl)-C(=O)-CH(CH₃)₂, -CH₂-N(cyclopropyl)-C(=O)-C(CH₃)₃ et -CH₂-N(cyclopropyl)-C(=O)-phényle ; B représente un radical alkyle choisi dans le groupe consistant en les radicaux méthyle, éthyle, n-propyle, isopropyle, n-butyle, isobutyle, sec-butyle, tert-butyle, n-pentyle et néopentyle; -C(=O)-phényle; -(CH₂)-phényle; -(CH₂)₂-phényle; -(CH₂)₃-phényle; -(CH₂)-pyridinyle; -(CH₂)₂-pyridinyle; -(CH₂)₃-pyridinyle; les radicaux phényle et pyridinyle mentionnés ci-dessus pouvant être éventuellement substitués par 1, 2, 3, 4 ou 5 substituants choisis indépendamment les uns des autres dans le groupe consistant en F; Cl; Br; -CF₃; -OCF₃; -SCF₃; -SF₅; -CN; -OH; les radicaux méthyle, éthyle, n-propyle, isopropyle, n-butyle, isobutyle, sec-butyle, tert-butyle, méthoxy, éthoxy, -N(CH₃)₂; -N(C₂H₅)₂; phényle; phénoxy; benzyle; thiophényle (thiényle), furannyle et pyridinyle,
dans tous les cas éventuellement sous forme des sels correspondants, en particulier des sels d'addition chlorhydrates, ou dans chaque cas sous forme des produits de solvatation correspondants.

6. Composés selon la revendication 5, **caractérisés en ce que** A² représente l'un des radicaux A²¹ ou A²² ci-après : 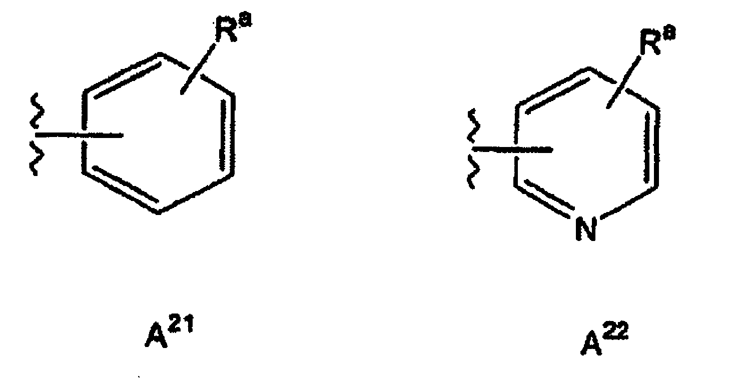 dans lesquels
chaque radical R^{a} représente un radical qui est choisi dans le groupe consistant en H, F, Cl, Br, I, -CN, -CF₃, -OCF₃, -SCF₃, -SF₅, -OH, -SH, les radicaux méthyle; éthyle; n-propyle; isopropyle; n-butyle; isobutyle; sec-butyle; tert-butyle; -OCH₃; -OC₂H₅; -S-CH₃, -S-C₂H₅, -C(=O)-NH-CH₃, -C(=O)-NH-C₂H₅, -C(=O)-H, -C(=O)-O-C₂H₅, -C(=O)-OH, -CH₂-NH-cyclopropyle, -CH₂-N(cyclopropyl)-S(=O)₂-CH₃, -CH₂-N(cyclopropyl) -C(=O)-CH₃ et -CH₂-N(cyclopropyl)-C(=O)-phényle, et
B représente un radical alkyle choisi dans le groupe consistant en les radicaux méthyle, éthyle, n-propyle, isopropyle, n-butyle, isobutyle, sec-butyle, tert-butyle, n-pentyle et néopentyle ;
ou représente l'un des radicaux B¹, B² ou B³ ci-après 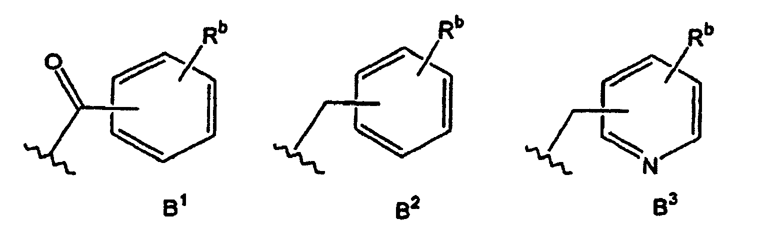 dans lesquels
chaque radical R^{b} est un radical qui est choisi dans le groupe consistant en H, F, Cl, Br, I, -CN, -CF₃, -OCF₃, -SCF₃, -SF₅, -OH, -SH, les radicaux méthyle; éthyle; n-propyle; isopropyle; n-butyle; isobutyle; sec-butyle; tert-butyle; -OCH₃; -OC₂H₅; -S-CH₃ et -S-C₂H₅.

7. Composés selon l'une ou plusieurs des revendications 1 à 6, choisis dans le groupe consistant en les composés suivants
[1] benzyl-[6-(3-chloro-phényl)-benzo[d]isoxazol-3-yl]-amine
[2] benzyl-[6-(4-chloro-phényl)-benzo[d]isoxazol-3-yl]-amine
[3] benzyl-(6-p-tolyl-benzo[d]isoxazol-3-yl)-amine
[4] benzyl-[6-(2-méthoxy-phényl)-benzo[d]isoxazol-3-yl]-amine
[5] N-[6-(3-chloro-phényl)-benzo[d]isoxazol-3-yl]-benzamide
[6] N-[6-(4-chloro-phényl)-benzo[d]isoxazol-3-yl]-benzamide
[7] N-(6-p-tolyl-benzo[d]isoxazol-3-yl]-benzamide
[8] N-[6-(2-méthoxy-phényl)-benzo[d]isoxazol-3-yl]-benzamide
[9] (4-tert-butyl-benzyl)-(6-pyridin-2-yl-benzo[d]-isoxazol-3-yl)-amine
[10] (4-tert-butyl-benzyl)-[6-(3-méthyl-pyridin-2-yl)-benzo[d]isoxazol-3-yl)-amine
[11] (4-tert-butyl-benzyl)-[6-(6-méthyl-pyridin-2-yl)-benzo[d]isoxazol-3-yl)-amine
[12] (4-tert-butyl-benzyl)-(6-phényl-benzo[d]isoxazol-3-yl)-amine
[13] [6-(4-chloro-phényl)-benzo[d]isoxazol-3-yl]-pyridine-2-ylméthy-amine
[14] (6-phényl-benzo[d]isoxazol-3-yl)-pyridin-2-ylméthy-amine
[15] pyridin-2-ylméthyl-(6-o-tolyl-benzo[d]isoxazol-3-yl)-amine
[16] [6-(4-chloro-phényl)-benzo[d]isoxazol-3-yl]-pyridin-3-ylméthy-amine
[17] (6-phényl-benzo[d]isoxazol-3-yl]-pyridin-3-ylméthy-amine
[18] pyridin-3-ylméthyl-(6-o-tolyl-benzo[d]isoxazol-3-yl)-amine
[19] 2-{3-[(pyridin-3-ylméthyl)-amino]-benzo[d]-isoxazol-6-yl}-phénol
[20] [6-(4-chloro-phényl)-benzo[d]isoxazol-3-yl]-pyridin-4-ylméthy-amine
[21] (6-phényl-benzo[d]isoxazol-3-yl)-pyridin-4-ylméthy-amine
[22] pyridin-4-ylméthyl-(6-o-tolyl-benzo[d]isoxazol-3-yl)-amine
[23] 4-{[6-(4-chloro-phényl)-benzo[d]isoxazol-3-ylamino]-méthyl}-benzonitrile
[24] 4-[(6-phényl-benzo[d]isoxazol-3-ylamino)-méthyl]-benzonitrile
[25] 4-[(6-o-tolyl-benzo[d]isoxazol-3-ylamino)-méthyl]-benzonitrile
[26] 4-{[(6-(2-hydroxy-phényl)-benzo[d]isoxazol-3-ylamino]-méthyl}-benzonitrile
[27] [6-(4-chloro-phényl)-benzo[d]isoxazol-3-yl]-(3-méthyl-benzyl)-amine
[28] (3-méthyl-benzyl)-(6-phényl-benzo[d]isoxazol-3-yl)-amine
[29] (3-méthyl-benzyl)-(6-o-tolyl-benzo[d]isoxazol-3-yl)-amine
[30] 2-[3-(3-méthyl-benzylamino)-benzo[d]isoxazol-6-yl]-phénol
[31] (3-chloro-benzyl)-[6-(4-chloro-phényl)-benzo[d]-isoxazol-3-yl]-amine
[32] (3-chloro-benzyl)-(6-phényl-benzo[d]isoxazol-3-yl)-amine
[33] (3-chloro-benzyl)-(6-o-tolyl-benzo[d]isoxazol-3-yl)-amine
[34] 2-[3-(3-choro-benzylamino)-benzo[d]isoxazol-6-yl]-phénol
[35] acide 2-(3-néopentylamino)benzo[d]isoxazol-5-yl)benzoïque
[36] 4-(3-(néopentylamino)benzo[d]isoxazol-5-yl)benzoate d'éthyle,
[37] N-néopentyl-5-(3-(trifluorométhyl)phényl)benzo[d]isoxazol-3-amine
[38] 3-(3-néopentylamino)benzo[d]isoxazol-5-yl)-benzonitrile
[39] N-méthyl-3-(3-(néopentylamino)benzo[d]isoxazol-5-yl)benzamide
[40] acide 3-(3-(néopentylamino)benzo[d]isoxazol-5-yl)benzoïque
[41] 3-(3-(néopentylamino)benzo[d]isoxazol-5-yl)benzoate d'éthyle
[42] N-cyclopropyl-N-(2-méthoxy-5-(3-(néopentylamino)-benzo[d]isoxazol-5-yl)benzyl)méthanesulfonamide
[43] N-cyclopropyl-N-(2-méthoxy-5-(3-(néopentylamino)-benzo[d]isoxazol-5-yl)benzyl)acétamide
[44] N-cyclopropyl-N-(2-fluoro-5-(3-(néopentylamino)-benzo[d]isoxazol-5-yl)benzyl)acétamide
[45] 5-(3-((cyclopropylamino)méthyl)-4-méthoxyphényl)-N-néopentylbenzo[d]isoxazol-3-amine
[46] 5-(3-((cyclopropylamino)méthyl)-4-fluorophényl)-N-néopentylbenzo[d]isoxazol-3-amine
[47] 2-méthoxy-5-(3-(néopentylamino)benzo[d]isoxazol-5-yl)benzaldéhyde
[48] 2-fluoro-5-(3-(néopentylamino)benzo[d]isoxazol-5-yl)benzaldéhyde
[49] N-cyclopropyl-N-(3-(3-(néopentylamino)benzo[d]-isoxazol-5-yl)benzyl)benzamide
[50] N-cyclopropyl-N-(3-(3-(néopentylamino)benzo[d]-isoxazol-5-yl)benzyl)acétamide
[51] 5-(3-((cyclopropylamino)méthyl)phényl)-N-(néopentylbenzo[d]isoxazol-3-amine et
[52] 3-(3-(néopentylamino)benzo[d]isoxazol-5-yl)benzaldéhyde ;
ainsi que, dans chaque cas, leurs sels correspondants, en particulier leurs sels d'addition chlorhydrates, et éventuellement dans chaque cas leurs produits de solvatation correspondants.

8. Composés selon l'une ou plusieurs des revendications 1 à 7, **caractérisés en ce que,** dans le cadre d'un essai FLIPR, ils présentent à une concentration de 10 µM une inhibition de l'influx des ions Ca²⁺ dans les ganglions de la racine postérieure du rat d'au moins 10 %, de préférence d'au moins 30 %, d'une manière particulièrement préférée d'au moins 50 %, d'une manière tout particulièrement préférée d'au moins 70 %, d'une manière encore plus préférée d'au moins 90 %, par comparaison avec l'inhibition maximale réalisable de l'influx des ions Ca²⁺ par la capsaïcine à une concentration de 10 µM.

9. Procédé de préparation de composés selon l'une ou plusieurs des revendications 1 à 8, **caractérisé en ce qu'**on fait réagir un composé 2-fluorobenzonitrile éventuellement substitué de formule générale II 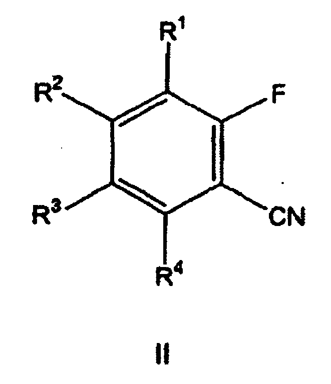 dans laquelle les radicaux R¹, R², R³ et R⁴ ont les significations selon l'une ou plusieurs des revendications 1 à 8, dans un milieu réactionnel en présence d'une base, avec de l'acide acétohydroxamique de formule III 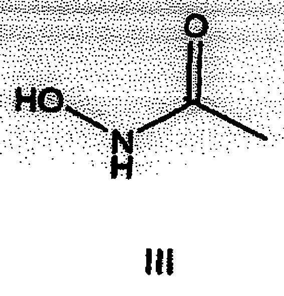 pour obtenir un composé de formule générale I 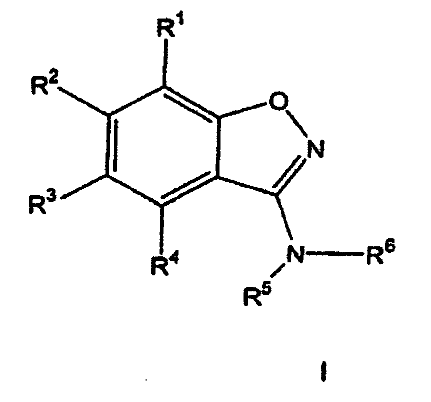 dans laquelle les radicaux R¹ à R⁴ ont les significations données ci-dessus, et les radicaux R⁵ et R⁶ représentent chacun un résidu hydrogène, éventuellement on les purifie et éventuellement on les isole,
puis on fait éventuellement réagir ce composé dans un milieu réactionnel avec au moins un isocyanate de formule générale R¹⁹-N=C=O dans laquelle R¹⁹ a les significations selon l'une ou plusieurs des revendications 1 à 8, éventuellement en présence d'au moins une base, de préférence en présence d'au moins une base choisie dans le groupe consistant en la triéthylamine, la 4,4-diméthylaminopyridine et la diisopropyléthylamine, pour obtenir au moins un composé de formule générale I dans laquelle R¹, R², R³, R⁴ et R⁵ ont les significations données ci-dessus et R⁶ représente -C(=O)-NR¹⁹R²⁰, R¹⁹ ayant les significations données ci-dessus et R²⁰ représentant un résidu hydrogène, et éventuellement on le purifie et/ou éventuellement on l'isole
ou
on le fait ensuite réagir éventuellement dans un milieu réactionnel avec au moins un isocyanate de formule générale S=C=N-R²¹ dans laquelle R²¹ a les significations selon l'une ou plusieurs des revendications 1 à 8, éventuellement en présence d'au moins une base, de préférence en présence d'au moins une base choisie dans le groupe consistant en la triéthylamine, la 4,4-diméthylaminopyridine et la diisopropyléthylamine, pour obtenir au moins un composé de formule générale I dans laquelle R¹, R², R³, R⁴ et R⁵ ont les significations données ci-dessus et R⁶ représente -C(=S)-NR²¹R²² dans laquelle R²¹ a les significations données ci-dessus et R²² représente un résidu hydrogène, et éventuellement on le purifie et/ou éventuellement on l'isole,
et éventuellement on fait réagir au moins un composé de formule générale I dans laquelle R¹, R², R³, R⁴ et R⁵ ont les significations données ci-dessus et R⁶ représente -C(=O)-NR¹⁹R²⁰ ou -C(=S)-NR²¹R²², où R¹⁹ et R²¹ ont les significations données ci-dessus et chacun des radicaux R²⁰ et R²² représente un résidu hydrogène, dans un milieu réactionnel en présence d'au moins une base, de préférence en présence d'au moins un sel hydrure métallique ou d'un sel alcoolate métallique, d'une manière particulièrement préférée en présence d'un sel hydrure métallique ou d'un sel alcoolate métallique choisi dans le groupe consistant en l'hydrure de sodium, l'hydrure de potassium, le tert-butanolate de potassium, le tert-butanolate de sodium, le méthanolate de potassium, le méthanolate de sodium, l'éthanolate de sodium et l'éthanolate de potassium, avec au moins un composé de formule générale LG-R²⁰ ou de formule générale LG-R²², où LG représente un groupe partant, de préférence un atome d'halogène, d'une manière particulièrement préférée un atome de chlore, et chacun des radicaux R²² et R²⁴ a les significations selon l'une ou plusieurs des revendications 1 à 8, à l'exception de l'hydrogène, pour obtenir au moins un composé de formule générale I dans laquelle R¹ à R⁶ ont les significations données ci-dessus, et éventuellement on le purifie et/ou éventuellement on l'isole,
ou
éventuellement on fait réagir au moins un composé de formule générale I dans laquelle R¹, R², R³, R⁴ ont les significations données ci-dessus et R⁵ et R⁶ représentent chacun H, dans un milieu réactionnel en présence d'au moins une base, de préférence en présence d'au moins un sel hydrure métallique ou d'un sel alcoolate métallique, d'une manière particulièrement préférée en présence d'un sel hydrure métallique ou d'un sel alcoolate métallique choisi dans le groupe consistant en l'hydrure de sodium, l'hydrure de potassium, le tert-butanolate de potassium, le tert-butanolate de sodium, le méthanolate de potassium, le méthanolate de sodium, l'éthanolate de sodium et l'éthanolate de potassium, avec au moins un composé de formule générale LG-R⁵ ou de formule générale LG-R⁶ où LG représente un groupe partant, de préférence un atome d'halogène, d'une manière particulièrement préférée un atome de chlore, et R⁵ et R⁶ ont chacun les significations selon l'une ou plusieurs des revendications 1 à 8 à l'exception de l'hydrogène, pour obtenir au moins un composé de formule générale I dans laquelle R¹ à R⁶ ont les significations données ci-dessus, et éventuellement on le purifie et/ou éventuellement on l'isole,
ou
on fait réagir au moins un composé de formule générale I dans laquelle les radicaux R¹-R⁴ ont les significations données ci-dessus, et les radicaux R⁵ et R⁶ représentent chacun H, dans un milieu réactionnel en présence d'au moins un acide et d'au moins un agent réducteur, de préférence en présence d'acide trifluoracétique et de triéthylsilane, avec au moins un composé de formule générale R⁵-(C=O)-H dans laquelle R⁵ a les significations selon l'une ou plusieurs des revendications 1 à 8 à l'exception de H, de -C(=O)R¹⁸, de -C(=O)-NR¹⁹R²⁰, de -C(=S)-NR²¹R²² et de -S(=O)₂R²³, pour obtenir au moins un composé de formule générale I dans laquelle R¹ à R⁵ ont les significations données ci-dessus et R⁶ représente H, et éventuellement on le purifie et/ou éventuellement on l'isole
ou
on fait réagir au moins un composé de formule générale I dans laquelle les radicaux R¹-R⁴ ont les significations données ci-dessus, et les radicaux R⁵ et R⁶ représentent chacun H, dans un milieu réactionnel, avec au moins un composé de formule générale R¹⁸-C(=O)-LG dans laquelle R¹⁸ a les significations selon l'une ou plusieurs des revendications 1 à 8, et LG représente un groupe partant, de préférence un atome d'halogène, d'une manière particulièrement préférée un atome de chlore, pour obtenir au moins un composé de formule générale I dans laquelle R¹ à R⁴ ont les significations données ci-dessus, R⁵ représente un radical -C(=O)R¹⁸ et R⁶ représente un résidu hydrogène, et éventuellement on le purifie et/ou éventuellement on l'isole,
ou
on fait réagir au moins un composé de formule générale I dans laquelle les radicaux R¹-R⁴ ont les significations données ci-dessus, et les radicaux R⁵ et R⁶ représentent chacun H, dans un milieu réactionnel en présence d'au moins un réactif de couplage, éventuellement en présence d'au moins une base, avec au moins un composé de formule générale R¹⁸-C(=O)-OH dans laquelle R¹⁸ a les significations selon l'une ou plusieurs des revendications 1 à 8, pour obtenir au moins un composé de formule générale I dans laquelle R¹ à R⁴ ont les significations données ci-dessus, R⁵ est un radical -C(=O)R¹⁸ et R⁶ représente un résidu hydrogène ; et éventuellement on le purifie et/ou éventuellement on l'isole,
ou
on fait réagir au moins un composé de formule générale I dans laquelle les radicaux R¹-R⁴ ont les significations données ci-dessus, et les radicaux R⁵ et R⁶ représentent chacun H, dans un milieu réactionnel, éventuellement en présence d'au moins une base, avec au moins un composé de formule générale R²³-S(=O)₂-LG dans laquelle R²³ a les significations selon l'une ou plusieurs des revendications 1 à 8, et LG représente un groupe partant, de préférence un atome d'halogène, d'une manière particulièrement préférée un atome de chlore, pour obtenir au moins un composé de formule générale I dans laquelle R¹ à R⁴ ont les significations données ci-dessus, R⁵ est un groupe -S(=O)₂-R²³ et R⁶ représente un résidu hydrogène, et éventuellement on le purifie et/ou éventuellement on l'isole.

10. Procédé de préparation de composés selon l'une ou plusieurs des revendications 1 à 8, **caractérisé en ce qu'**on fait réagir un composé 2-fluorobenzonitrile éventuellement substitué de formule générale II 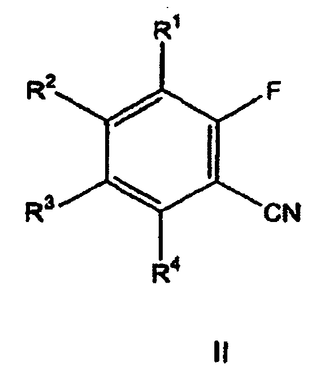 dans laquelle au moins l'un des radicaux R¹, R², R³ et R⁴ représente un groupe partant, de préférence un radical halogéno ou un ester d'acide sulfonique, d'une manière particulièrement préférée un groupe partant choisi dans le groupe consistant en le chlore, le brome, l'iode, le triflate, le mésylate ou le tosylate, d'une manière particulièrement préférée représente un atome de brome, et les autres radicaux ont les significations selon l'une ou plusieurs des revendications 1 à 8 et ne représentent pas un radical aryle ou hétéroaryle éventuellement substitué, dans un milieu réactionnel en présence d'une base, avec de l'acide acétohydroxamique de formule III 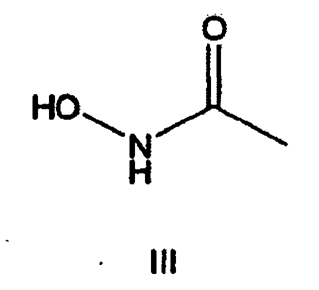 pour donner un composé de formule générale I 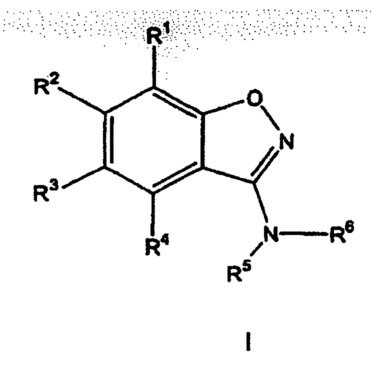 dans laquelle les radicaux R¹ à R⁴ ont les significations données ci-dessus, et les radicaux R⁵ et R⁶ représentent chacun un résidu hydrogène, éventuellement on les purifie et éventuellement on les isole,
puis on fait éventuellement réagir ce composé dans un milieu réactionnel avec au moins un isocyanate de formule générale R¹⁹-N=C=O dans laquelle R¹⁹ a les significations selon l'une ou plusieurs des revendications 1 à 8, éventuellement en présence d'au moins une base, de préférence en présence d'au moins une base choisie dans le groupe consistant en la triéthylamine, la 4,4-diméthylaminopyridine et la diisopropyléthylamine, pour obtenir au moins un composé de formule générale I dans laquelle R¹, R², R³, R⁴ et R⁵ ont les significations données ci-dessus et R⁶ représente -C(=O)-NR¹⁹R²⁰, R¹⁹ ayant les significations données ci-dessus et R²⁰ représentant un résidu hydrogène, et éventuellement on le purifie et/ou éventuellement on l'isole
ou
on le fait ensuite réagir éventuellement dans un milieu réactionnel avec au moins un isocyanate de formule générale S=C=N-R²¹ dans laquelle R²¹ a les significations selon l'une ou plusieurs des revendications 1 à 8, éventuellement en présence d'au moins une base, de préférence en présence d'au moins une base choisie dans le groupe consistant en la triéthylamine, la 4,4-diméthylaminopyridine et la diisopropyléthylamine, pour obtenir au moins un composé de formule générale I dans laquelle R¹, R², R³, R⁴ et R⁵ ont les significations données ci-dessus et R⁶ représente -C(=S)-NR²¹R²² dans laquelle R²¹ a les significations données ci-dessus et R²² représente un résidu hydrogène, et éventuellement on le purifie et/ou éventuellement on l'isole,
et éventuellement on fait réagir au moins un composé de formule générale I dans laquelle R¹, R², R³, R⁴ et R⁵ ont les significations données ci-dessus et R⁶ représente -C(=O)-NR¹⁹R²⁰ ou -C(=S)-NR²¹R²², où R¹⁹ et R²¹ ont les significations données ci-dessus et chacun des radicaux R²⁰ et R²² représente un résidu hydrogène, dans un milieu réactionnel en présence d'au moins une base, de préférence en présence d'au moins un sel hydrure métallique ou d'un sel alcoolate métallique, d'une manière particulièrement préférée en présence d'un sel hydrure métallique ou d'un sel alcoolate métallique choisi dans le groupe consistant en l'hydrure de sodium, l'hydrure de potassium, le tert-butanolate de potassium, le tert-butanolate de sodium, le méthanolate de potassium, le méthanolate de sodium, l'éthanolate de sodium et l'éthanolate de potassium, avec au moins un composé de formule générale LG-R²⁰ ou de formule générale LG-R²², où LG représente un groupe partant, de préférence un atome d'halogène, d'une manière particulièrement préférée un atome de chlore, et chacun des radicaux R²² et R²⁴ a les significations selon l'une ou plusieurs des revendications 1 à 8, à l'exception de l'hydrogène, pour obtenir au moins un composé de formule générale I dans laquelle R¹ à R⁶ ont les significations données ci-dessus, et éventuellement on le purifie et/ou éventuellement on l'isole,
ou
éventuellement on fait réagir au moins un composé de formule générale I dans laquelle R¹, R², R³, R⁴ ont les significations données ci-dessus et R⁵ et R⁶ représentent chacun H, dans un milieu réactionnel en présence d'au moins une base, de préférence en présence d'au moins un sel hydrure métallique ou d'un sel alcoolate métallique, d'une manière particulièrement préférée en présence d'un sel hydrure métallique ou d'un sel alcoolate métallique choisi dans le groupe consistant en l'hydrure de sodium, l'hydrure de potassium, le tert-butanolate de potassium, le tert-butanolate de sodium, le méthanolate de potassium, le méthanolate de sodium, l'éthanolate de sodium et l'éthanolate de potassium, avec au moins un composé de formule générale LG-R⁵ ou de formule générale LG-R⁶ où LG représente un groupe partant, de préférence un atome d'halogène, d'une manière particulièrement préférée un atome de chlore, et R⁵ et R⁶ ont chacun les significations selon l'une ou plusieurs des revendications 1 à 8 à l'exception de l'hydrogène, pour obtenir au moins un composé de formule générale I dans laquelle R¹ à R⁶ ont les significations données ci-dessus, et éventuellement on le purifie et/ou éventuellement on l'isole,
ou
on fait réagir au moins un composé de formule générale I dans laquelle les radicaux R¹-R⁴ ont les significations données ci-dessus, et les radicaux R⁵ et R⁶ représentent chacun H, dans un milieu réactionnel en présence d'au moins un acide et d'au moins un agent réducteur, de préférence en présence d'acide trifluoracétique et de triéthylsilane, avec au moins un composé de formule générale R⁵-(C=O)-H dans laquelle R⁵ a les significations selon l'une ou plusieurs des revendications 1 à 8 à l'exception de H, de -C(=O)R¹⁸, de -C(=O)-NR¹⁹R²⁰, de -C(=S)-NR²¹R²² et de -S(=O)₂R²³, pour obtenir au moins un composé de formule générale I dans laquelle R¹ à R⁵ ont les significations données ci-dessus et R⁶ représente H, et éventuellement on le purifie et/ou éventuellement on l'isole
ou
on fait réagir au moins un composé de formule générale I dans laquelle les radicaux R¹-R⁴ ont les significations données ci-dessus, et les radicaux R⁵ et R⁶ représentent chacun H, dans un milieu réactionnel, avec au moins un composé de formule générale R¹⁸-C(=O)-LG dans laquelle R¹⁸ a les significations selon l'une ou plusieurs des revendications 1 à 8, et LG représente un groupe partant, de préférence un atome d'halogène, d'une manière particulièrement préférée un atome de chlore, pour obtenir au moins un composé de formule générale I dans laquelle R¹ à R⁴ ont les significations données ci-dessus, R⁵ représente un radical -C(=O)R¹⁸ et R⁶ représente un résidu hydrogène, et éventuellement on le purifie et/ou éventuellement on l'isole,
ou
on fait réagir au moins un composé de formule générale I dans laquelle les radicaux R¹-R⁴ ont les significations données ci-dessus, et les radicaux R⁵ et R⁶ représentent chacun H, dans un milieu réactionnel en présence d'au moins un réactif de couplage, éventuellement en présence d'au moins une base, avec au moins un composé de formule générale R¹⁸-C(=O)-OH dans laquelle R¹⁸ a les significations selon l'une ou plusieurs des revendications 1 à 8, pour obtenir au moins un composé de formule générale I dans laquelle R¹ à R⁴ ont les significations données ci-dessus, R⁵ est un radical -C(=O)R¹⁸ et R⁶ représente un résidu hydrogène ; et éventuellement on le purifie et/ou éventuellement on l'isole,
ou
on fait réagir au moins un composé de formule générale I dans laquelle les radicaux R¹-R⁴ ont les significations données ci-dessus, et les radicaux R⁵ et R⁶ représentent chacun H, dans un milieu réactionnel, éventuellement en présence d'au moins une base, avec au moins un composé de formule générale R²³-S(=O)₂-LG dans laquelle R²³ a les significations selon l'une ou plusieurs des revendications 1 à 8, et LG représente un groupe partant, de préférence un atome d'halogène, d'une manière particulièrement préférée un atome de chlore, pour obtenir au moins un composé de formule générale I dans laquelle R¹ à R⁴ ont les significations données ci-dessus, R⁵ est un groupe -S(=O)₂-R²³ et R⁶ représente un résidu hydrogène, et éventuellement on le purifie et/ou éventuellement on l'isole,
et on fait réagir chacun des composés ainsi obtenus de formule générale I dans laquelle au moins l'un des radicaux R¹ à R⁴ représente un groupe partant, de préférence un radical halogéno ou un ester d'acide sulfonique, d'une manière particulièrement préférée un groupe partant choisi dans le groupe consistant en le chlore, le brome, l'iode, le triflate, le mésylate et le tosylate, d'une manière tout particulièrement préférée un atome de brome, et les autres radicaux ont les significations données ci-dessus, éventuellement dans un milieu réactionnel, éventuellement en présence d'une base et d'un catalyseur pouvant être lié à un polymère, de préférence en présence d'au moins une base choisie dans le groupe consistant en le carbonate de potassium, le carbonate de sodium, le phosphate de potassium, l'hydrogénophosphate de sodium, le carbonate de césium, la triéthylamine, le [1,4]-diazabicyclo-[2.2.2]-octane, la diisopropylamine, la diisopropyléthylamine et la N-méthylmorpholine, et d'un catalyseur pouvant être lié à un polymère, choisi dans le groupe consistant en l'acétate de palladium(II), le tris(dibenzylidène-acétone)-dipalladium, la palladium(0)bis(dibenzylidène-acétone), le tétrakis(triphénylphosphine)palladium(0), le (1,1'-bis(diphénylphosphino)-ferrocène)dichloropalladium(II), le bis(acétonitrile)dichloropalladium(II), le chlorure de palladium(II), le dichlorobis(triphénylphosphine)-palladium(II), le dichloro(tricyclohexylphosphine)-palladium(II), le bis(acétato)bis(triphénylphosphine)-palladium(II), le dichlorure de bistriphénylphosphine-palladium(II), l'acétate de bistriphénylphosphine-palladium(II) et le chlorure de fer(III), d'une manière particulièrement préférée en présence d'un carbonate d'un métal alcalin et d'un catalyseur au palladium, d'une manière tout particulièrement préférée en présence de carbonate de sodium et de tétrakis(triphénylphosphine)-palladium(0), éventuellement en présence d'au moins un ligand pouvant être lié à un polymère, choisi dans le groupe consistant en le 2-dicyclohexylphosphino-2',6'-diméthoxybiphényle (S-Phos), le 2-dicyclohexylphosphino-2',4',6'-triisopropylbiphényle (X-Phos), la tricyclohexylphosphine, le tétrafluoroborate de tricyclohexylphosphine, le tétrafluoroborate de tri-tert-butylphosphine, la triphénylphosphine et les sels d'imidazolium, éventuellement par utilisation d'un rayonnement micro-ondes, avec au moins un composé de l'acide boronique de formule générale 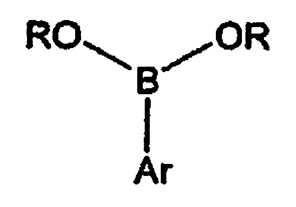 dans laquelle Ar représente un radical aryle ou hétéroaryle non substitué ou au moins monosubstitué selon l'une ou plusieurs des revendications 1 à 8 et R représente l'hydrogène ou un radical organique, de préférence l'hydrogène ou un radical alkyle, ou deux radicaux R, avec le groupe -O-B-O- qui les relie, forment un radical hétérocycloalkyle, et éventuellement on purifie et/ou éventuellement on isole le composé ainsi obtenu de formule générale I dans laquelle au moins l'un des radicaux R¹ à R⁴ représente un radical aryle ou hétéroaryle non substitué ou au moins monosubstitué,
ou on fait réagir chacun des composés ainsi obtenus de formule générale I dans laquelle au moins l'un des radicaux R¹ à R⁴ représente un groupe partant, de préférence un radical halogéno ou un ester d'acide sulfonique, d'une manière particulièrement préférée un groupe partant choisi dans le groupe consistant en le chlore, le brome, l'iode, le triflate, le mésylate et le tosylate, d'une manière tout particulièrement préférée un atome de brome, et les autres radicaux ont les significations données ci-dessus, avec un composé organométallique de formule générale R'-M-X dans laquelle R' représente un radical aryle ou hétéroaryle non substitué ou au moins monosubstitué selon l'une ou plusieurs des revendications 1 à 8, M est un ion d'un métal de transition, de préférence un ion magnésium ou zinc, et X représente un groupe partant, de préférence un radical halogéno ou un ester d'acide sulfonique, d'une manière particulièrement préférée un groupe partant choisi dans le groupe consistant en le chlore, le brome, l'iode, le triflate, le mésylate et le tosylate, d'une manière tout particulièrement préférée un atome de brome, éventuellement dans au moins un milieu réactionnel, éventuellement en présence d'au moins un catalyseur pouvant être lié à un polymère, de préférence en présence d'un catalyseur pouvant être lié à un polymère, choisi dans le groupe consistant en l'acétate de palladium(II), le tris(dibenzylidène-acétone)-dipalladium, la palladium(0)bis(dibenzylidène-acétone), le tétrakis(triphénylphosphine)palladium(0), le (1,1'-bis(diphénylphosphino)-ferrocène)dichloro-palladium(II), le bis(acétonitrile)dichloro-palladium(II), le chlorure de palladium(II), le dichlorobis(triphénylphosphine)palladium(II), le dichloro(tricyclohexylphosphine)palladium(II), le bis(acétato)bis(triphénylphosphine)-palladium(II), le dichlorure de bistriphénylphosphinepalladium(II), l'acétate de bistriphénylphosphinepalladium(II) et le chlorure de fer(III), d'une manière particulièrement préférée en présence de tétrakis(triphénylphosphine)-palladium(0), et éventuellement on purifie et/ou on isole le composé ainsi obtenu de formule générale I dans laquelle au moins l'un des radicaux R¹ à R⁴ représente un radical aryle ou hétéroaryle non substitué ou au moins monosubstitué.

11. Médicament contenant au moins un composé selon l'une ou plusieurs des revendications 1 à 8 et éventuellement un ou plusieurs adjuvants compatibles d'un point de vue physiologique.

12. Médicament selon la revendication 11 pour le traitement et/ou la prophylaxie d'une ou plusieurs maladies choisies dans le groupe consistant en les douleurs, de préférence les douleurs choisies dans le groupe consistant en les douleurs aiguës, les douleurs chroniques, les douleurs neuropathiques et les douleurs viscérales ; les douleurs articulaires ; les migraines ; les dépressions; les névropathies ; les lésions nerveuses ; les maladies neurodégénératives, de préférence choisies dans le groupe consistant en la sclérose en plaques, la maladie d'Alzheimer, la maladie de Parkinson et la chorée de Huntington, les dysfonctionnements cognitifs, de préférence les états de carence cognitive, d'une manière particulièrement préférée les troubles de la mémoire ; l'épilepsie ; les maladies des voies respiratoires, choisies de préférence dans le groupe consistant en l'asthme et les pneumonies ; la toux ; l'incontinence urinaire ; la vessie hyperactive (overactive bladder, OAB) ; les ulcères gastriques ; le syndrome de l'intestin irritable ; les accidents vasculaires cérébraux ; les irritations oculaires ; les irritations cutanées ; les maladies neurotiques de la peau ; les maladies inflammatoires, de préférence les inflammations de l'intestin ; la diarrhée ; le prurit ; les troubles de l'alimentation, choisis de préférence dans le groupe consistant en la boulimie, la cachexie, l'anorexie et l'obésité ; la dépendance aux médicaments ; le mésusage des médicaments ; les phénomènes de sevrage en présence d'une dépendance aux médicaments ; le développement d'une tolérance aux médicaments, de préférence aux opioïdes naturels ou synthétiques ; la dépendance aux drogues ; le mésusage des drogues ; les phénomènes de sevrage en présence d'une dépendance aux drogues ; la dépendance à l'alcool ; le mésusage de l'alcool et les phénomènes de sevrage en présence d'une dépendance à l'alcool ; pour la diurèse ; pour l'anti-natriurèse ; pour influer sur le système cardiovasculaire ; pour augmenter la vigilance ; pour augmenter la libido ; pour la modulation de l'activité motrice ; pour l'anxiolyse ; pour l'anesthésie locale et/ou l'inhibition des effets secondaires indésirables, choisis de préférence dans le groupe consistant en l'hyperthermie, l'hypertension artérielle et la constriction des bronches, déclenchés par l'administration d'agonistes du récepteur vanilloide 1 (récepteurs VR1/TRPV1), choisis de préférence dans le groupe consistant en la capsaïcine, la résinifératoxine, l'olvanil, l'arvanil, le SDZ-249 665, le SDZ-249 482, le nuvanil et le capsavanil.

13. Utilisation d'au moins un composé selon l'une ou plusieurs des revendications 1 à 8 pour préparer un médicament pour le traitement et/ou la prophylaxie d'une ou plusieurs maladies choisies dans le groupe consistant en les douleurs, de préférence les douleurs choisies dans le groupe consistant en les douleurs aiguës, les douleurs chroniques, les douleurs neuropathiques et les douleurs viscérales ; les douleurs articulaires ; les migraines ; les dépressions; les névropathies ; les lésions nerveuses ; les maladies neurodégénératives, de préférence choisies dans le groupe consistant en la sclérose en plaques, la maladie d'Alzheimer, la maladie de Parkinson et la chorée de Huntington, les dysfonctionnements cognitifs, de préférence les états de carence cognitive, d'une manière particulièrement préférée les troubles de la mémoire ; l'épilepsie ; les maladies des voies respiratoires, choisies de préférence dans le groupe consistant en l'asthme et les pneumonies ; la toux ; l'incontinence urinaire ; la vessie hyperactive (overactive bladder, OAB) ; les ulcères gastriques ; le syndrome de l'intestin irritable ; les accidents vasculaires cérébraux ; les irritations oculaires ; les irritations cutanées ; les maladies neurotiques de la peau ; les maladies inflammatoires, de préférence les inflammations de l'intestin ; la diarrhée ; le prurit ; les troubles de l'alimentation, choisis de préférence dans le groupe consistant en la boulimie, la cachexie, l'anorexie et l'obésité ; la dépendance aux médicaments ; le mésusage des médicaments ; les phénomènes de sevrage en présence d'une dépendance aux médicaments ; le développement d'une tolérance aux médicaments, de préférence aux opioïdes naturels ou synthétiques ; la dépendance aux drogues ; le mésusage des drogues ; les phénomènes de sevrage en présence d'une dépendance aux drogues ; la dépendance à l'alcool ; le mésusage de l'alcool et les phénomènes de sevrage en présence d'une dépendance à l'alcool ; pour la diurèse ; pour l'anti-natriurèse ; pour influer sur le système cardiovasculaire ; pour augmenter la vigilance ; pour augmenter la libido ; pour la modulation de l'activité motrice ; pour l'anxiolyse ; pour l'anesthésie locale et/ou l'inhibition des effets secondaires indésirables, choisis de préférence dans le groupe consistant en l'hyperthermie, l'hypertension artérielle et la constriction des bronches, déclenchés par l'administration d'agonistes du récepteur vanilloide 1 (récepteurs VR1/TRPV1), choisis de préférence dans le groupe consistant en la capsaïcine, la résinifératoxine, l'olvanil, l'arvanil, le SDZ-249 665, le SDZ-249 482, le nuvanil et le capsavanil (DA-5018).

14. Utilisation selon la revendication 13 pour préparer un médicament destiné au traitement et/ou à la prophylaxie des douleurs choisies dans le groupe consistant en les douleurs aiguës, les douleurs chroniques, les douleurs neuropathiques et les douleurs viscérales.
